Europäisches Patentamt

European Patent Office

Office européen des brevets

(19)

(11) EP 1 378 518 A1

(12) EUROPEAN PATENT APPLICATION

(43) Date of publication:
07.01.2004 Bulletin 2004/02

(51) Int Cl.$^7$: C07K 14/47

(21) Application number: 02291645.6

(22) Date of filing: 01.07.2002

(84) Designated Contracting States:
AT BE BG CH CY CZ DE DK EE ES FI FR GB GR
IE IT LI LU MC NL PT SE SK TR
Designated Extension States:
AL LT LV MK RO SI

(71) Applicant: Warner-Lambert Company LLC
Morris Plains, New Jersey 07950 (US)

(72) Inventors:
• Bloes-Malderez, Carole
91400 Orsay (FR)
• Schindler, Véronique
75005 Paris (FR)
• Grentzmann, Guido
91370 Verrieres le Buisson (FR)

• Allen, Janet
London EC1V 3RF (GB)

(74) Representative:
Dufresne, Guillaume Alain François et al
Pfizer SCA,
Fresnes Laboratories
3-9, rue de la Loge
B.P. 100
94265 Fresnes Cédex (FR)

Remarks:
The sequence listing, which is published as annex
to the application documents, was filed after the date
of filing. The applicant has declared that it does not
include matter which goes beyond the content of the
application as filed.

(54) **RGS2 as a biomarker for macrophage activation and for activated-macrophage-related disorder**

(57) The present invention relates to use of RGS2 as a biomarker for macrophage activation and activated-macrophage-related disorders, particularly inflammatory disorders. The invention also relates to compounds acting as RGS2 modulators, methods of screening therefore and methods of using them in prevention and treatment of activated-macrophage-related disorders. In addition, the invention provides methods of determining whether macrophages are activated in a sample as well as methods and kits of diagnosis, prognosis, monitoring and assessment of the efficacy of a treatment for activated-macrophage-related disorders using quantification of RGS2 expression and/or activity levels. The invention also provides methods of screening for agents able to interfere with macrophage activation.

EP 1 378 518 A1

**Description**

**Field of the invention**

The present invention is in the field of molecular biology and diagnosis. In particular, the invention relates to RGS2 as a target in activated-macrophage-related disorders such as inflammatory disorders. RGS2 may be useful in the prevention and treatment of such disorders. In addition, RGS2 is a biomarker newly found to be associated with macrophage activation. Such a biomarker may be used in the diagnosis, prognosis, and monitoring of activated-macrophage-related disorders. Such biomarker may also be useful to assess the efficacy of a treatment for activated-macrophage-related disorders as well as in screening assay to assess the efficiency of an agent to modulate macrophage activation.

**Background of the invention**

Macrophages can be segregated into two broad groups: resident tissue macrophages and recruited macrophages. Tissue macrophages are heterogeneous, and those isolated from different anatomical sites, such as liver, kidney or lung, differ in function presumably because of adaptive responses to the local micro-environment. Recruited macrophages are derived largely from circulating monocytes, which infiltrate damaged tissue, but some arise by local cell division. There is now increasing evidence for the heterogeneity of macrophages that have infiltrated inflamed or otherwise damaged tissue, depending on the type and severity of injury, the stage of its evolution and the localization of the macrophages within the tissue.

Macrophages are known to play a central role in the immune response and in inflammation. Macrophages and their released products are indeed involved in inflammation induction and resorption as well as in tissue destruction and subsequent repair. They also display tumoricidal and bactericidal properties. To be able to display those different properties, macrophages need to be activated.

Macrophages are also involved in the development of diseases such as autoimmune disorders and inflammatory diseases. Many clinically significant disorders are indeed accompanied by inflammation, e.g. arthritis, bacterial infections, hypersensitivity, wound, cancer, etc

In active inflammatory bowel disease, there is an increase in the number of mucosal macrophages derived from circulating monocytes. These recruited macrophages are phenotypically different from the resident population of cells and play a major role in mediating the chronic mucosal inflammation seen in patients with ulcerative colitis and Crohn's disease (See Mahida, (2000) Inflamm. Bowel Dis., 6(1):21-33).

Whitney *et al* (2000) (Dig. Dis. Sci.; 45(7):1337.42.) reported an increased number of gastric mucosal macrophages observed in biopsies of *H. pylori*-infected versus uninfected children and that this increase is correlated with gastritis severity.

Macrophages are also known to play an important role in rheumatoid arthritis. The abundance and activation of macrophages in the inflamed synovial membrane/pannus significantly correlates with the severity of rheumatoid arthritis (Kinne *et al,* (2000) Arthritis Res.; 2(3):189-202).

In Experimental autoimmune neuritis, an animal model of the human autoimmune inflammatory disease named Guillain-Barre syndrome, CD4+ T cells mediate demyelination in the peripheral nervous system. Infiltrating macrophages and T cells as well as secretion of cytokines like gamma-interferon are intimately involved in causing pathogenic effects (see Zhu *et al.,* (2001) Exp. Neurol; 169(2):472-8 and Hartung *et al,* (1990) Ann. Neurol.;27 Suppl: 557-63).

In Lab. Invest (1994);71(4):456-64, Van Goor *et al.* provide seminal experimental evidences for the crucial contribution of macrophages in the progression of glomerular and interstitial fibrosis.

In J. Neuropathol. Exp. Neurol. (1998);57(2):168-78, Gveric *et al.* suggest that the activation of the inducible NF-κB pool in macrophages could amplify the inflammatory reaction seen in multiple sclerosis lesions through up regulation of NF-κB-controlled adhesion molecules and cytokines.

In Pathol. Int. (2000);50(6):441-57, Shiozawa discloses results suggesting that persistent accumulation of macrophages in mesangium induce glomerular sclerosis through expression and activation of matrix metalloproteinases.

Smits *et al.* (Eur J Clin Invest. (2000);30(6):469-70) describe the clinical and pathological features of both Alzheimer's disease and HIV-1-associated dementia and tries to interpret the role of the macrophage and astrocytes therein. Although the neuropathology of Alzheimer's disease and HIV-1-associated dementia differs, Alzheimer's disease being a cortical dementia and HIV-1-associated dementia a subcortical dementia, the process of macrophage activation and the resulting pathways leading to neurotoxicity seem very similar. In both Alzheimer's disease and HIV-1-associated dementia, interaction of macrophages and astrocytes appear to play an important role.

Sakai *et al.,* Curr. Opin. Lipidol. (2000);11(5):503-9, suggest that the capacity of oxidized LDL to induce macrophage proliferation *in vitro* may be involved in the enhanced progression of atherosclerosis *in vivo.*

Liu *et al.* (AIDS Res. Hum. Retroviruses (2001);17(15):1423-33) suggest that cyclooxygenase-2-activated and HN-I-infected monocyte-macrophages and T cells play a crucial role in the progression of HIV-1 myocarditis to HIV-1 cardiomyopathy.

In Am. J. Respir. Crit. Care Med. (1997);155(3):858-63, Viksman *et al.* provide evidence for activation of alveolar macrophages, but not peripheral blood monocytes, in subjects with allergic rhinitis and asthma.

In Arch. Pharm. Res. (1999);22(5):437-47, Yoon *et al.* suggest that macrophages, CD4$^+$ T cells and CD8$^+$ T cells act synergistically to kill the beta cells of Langerhans islets in conjunction with beta cell auto antigens and MHC class I and class II antigens, resulting in the onset of autoimmune type I diabetes.

In Am. Rev. Respir. Dis. (1993);147(6 Pt 1):1585-9, Tran Van Nhieu *et al.* demonstrate the major role of alveolar macrophages in the intra-alveolar production of TNF-alpha, and they point out the necessity in adult respiratory distress syndrome for a specific infra-alveolar therapy.

Chronic obstructive pulmonary disease (COPD) is a chronic, slowly progressive disorder characterized by airflow obstruction (associated with airways inflammation). COPD include emphysema, chronic bronchitis, chronic airflow limitation, chronic airways obstruction, non-reversible obstructive airways disease, chronic obstructive airways disease and chronic obstructive lung disease. The clinical presentation of COPD can vary in severity from simple chronic bronchitis without disability to a severely disabled state with chronic respiratory failure. The diagnosis of COPD is usually suggested by symptoms but can only be established by quantitative measurements, preferably using spirometry (see Thorax, 1997; 52 suppl 5: S1-S32), hereby incorporated by reference in its entirety.

The Global Initiative for Chronic Obstructive Lung Disease recently issued guidelines (Pauwels *et al.* (2001) Resp. Care 46(8):798-825), hereby incorporated by reference in its entirety that proposed a four-stage classification of COPD severity, namely:

Stage 0: At Risk— Characterized by chronic cough and sputum production. Lung function, as measured by spirometry, is still normal.

Stage I: Mild COPD-Characterized by mild airflow limitation ($FEV_1$/FVC < 70% but $FEV_1$ > 80% predicted) and usually, but not always, by chronic cough and sputum production.

Stage II-Moderate COPD: Characterized by worsening airflow limitation (30% < $FEV_1$ < 80% predicted) and usually the progression of symptoms with shortness of breath typically developing on exertion. The division into stages IIA (50% < $FEV_1$ < 80% predicted) and IIB (30% < $FEV_1$ < 50% predicted ) is based on the fact that exacerbations are especially seen in patients with a $FEV_1$ below 50% predicted.

Stage III—Severe COPD: Characterized by severe airflow limitation with either; $FEV_1$ < 30% predicted or $FEV_1$ < 50% and the presence of respiratory failure or clinical signs of right heart failure.

FVC is the forced vital capacity and FEV1 the forced expiratory volume in one second as measured by spirometry.

Pulmonary emphysema is a major component of the morbidity and mortality of COPD, a condition that afflicts more than 14 million persons in the United States and has become the fourth leading cause of death. In healthy nonsmoker, macrophages comprise the major host cell defense cell in the lower airspace. Cigarette smoking is associated with a more than fivefold increase in total cells recovered by bronchoalveolar lavage (BAL), with macrophages comprising 95-98%. Moreover, macrophages are prominent in the respiratory bronchioles of cigarette smokers, where emphysematous changes are first manifest. (see Grashoff *et al.,* Am. J. Pathol. (1997);151(6):1785-90 and Shapiro (1999) Am. J. Respir. Crit. Care Med;160(5 Pt 2):S29-32).

In Eur. Respir. J., (1999); 14: 245-248, Haslam *et al.* suggest that the investigatory technique of bronchoalveolar lavage (BAL) has become one of the most valuable research tools for studying inflammatory mechanism in a wide range of diseases that affect the lung and airways in humans and that cytological and microbiological testing of BAL samples are of established value for assisting in clinical diagnosis and management of many lung diseases. These procedures are routinely available in most modern specialist respiratory centers.

Inflammatory and autoimmune diseases are often severely incapacitating, cause the patient great inconvenience and make the patient susceptible to other complications. Diagnostic and treatment of some autoimmune and inflammatory disorders may be difficult to perform just according to disorder-related symptoms. Providing tools and methods allowing establishing a more precise diagnostic are then very useful.

There is then a real need to find biomarkers of macrophage activation that can be used for the different purposes including diagnostic and treatment of auto-immune and inflammatory diseases and particularly of lung and airway inflammatory diseases. The results obtained using these biomarkers may be used for diagnostic purposes, alone or in combination with results obtained using other diagnostic methods in order to get a better diagnostic. Such biomarkers may also be useful for prognosis, monitoring, assessing the efficacy of treatment of autoimmune and inflammatory disorders as well as for preventing and treating such diseases.

RGS2 (Regulator of G protein signaling 2) was first cloned as an early response gene which expression was up-regulated in T cells and was first named G0S8 for putative G0/G1 switch regulatory gene 8 (Siderovski *et al.* (1990) *DNA Cell Biol.* 9:579-587; Siderovski *et al.* (1994) *DNA Cell. Biol.* 13: 125-47, which disclosures are incorporated by reference in their entireties). It was later found to belong to a family of proteins named "Regulator of G protein signaling family" or RGS. G proteins are heterotrimeric proteins consisting of 3 major subunits, $\alpha$, $\beta$ and $\gamma$ and can be classified as Gs, Gi, Gq, $G_{12}$ and $G_{13}$ depending on the interaction of $G_\alpha$-subunits with G Protein Coupled Receptors. RGS proteins act as GAP (GTPase activating proteins) by increasing the rate at which $G_\alpha$ hydrolyze bound GTP and thus return to the inactive state (see Ross and Wilkie, (2000) *Annu. Rev. Biochem.* 69:795-827; De Vries *et al.* (2000) *Ann. Rev. Pharmacol Toxicol.* 40:234-71 for recent reviews which disclosures are incorporated by reference in their entireties). The RGS protein family contains at least 20 members throughout the eukaryotic kingdom (yeasts, worms, fly and mammals) RGS proteins are characterized by a 120-130 amino acid domain called the RGS domain that interacts directly with certain $G_\alpha$ subunits (Chatterjee and Fisher (2000) *J Biol Chem* 275:24013-21; Zheng *et al.* (1999) *Trends. Biochem. Sci.* 24:411-414, which disclosures are incorporated by reference in their entireties).

RGS2 was first shown to preferentially bind *in vitro* to Gq compared to Gi, to accelerate GTP hydrolysis of Gq and inhibit Gq-mediated intracellular signaling (Heximer *et al.* (1997a) *Proc. Natl. Acad. Sci. USA* 94:14389-14393; Ingi *et al.* (1998) *J. Neurosci.* 18 :7178-7188 ; Heximer *et al.* (1999) *J. Biol. Chem.* 274:34253-34259; Beadling *et al.* (1999) *J. Immunol.* 162:2677-2682; Cunningham *et al.* (2001) *J. Biol. Chem.* 276:5438-44; Heximer *et al.* (2001) *J. Biol. Chem.* 276:14195-14203, which disclosures are incorporated by reference in their entireties). Later studies however showed binding to both $Gi_1$ and Gq using an ex *vivo* assay as well as inhibition of both Gi-mediated and Gq-mediated MAP kinase activation by mAChR (Ingi *et al.* (1998) *supra*). *In vitro* binding to $G_\alpha$s has also been reported but remains controversial (Tseng and Zhang (1998) *Endocrinology* 139:4470-5; Heximer *et al.* (1997a) *supra;* Chen *et al.* (1997) *J. Biol. Chem.* 272:8679-868, which disclosures are incorporated by reference in their entireties).

RGS2 was shown to inhibit a wide variety of G-mediated intracellular signaling. For example, RGS2 inhibits muscarininc acetylcholine receptor-mediated MAP kinase activation (Ingi *et al.* (1998) *supra),* glucose-dependent insulinotropic peptide-mediated insulin secretion (Tseng and Zhang (1998) *supra)* and alters the coupling of metabotropic glutamate receptor 1a to M-type $K^+$ and N-type $Ca^{2+}$ channels (Kammermeier and Ikeda (1999) *Neuron* 22:819-829, hereby incorporated by reference in its entirety).

RGS2 knockout mice show reduced T cell proliferation and IL-2 production, increased anxiety responses and decreased male aggression, thus suggesting that RGS2 plays an important role in T cell activation, synapse development and emotive behaviors (Oliveira-Dos-Santos *et al.* (2000) *Proc. Natl. Acad. Sci. U S A* 97:12272-12277, hereby incorporated by reference in its entirety). RGS2 is also thought to promote adipocyte differentiation (Nishizuka *et al.* (2001) *J. Biol. Chem.* 276:29625-29627, hereby incorporated by reference in its entirety) and to play a role in leukemogenesis (Wu *et al.* (1995) *Leukemia* 9:1291-1298, hereby incorporated by reference in its entirety).

In addition to its activity as a GAP, RGS2 was also shown to inhibit the activity of adenyl cyclase III induced by odorant-stimulated olfactory epithelium membranes by acting directly on this effector (Sinarajah *et al.* (2001) *Nature* 409:1051-1055, hereby incorporated by reference in its entirety). RGS2 is also thought to participate in the maintenance of Golgi integrity and intracellular transport via its binding to the COPI coatomer (Sullivan *et al.* (2000) *Mol. Cell. Biol.* 11:3155-3168, hereby incorporated by reference in its entirety).

RGS2 mRNA levels were shown to be up- or down- regulated depending on the cellular context and depending on the type of stimulus. For example, RGS2 expression may be induced by protein kinase C-dependent mechanisms in neuroblastoma cells (Song *et al.* (2001) *Biochem. Biophys. Res. Commun.* 283:102-106, hereby incorporated by reference in its entirety) and in vascular smooth muscle cells (Grant *et al.* (2000) *Mol. Pharmacol.* 57:460-467, hereby incorporated by reference in its entirety). The calcium ionophore ionomycine was shown to up-regulate RGS2 mRNA levels in peripheral blood mononuclear cells (Heximer *et al.* (1997b) *DNA Cell. Biol.* 16:589-598, hereby incorporated by reference in its entirety) and in human myometrial cells (Park *et al.* (2002) *Am. J. Physiol. Endocrinol. Metab.* 282: E580-E584, hereby incorporated by reference in its entirety). Elevation of cAMP levels was also shown to be able to up-regulate RGS2 mRNA levels in rat pheochomocytoma PC12 cells (Pepperl et al. (1998) Biochem. Biophys. Res. Commun. 243:52-55, which disclosure is hereby incorporated by reference in its entirety), in pancreatic islet cells (Tseng and Zhang (1998) supra) and in osteoblasts (Miles *et al.* (2000) *Endocrinol.* 141:28-36, hereby incorporated by reference in its entirety). RGS2 mRNA levels were also shown to be up-regulated by psychoactive agents and long-term neuronal plasticity-evoking stimuli (Ingi *et al* (1998) *supra,* Burchett *et al.* (1998) *J. Neurochem.* 70 :2216-2219, which disclosure is hereby incorporated by reference in its entirety).

Human RGS2 is composed of 5 exons, 3 encoding the RGS domain (Siderovski *et al* (1994) *supra).* Three regions within the RGS domain are thought to be important for interaction with $G_\alpha$ proteins, the first and third one being probably essential for Gq interaction (Heximer *et al.* (1999) *supra).* The Asn residue at position 159 could be crucial for GAP activity and may be involved in stabilizing the transition state of $G_\alpha$ (Zheng *et al.* (1999) *supra).* Mouse RGS2 as well as rat RGS2 cDNAs and proteins have also been reported (Chen *et al.* (1997) *supra;* Miles *et al.* (2000) *supra).*

Although expressed in a wide variety of cells, RGS2 has neither been shown to be expressed in macrophages nor linked to macrophage activation.

**Summary of the invention**

**a) Uses of biomarkers**

The invention relates to the use of RGS2 as a biomarker for macrophage activation.

Particularly, the invention relates to the use of RGS2 as a biomarker for activated-macrophage-related disorders, preferably inflammatory disorders, more preferably respiratory inflammatory disorders, even more preferably COPD.

In a first embodiment, it relates to the use of RGS2 in the *in vitro* diagnosis, prognosis or monitoring of activated-macrophage-related disorders, preferably inflammatory disorders, more preferably respiratory inflammatory disorders, even more preferably COPD.

In a second embodiment, it relates to the use of RGS2 in assessing the efficacy of treatment for activated-macrophage-related disorders, preferably inflammatory disorders, more preferably respiratory inflammatory disorders, even more preferably COPD.

In a third embodiment, it relates to the use of RGS2 in any of the methods and kits disclosed herein.

In a fourth embodiment, said use comprises the use of a RGS2 polynucleotide, a sequence complementary thereto, or a fragment thereof in a hybridization or amplification assay and/or the use of compounds able to bind to a RGS2 polynucleotide, a sequence complementary thereto, or a fragment thereof.

In a fifth embodiment, said use comprises the use of a RGS2 polypeptide or a fragment thereof, and/or the use of compounds able to bind to a RGS2 polypeptide or a fragment thereof in protein quantifying assays.

In a sixth embodiment, it relates to the use of RGS2 in screening assays to determine whether an agent is able to modulate macrophage activation.

**b) Method of determining whether macrophases are activated**

Another object of the invention is a method of determining whether macrophages are activated in a biological sample, said method comprising the steps of

a) quantifying RGS2 expression and/or activity level in macrophages of said biological sample; and
b) determining whether said macrophagic RGS2 expression and/or activity level in said biological sample is elevated compared to the macrophagic RGS2 expression and/or activity level in a control sample.

In a first embodiment, said control sample is a sample containing non activated macrophages.

In a second embodiment, said determining step comprises determining whether said RGS2 expression and/or activity level in macrophages of said biological sample is elevated approximately from 1.7-fold to 1000-fold, preferably from 10-fold to 100-fold, more preferably from 20-fold to 50-fold compared to said macrophagic RGS2 expression and/or activity in said control sample.

In a third embodiment, said quantifying step comprises quantifying RGS2 polynucleotides present in macrophages of said biological sample. In a further embodiment, said quantifying step is performed using a hybridization-based assay. In another further embodiment, said quantifying step is performed using an amplification-based assay selected from the group consisting of PCR assays, competitive PCR assays, real-time PCR assays, branched DNA-based signal amplification assays, nucleic acid sequence based amplification assays (NASBA), and transcription mediated amplification (TMA). In a preferred embodiment, said amplification is performed using PCR. In a further preferred embodiment, said amplification-based assay is real-time PCR. In another further preferred embodiment, said PCR is performed using the amplification primers of SEQ ID No 1 and 2.

In a fourth embodiment, said quantifying step comprises quantifying RGS2 polypeptides present in macrophages of said biological sample. In a further embodiment, said quantifying step comprises the use of a compound able to bind to a RGS2 polypeptide or a fragment thereof. In an even further embodiment, said compound is an antibody or fragment thereof that binds specifically to said RGS2 polypeptide or fragment thereof. In another even further embodiment, said compound is an exogenous $G_\alpha$ subunit, preferably Gq, or a fragment thereof able to bind to RGS2.

In a fifth embodiment, said quantifying step comprises quantifying RGS2 activity in macrophages of said biological sample. In a further embodiment, said RGS2 activity is quantified by measuring the induction of GTPase activity. In an even further embodiment, said induction of GTPase activity is quantified by measuring the degree of acceleration of GTP hydrolysis. In another even further embodiment, said induction of GTPase activity is quantified by measuring the degree of modulation of a $G_\alpha$-, preferably Gq-, mediated intracellular event.

In a sixth embodiment, said biological sample comprises recruited and/or tissue-resident macrophages, preferably from bronchio-pulmonary origin.

Another aspect of the invention is a method of determining whether macrophages are activated in a biological sample, said method comprising the steps of:

a) optionally, purifying macrophages from said biological sample;
b) extracting nucleic acids from said macrophages;
c) contacting said extracted nucleic acids from said macrophages from said biological sample with at least one nucleic acid probe under conditions allowing hybridization to occur between said at least one nucleic acid probe and RGS2 polynucleotides present in said biological sample in order to form a complex;
d) quantifying the amount of hybridized RGS2 polynucleotides in said biological sample; and
e) determining whether said amount of hybridized RGS2 polynucleotides in said biological sample is elevated compared to the macrophagic RGS2 expression level in a control sample.

In a first embodiment, said at least one nucleic acid probe is labeled with a detectable molecule. In a second embodiment, said at least one nucleic acid probe has been immobilized on a substrate. In a further embodiment, said at least one nucleic acid probe that has been immobilized is immobilized on an array. In a third embodiment, said at least one nucleic acid probe has a sequence comprised in a sequence complementary to a RGS2 polynucleotide.

Still another object of the invention is a method of determining whether macrophages are activated in a biological sample, said method comprising the steps of

a) optionally, purifying macrophages from said biological sample;
b) extracting nucleic acids from said macrophages;
c) contacting said nucleic acids from said macrophages from said biological sample with amplification reaction reagents comprising a pair of amplification primers located on either side of a selected RGS2 region to be amplified;
d) performing an amplification reaction to synthesize RGS2 amplicons containing said selected RGS2 region;
e) quantifying the amount of said RGS2 amplicons; and
f) determining whether said amount of RGS2 amplicons in said biological sample is elevated compared to the macrophagic RGS2 expression level in a control sample.

Optionally, when the selected RGS2 polynucleotide to be amplified is RNA, reverse transcription, and optionally synthesis of a second cDNA strand, are performed prior to said contacting step.

In a first embodiment, said quantifying step comprises hybridization with a labeled probe having a sequence complementary to the RGS2 amplicon. In a second embodiment, said quantifying step comprises using incorporated labeled dNTPs, preferably fluorescent dNTPs. In a third embodiment, said quantifying step comprises using a double-stranded DNA specific dye, preferably a fluorescent dye. In a fourth embodiment, said amplification reaction is performed using semi-quantitative PCR or quantitative PCR, preferably real-time PCR.

Still another object of the invention is a method of determining whether macrophages are activated in a biological sample, said method comprising the steps of

a) optionally, purifying macrophages from said biological sample;
b) extracting proteins from said macrophages;
c) contacting said proteins from said macrophages of said biological sample with a compound able to bind to a RGS2 polypeptide or a fragment thereof under conditions allowing said compound to bind to said RGS2 polypeptide or said fragment thereof;
d) quantifying the amount of said RGS2 polypeptides; and
e) determining whether said amount of RGS2 polypeptides in said biological sample is elevated compared to the macrophagic RGS2 expression level in a control sample.

In a first embodiment, said compound is a $G_\alpha$ subunit, preferably a Gq subunit, or a fragment thereof able to bind to RGS2.

In a second embodiment, said compound is an antibody or a fragment thereof able to bind to said RGS2 polypeptide or fragment thereof. In a further embodiment, said antibody or fragment thereof is labeled with a detectable molecule. In another further embodiment, said antibody has been immobilized on a substrate.

In a third embodiment, said quantifying step comprises using an assay selected from the group consisting of enzyme-linked immunosorbent assays, radioimmunoassays, immunoblotting assays, immunofluorescent assays, immunoprecipitation assays, chemiluminescent assays, and immunohistochemical assays.

In a preferred embodiment, said assay is an enzyme-linked immunosorbent assay. In a first embodiment of said preferred embodiment, said contacting step comprises contacting a solid support or matrix to which are bound antibodies or fragments thereof that recognize a RGS2 polypeptide or fragment thereof with said biological sample under conditions allowing the formation of immobilized RGS2-antibody complexes, arid wherein said quantifying step comprises detecting said immobilized RGS2-antibody complexes via a labeled detection molecule, preferably another antibody, that specifically binds to said RGS2 polypeptide or fragment thereof in said immobilized RGS2-antibody complexes.

In a second embodiment of said preferred embodiment, said contacting step comprises incubating said sample with a first antibody that specifically recognize and bind to a RGS2 polypeptide or fragment thereof under conditions that allow the formation of RGS2-antibody complexes, and wherein said quantifying step comprises adding a second antibody that bind to said RGS2-antibody complexes, wherein said second antibody is an antibody that binds to a different RGS2 epitope or an antibody that bind to the first antibody already bound to said RGS2 polypeptides or fragment thereof. Said second antibody may either be labeled or coupled to a solid support in order to provide for a selectable marker.

In a third embodiment of said preferred embodiment, said contacting step comprises incubating said sample with an excess of antibody that specifically recognizes and binds to a RGS2 polypeptide or fragment thereof under conditions that allow the formation of RGS2-antibody complexes, and wherein said quantifying step comprises adding a known amount of unsolubilized recombinant RGS2 polypeptide or fragment thereof to determine the amount of remaining unbound antibody. Preferably, said recombinant RGS2 polypeptide or fragment thereof is labeled.

Still another object of the invention is a method of determining whether macrophages are activated in a biological sample, said method comprising the steps of

a) optionally, purifying macrophages from said biological sample;
b) quantifying the amount of RGS2 activity; and
c) determining whether said RGS2 activity in said biological sample is elevated compared to the macrophagic RGS2 activity level in a control sample.

In one embodiment, said RGS2 activity is assayed by measuring the induction of GTPase activity. In a further embodiment, said induction of GTPase activity is quantified by measuring the degree of acceleration of GTP hydrolysis. In another further embodiment, said induction of GTPase activity is quantified by measuring the degree of modulation of a $G_\alpha$-, preferably Gq-, mediated intracellular event.

**c) Diagnostic, prognosis and monitoring methods for activated-macrophage-related disorders using quantification of RGS2 expression and/or activity.**

Other objects of the invention are method of diagnosis, prognosis and monitoring of activated-macrophage-related disorder for an individual, wherein said method comprises the step of quantifying the level of macrophagic RGS2 expression and/or activity levels in at least one biological sample recovered from said individual using any one of the methods described herein.

In a first embodiment, said method is a method of determining whether an individual suffers from an activated-macrophage-related disorder, wherein said method comprises the steps of

a) quantifying the level of macrophagic RGS2 expression and/or activity levels in a biological sample recovered from said individual using any one of the methods described herein; and
b) determining whether said macrophagic RGS2 expression and/or activity levels are elevated in said individual compared to the macrophagic RGS2 expression level and/or activity in a control sample,

wherein said elevated level of macrophagic RGS2 expression and/or activity is taken as a sign of the presence of an activated-macrophage-related disorder.

In a further embodiment, said control sample originates from an individual not suffering from said activated-macrophage-related disorder. In an even further embodiment, said individual not suffering from said activated-macrophage-related disorder is a healthy individual. In another further embodiment, said individual not suffering from said activated-macrophage-related disorder is said individual before he suffers from said activated-macrophage-related disorder.

In a second embodiment, said method is a method of determining whether an individual who already suffers from a disorder different from an activated-macrophage-related disorder is susceptible to develop an activated-macrophage-related disorder, wherein said method comprises the steps of

a) quantifying the level of macrophagic RGS2 expression and/or activity levels in a biological sample recovered from said individual using any one of the methods described herein; and

b) determining whether said macrophagic RGS2 expression and/or activity level in said individual is similar to the macrophagic RGS2 expression and/or activity level of an individual suffering from an activated-macrophage-related-disorder,

wherein said similar level of macrophagic RGS2 expression and/or activity is taken as a sign of the evolution of an activated-macrophage-related disorder.

In a third embodiment, said method is a method of determining whether an individual who already suffers from an activated-macrophage-related disorder is susceptible to move into a more advanced stage of said activated-macrophage-related disorder, wherein said method comprises the steps of

a) quantifying the level of macrophagic RGS2 expression and/or activity in a biological sample recovered from said individual using any one of the methods described herein; and
b) determining whether said macrophagic RGS2 expression and/or activity level in said individual is similar to the macrophagic RGS2 expression and/or activity level of an individual suffering from a more advanced stage of said activated-macrophage-related-disorder;

wherein said similar level of macrophagic RGS2 expression and/or activity is taken as a sign of the evolution for said individual towards said more advanced stage of said activated-macrophage-related disorder.

In a further embodiment, said activated-macrophage-related disorder is chronic obstructive pulmonary disease. In an even further embodiment, said individual who already suffers from chronic obstructive pulmonary disease is at stage 0 and said individual suffering from a more advanced stage of chronic obstructive pulmonary disease is at stage I. In another even further embodiment, said individual who already suffers from chronic obstructive pulmonary disease is at stage I and said individual suffering from a more advanced stage of chronic obstructive pulmonary disease is at stage II. In another even further embodiment, said individual who already suffers from chronic obstructive pulmonary disease is at stage II and said individual suffering from a more advanced stage of chronic obstructive pulmonary disease is at stage III.

In a fourth embodiment, said method is a method of monitoring an individual suffering from an activated-macrophage-related-disorder, wherein said method comprises the steps of

a) quantifying the macrophagic level of RGS2 expression and/or activity in different samples recovered from said individual at different time points; and
b) comparing the macrophagic RGS2 expression and/or activity levels in said different samples.

In a further embodiment, said different time points correspond to different stages of said activated-macrophage-related disorder. In an even further embodiment, said activated-macrophage-related disorder is chronic obstructive pulmonary disease.

In another further embodiment, said method comprises the additional step of determining whether levels of macrophagic RGS2 expression and/or activity increases over time, wherein said increase is taken as a sign of an increased degree of severity of said activated-macrophage-related-disorder

Still another object of the invention is a method of assessing the efficacy of a treatment for an individual suffering from an activated-macrophage-related disorder and submitted to a treatment, wherein said method comprises the steps of

a) quantifying the macrophagic RGS2 expression levels and/or activity in a biological sample recovered from said individual using any one of the methods described herein; and
b) determining whether said macrophagic RGS2 expression level and/or activity is modulated in said biological sample compared to the macrophagic RGS2 expression and/or activity level in a control sample.

In a further embodiment, said method is a method of assessing the efficacy of a treatment for an individual suffering from an activated-macrophage-related disorder, wherein said method comprises the steps of

a) quantifying the macrophagic RGS2 expression levels and/or activity in a biological sample recovered from said individual using any one of the methods described herein; and
b) determining whether said macrophagic RGS2 expression and/or activity level in said individual is decreased compared to the level of macrophagic RGS2 expression and/or activity in an individual suffering from said activated-macrophage-related disorder but not submitted to said treatment,
wherein a decreased level of said macrophagic RGS2 expression and/or activity is taken as a sign of the efficacy of said treatment.

In any of the methods cited above, said activated-macrophage-related disorders is selected from the group consisting of inflammatory diseases of the gastrointestinal tract such as Crohn's disease, inflammatory bowel disease, gastritis, colitis, ulcerative colitis, colon irritable, gastric ulcer and duodenal ulcer, inflammatory diseases of the skin such as psoriasis, inflammatory diseases of the respiratory system such as asthma, allergic rhinitis or chronic obstructive pulmonary disease (COPD), inflammatory diseases of the musculoskeletal system such as rheumatoid arthritis, or neuritis, renal inflammation, multiple sclerosis, Alzheimer's disease, atherosclerosis or HIV-1-associated dementia, myocarditis, autoimmune diabete, sepsis, septic shock, endotoxic shock, adult respiratory distress syndrome or graft versus host reaction.

In a preferred embodiment, said activated-macrophage-related disorder is an inflammatory disorder, preferably a respiratory inflammatory disorder, even more preferably COPD.

In any of the methods cited above, said biological sample is a sample recovered from any one of blood, broncho alveolar lavage, sputum, synovial fluid, bone, central nervous system, skin, hematopoeitic tissues including but not limited to lung, spleen, thymus, bone marrow, lamina propria, liver or cell cultures derived from tissue biopsies.

In any of the methods cited above, said biological sample comprises recruited macrophages and/or tissue-resident macrophages, preferably from bronchio-pulmonary origin.

### d) Screening assays for agents modulating macrophage activation

Another object of the invention is a method of determining whether an agent interferes with macrophage activation, wherein said method comprises the steps of

a) contacting said agent with macrophages under conditions allowing quantification of RGS2 expression and/or activity; and

b) determining whether RGS2 expression levels and/or activity are modulated in said macrophages upon macrophage activation using any method described herein.

In a first embodiment, said method is a method of determining whether an agent is able to reduce or inhibit macrophage activation, wherein said method comprises the steps of

a) contacting said agent with macrophages under conditions allowing quantification of RGS2 expression and/or activity; and

b) determining whether RGS2 expression levels and/or activity is reduced upon macrophage activation in said sample compared to the macrophagic RGS2 expression and/or activity level of a control sample using any method described herein.

In a second embodiment, said method is a method of determining whether an agent is able to trigger or enhance macrophage activation, wherein said method comprises the steps of

a) contacting said agent with a sample containing macrophages under conditions allowing quantification of RGS2 expression and/or activity; and

b) determining whether RGS2 expression levels and/or activity is elevated in said sample upon macrophage activation compared to the macrophagic RGS2 expression and/or activity level of a control sample using any method described herein.

In one embodiment of any of the screening methods described above, said contacting step occurs prior to macrophage activation. In another embodiment of any of the screening methods described above, said contacting step occurs after macrophage activation.

### e) Kits

Another aspect of the invention is to provide kits for performing methods according to the invention.

Therefore another object of the invention is a kit, preferably a diagnostic kit for detection of an activated-macrophage-related disorder, wherein said kit comprises reagents to determine the level of RGS2 expression and/or activity.

In a first embodiment, the invention relates to kits, preferably diagnostic kits for activated-macrophage-related disorders, containing necessary material to quantify the amount of RGS2 polynucleotides in a biological sample.

In a further embodiment, said kit comprises at least one oligonucleotide able to hybridize to a RGS2 polynucleotide, a sequence complementary thereto or a fragment thereof. In an even further embodiment, said oligonucleotide is a nucleic acid probe able to hybridize specifically to said RGS2 polynucleotide, sequence complementary thereto or fragment thereof. In a preferred embodiment, said kit comprises amplification primers, preferably primers of SEQ ID No 1 and/or SEQ ID No 2, able to amplify a region of a RGS2 polynucleotide, preferably using real-time PCR.

In a second embodiment, the invention also relates to kits, preferably diagnostic kits for activated-macrophage-related disorders, containing necessary material to quantify the amount of RGS2 polypeptides in a biological sample.

In a further embodiment, said kit comprises an anti-RGS2 antibody able to bind specifically to a RGS2 polypeptide or fragment thereof. In an even further embodiment, said anti-RGS2 antibody is labeled. In another even further embodiment, said kit comprises necessary reagent to perform an ELISA. In still another even further embodiment, said kit further comprises a reagent allowing the detection of the antigen-antibody complexes formed, said reagent optionally carrying a label, or being able to be recognized itself by a label reagent, particularly in the case when the above mentioned anti-RGS2 antibody is not itself labeled. In still another embodiment, said kit comprises a $G_\alpha$ polypeptide, preferably a Gq polypeptide, or a fragment thereof, able to bind to RGS2.

In a third embodiment, the invention also relates to kits, preferably diagnostic kits for activated-macrophage-related disorders, containing necessary material to quantify the amount of RGS2 activity in a biological sample. In a preferred embodiment, said kit comprises necessary reagents to measure the degree of induction of GTPase activity. In a further preferred embodiment, said kit comprises necessary reagent to measure GTPase activity. In another further preferred embodiment, said kit comprises necessary reagents to measure the degree of modulation of a $G_\alpha$-, preferably Gq-, mediated intracellular event.

## f) Methods to find modulators of RGS2-dependent-macrophage activation and uses thererof

Another object of the invention relates to methods of determining whether a compound is able to modulate macrophage activation, said method comprising the steps of:

    a) contacting said compound with macrophages in a sample under conditions allowing quantification of RGS2 expression and/or activity;
    b) activating said macrophages in said sample; and
    c) determining whether the RGS2 expression and/or activity level in activated macrophages in said sample changes significantly compared to the macrophagic RGS2 expression and/or activity level in a control sample containing activated macrophages not contacted with said compound, using any one of the methods disclosed herein.

In a first embodiment, said invention relates to methods of determining whether a compound is able to trigger or enhance the RGS2 expression and/or activity induced by macrophage activation, wherein said step c) comprising determining whether the RGS2 expression and/or activity level in activated macrophages in said sample is higher than the macrophagic RGS2 expression and/or activity level in said control sample.

In a second embodiment, said invention relates to methods of determining whether a compound is able to reduce or inhibit the RGS2 expression and/or activity induced by macrophage activation, wherein said step c) comprising determining whether the RGS2 expression and/or activity level in activated macrophages in said sample is lower than the macrophagic RGS2 expression and/or activity level in said control sample.

The invention also relates to methods of determining whether a compound is able to modulate the RGS2 expression and/or activity in activated macrophages, said method comprising the steps of:

    a) contacting said compound with activated macrophages in a sample under conditions allowing quantification of RGS2 expression and/or activity; and
    b) determining whether the RGS2 expression and/or activity level in activated macrophages in said sample changes significantly compared to the macrophagic RGS2 expression and/or activity level in a control sample containing activated macrophages not contacted with said compound, using any one of the methods disclosed herein.

In a first embodiment, said invention relates to methods of determining whether a compound is able to reduce or inhibit the RGS2 expression and/or activity in activated macrophages, wherein said step b) comprising determining whether the RGS2 expression and/or activity level in activated macrophages in said sample is lower than the macrophagic RGS2 expression and/or activity level in said control sample.

In a second embodiment, said invention relates to methods of determining whether a compound is able to trigger or enhance the RGS2 expression and/or activity in activated macrophages, wherein said step b) comprising determining whether the RGS2 expression and/or activity level in activated macrophages in said sample is higher than the macrophagic RGS2 expression and/or activity level in said control sample.

In a preferred embodiment of any of the methods cited above, said compound able to inhibit or reduce RGS2 expression and/or activity induced by macrophage activation or already present in activated macrophages is able to bind to a RGS2 polynucleotide or a fragment thereof. In a further embodiment, said compound is able to bind to regulatory sequences of said RGS2 polynucleotide or fragment thereof. In another further embodiment, said compound is an antisense oligonucleotide or an oligonucleotide able to form a triple helix with said RGS2 polynucleotide or fragment thereof. In a second more preferred embodiment, said compound able to inhibit or reduce RGS2 expression and/or activity induced by macrophage activation or already present in activated macrophages is able to bind to a RGS2 polypeptide or fragment thereof. In a further preferred embodiment, said compound able to bind to said RGS2 polypeptide or fragment thereof is an anti-RGS2 antibody or a fragment thereof. In another further preferred embodiment, said compound able to bind to said RGS2 polypeptide or fragment thereof is a RGS2 antagonist or a fragment thereof. In an even further embodiment, said RGS2 antagonist is a $G_\alpha$ polypeptide, preferably a Gq polypeptide, or a fragment thereof, unable to hydrolyze GTP but able to bind to RGS2. In a third more preferred embodiment, said compound able to inhibit or reduce RGS2 expression and/or activity induced by macrophage activation or already present in activated macrophages is a non functional RGS2 polypeptide or a fragment thereof.

In another preferred embodiment, said compound able to trigger or enhance RGS2 expression and/or activity induced by macrophage activation or already present in activated macrophages is able to bind to a RGS2 polynucleotide or a fragment thereof. In a further embodiment, said compound is able to bind to regulatory sequences of said RGS2 polynucleotide or fragment thereof. In a second more preferred embodiment, said compound able to trigger or enhance RGS2 expression and/or activity induced by macrophage activation or akeady present in activated macrophages is able to bind to a RGS2 polypeptide or fragment thereof. In a further preferred embodiment, said compound able to bind to said RGS2 polypeptide or fragment thereof is a RGS2 agonist or a fragment thereof.

Another object of the invention relates to a RGS2 modulator, preferably a RGS2 inhibitor, identified by any of the methods described herein.

Other objects of the invention are compounds able to inhibit or reduce macrophage activation via inhibition or reduction of RGS2 expression and/or activity.

In a first embodiment, said compound is able to inhibit or reduce RGS2 expression and/or activity induced by macrophage activation.

In a second embodiment, said compound is able to reduce RGS2 expression and/or activity in activated macrophages.

In a third embodiment, said compound is able to bind to a RGS2 polynucleotide or a fragment thereof. In a further embodiment, said compound is able to bind to regulatory sequences of said RGS2 polynucleotide or fragment thereof. In another further embodiment, said compound is an antisense oligonucleotide or an oligonucleotide able to form a triple helix with said RGS2 polynucleotide or fragment thereof.

In a fourth embodiment, said compound is able to bind to a RGS2 polypeptide or a fragment thereof. In a further embodiment, said compound able to bind to said RGS2 polypeptide or fragment thereof is an anti-RGS2 antibody or a fragment thereof. In another further embodiment, said compound able to bind to said RGS2 polypeptide or fragment thereof is a RGS2 antagonist or a fragment thereof.

In a fifth embodiment, said compound is a non functional RGS2 polypeptide or a fragment thereof.

In a sixth embodiment, said compound is a $G_\alpha$ polypeptide, preferably a Gq polypeptide, or a fragment thereof, unable to hydrolyze GTP but able to bind to RGS2.

The invention also relates to methods for the production of a pharmaceutical composition comprising the steps of

a) identifying compounds able to modulate RGS2 expression and/or activity induced by macrophage activation or present in activated macrophages using any of the methods disclosed herein; and
b) mixing the identified compound with a pharmaceutically acceptable carrier.

Another object of the invention is a pharmaceutical composition comprising a RGS2 modulator, preferably a RGS2 inhibitor, preferably identified by any one of the methods described herein in combination with a pharmaceutically acceptable carrier.

Another object of the invention relates to the use of a RGS2 modulator, preferably a RGS2 inhibitor, preferably identified by any of the methods described herein for the prevention or the treatment of an activated-macrophage-related disorder, preferably an inflammatory disorder, more preferably a respiratory inflammatory disorder, even more preferably COPD.

Another object of the invention relates to the use of a RGS2 inhibitor identified by any of the methods described herein for the preparation of a medicament for the prevention or the treatment of an activated-macrophage-related disorder, preferably an inflammatory disorder, more preferably a respiratory inflammatory disorder, even more preferably COPD.

Another object of the invention is a method for the prevention or the treatment of an activated-macrophage-related disorder comprising administering to a mammal in need thereof, an effective amount of a RGS2 inhibitor identified by any of the methods described herein.

## Detailed description of the invention

### I. Definition of terms

### Macrophage

As used herein, the term « macrophage » encompasses any type of macrophages including recruited macrophages, tissue resident macrophages, or a mixture of recruited macrophages and tissue resident macrophages, irrespective of their tissue origin. Preferably, this term refers to macrophages from bronchio-pulmonary origin such as BAL, lung tissue or sputum.

Macrophage activation

The term "macrophage activation", as used herein, relates to the process by which one or several functions are modulated, enhanced or inhibited in a macrophage, by a macrophage, or in circulating monocytes during their recruitment and differentiation into macrophages, in response to one or several stimuli.
Said functions which are modulated, enhanced or inhibited in a macrophage or in circulating monocytes during their recruitment and differentiation into macrophages are selected from phagocytosis, synthesis and secretion of cytokines, production of proteases, production of angiogenic factors, antigen processing and presentation, and any inflammatory functions.
Said functions which are modulated, enhanced or inhibited by a macrophage are selected from lymphocyte activation, T-cell activation, TH1 or TH2 response, inflammation, fever, anti-infectious activity, anti-tumoral activity, tissue injury, tissue repair, and immune cells attraction.
Said stimuli include any stimulus modulating, enhancing or inhibiting any of the above functions. Illustrating but not limiting examples of such stimuli are aggregation of Fc$\gamma$ receptors, lipopolysaccharide (LPS), gamma-interferon ($\gamma$-INF), Migration inhibitory factor (MIF), interleukine 1(IL-1), interleukine 4 (IL4), interleukine 6 (IL-6), interleukine 10 (IL-10), Transforming growth factor-beta (TGF$\beta$), glucocorticoids and tumor necrosis factor alpha (TNF$\alpha$).
Preferably, the term «macrophage activation » relates to the process by which inflammatory functions are modulated, preferably induced or increased, in a macrophage in response to one or several stimuli.
Preferably, said stimuli include any stimulus modulating, preferably inducing or increasing, inflammatory functions in a macrophage. Illustrating but not limiting examples of such stimuli are aggregation of Fc$\gamma$ receptors, lipopolysaccharide (LPS), gamma interferon- ($\gamma$-INF), and Migration inhibitory factor (MIF).
Said inflammatory functions include any function modulated, preferably induced or increased by said stimuli which results in modulating, preferably inducing or increasing an inflammatory reaction. Illustrating but not limiting examples of such inflammatory functions are secretion of inflammatory cytokines, such as interleukines (e.g. interleukine 1 (IL-1), interleukine 6 (I-L6), interleukine 8 (IL-8), interleukine 12 (IL-12), Tumor Necrosis Factor $\alpha$ (TNF$\alpha$)), leukocyte chemoattractant mediator (LTB4), chemokines (e.g. Monocyte Chemotactic Proteins (MCP-1), macrophage inflammatory protein 1 alpha (MIP1$\alpha$), macrophage inflammatory protein 1 beta (MIP1$\beta$), monokine induced by gamma interferon (MIG)), nitric oxide and superoxide production, increase of Fc$\gamma$RI expression and protease liberation.

### Activated-macrophage-related disorders

The term "activated-macrophage-related disorder", as used herein, encompasses all disorders in which the activation of macrophages or activated macrophages play a role such as auto-immune disorders and inflammatory disorders irrespective of the cause of such disorders (e.g. auto-antigen, microorganism infection, hypersensibility, wound, and the like). Illustrating but not limiting examples of such activated-macrophage-related disorders are inflammatory diseases of the gastrointestinal tract such as Crohn's disease, inflammatory bowel disease, gastritis, colitis, ulcerative colitis, colon irritable, gastric ulcer and duodenal ulcer, inflammatory diseases of the skin such as psoriasis, inflammatory diseases of the respiratory system such as asthma, allergic rhinitis or chronic obstructive pulmonary disease (COPD), inflammatory diseases of the musculoskeletal system such as rheumatoid arthritis, or neuritis, renal inflammation, multiple sclerosis, Alzheimer's disease, atherosclerosis and HN-1-associated dementia, myocarditis, autoimmune diabete, sepsis, septic shock, endotoxic shock, adult respiratory distress syndrome and graft versus host reaction.
Preferably, the term activated-macrophage-related disorder refers to inflammatory disorders including but not limited to inflammatory diseases of the gastrointestinal tract such as Crohn's disease, inflammatory bowel disease, gastritis, colitis, ulcerative colitis, colon irritable gastric ulcer and duodenal ulcer, inflammatory diseases of the skin such as psoriasis, inflammatory diseases of the respiratory system such as asthma, allergic rhinitis or chronic obstructive pulmonary disease (COPD), inflammatory diseases of the musculoskeletal system such as rheumatoid arthritis, or neuritis, renal inflammation and myocarditis

More preferably, the term activated-macrophage-related disorder refers to respiratory inflammatory disorders including but not limited to asthma, allergic rhinitis or chronic obstructive pulmonary disease (COPD).

More preferably, the term activated-macrophage-related disorder refers to chronic obstructive pulmonary disease (COPD).

**Biomarker**

The term "biomarker", as used herein refers to a biological macromolecule which expression and/or activity level is correlated with a biological phenomenon. The term "biomarker for macrophage activation", as used herein, refers to a biological macromolecule that presents expression and/or activity level changes upon macrophage activation. In the context of the invention, this term refers more particularly to a gene which expression and/or activity level increases upon macrophage activation.

This term usually refers to a gene which expression and/or activity level increases by approximately 1.7 fold to 1000-fold, preferably 10-fold to 100-fold, more preferably 20-fold to 50-fold in activated macrophages compared to non activated macrophages.

This term may also refers to a biological molecule which expression and/or activity level changes upon macrophage activation but not, or to a lesser extent, or not in the same direction during monocyte activation. In particular, this term refers more particularly to a gene which expression and/or activity level increases upon macrophage activation but not, or to a lesser extent, or decreases in monocyte activation. Preferably, this term refers to biomarkers defined using criteria for classifying genes specific of macrophage activation as detailed in Example 1.

RGS2

The term «RGS2», as used herein, refers to RGS2 expression products, i.e. RGS2 polynucleotides and polypeptides, and fragments thereof.

RGS2 polypeptides

The term "RGS2 polypeptide or RGS2 protein", when used herein, encompasses all RGS2 polypeptides, preferably from mammalian species, more preferably from human and rodent species, as well as their variants, analogs, orthologs, homologs, and derivatives, and fragments thereof that are characterized by their ability to exhibit a RGS2 biological activity as defined below. Illustrating but not limiting examples of amino acid sequences of such RGS2 polynucleotides may be found in Siderovski *et al* (1994) *supra,* Chen *et al.* (1997) *supra,* and Miles *et al.* (2000) *supra* as well as in sequence databases such as Genseq, Swissprot, Genbank, Embl, and PIR.

Preferably, the term "RGS2 polypeptide or protein" refers preferably to a mammalian RGS2 polypeptide, more preferably a human or rodent RGS2 polypeptide, as well as its variants, homologs and derivatives exhibiting essentially the same RGS2 biological activity.

The term "RGS2 polypeptide variants", as used herein, refers to polypeptides from the same species but differing from a reference RGS2 polypeptide. Generally, differences are limited so that the amino acid sequences of the reference and the variant are closely similar overall and, in many regions, identical. Preferably, RGS2 polypeptides are at least 70%, 80%, 90%, 95%, 96%, 97%, 98% or 99% identical to a reference RGS2 polypeptide, preferably a mammalian RGS2 polypeptide, more preferably a human or rodent RGS2 polypeptide. By a polypeptide having an amino acid sequence at least, for example, 95% "identical" to a query amino acid sequence, it is intended that the amino acid sequence of the subject polypeptide is identical to the query sequence except that the subject polypeptide sequence may include up to five amino acid alterations per each 100 amino acids of the query amino acid sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence. The query sequence may be an entire amino acid sequence of the reference sequence or any fragment specified as described herein.

Such RGS2 polypeptide variants may be naturally occurring variants, such as naturally occurring allelic variants encoded by one of several alternate forms of a gene occupying a given locus on a chromosome of an organism, or isoforms encoded by naturally occurring splice variants originating from a single primary transcript. Alternatively, a RGS2 polypeptide variant may be a variant that is not known to occur naturally and that can be made using art-known mutagenesis techniques.

It is known in the art that one or more amino acids may be deleted from the N-terminus or C-terminus of a bioactive peptide or protein without substantial loss of biological function (see for instance, Ron *et al.,* (1993), Biol Chem., 268 2984-2988 ; which disclosure is hereby incorporated by reference in its entirety).

It also will be recognized by one of ordinary skill in the art that some amino acid sequences of RGS2 polypeptides can be varied without significant effect of the structure or function of the protein. Such mutants include deletions, insertions, inversions, repeats, and substitutions selected according to general rules known in the art so as to have little effect on activity. For example, guidance concerning how to make phenotypically silent amino acid substitutions is provided in Bowie *et al.* (1990), Science 247:1306-1310, hereby incorporated by reference in its entirety, wherein the authors indicate that there are two main approaches for studying the tolerance of an amino acid sequence to change.

The first method relies on the process of evolution, in which mutations are either accepted or rejected by natural selection. The second approach uses genetic engineering to introduce amino acid changes at specific positions of a cloned gene and selections or screens to identify sequences that maintain functionality. These studies have revealed that proteins are surprisingly tolerant of amino acid substitutions. The authors further indicate which amino acid changes are likely to be permissive at a certain position of the protein. For example, most buried amino acid residues require nonpolar side chains, whereas few features of surface side chains are generally conserved. Other such phenotypically silent substitutions are described in Bowie *et al.,* (1990) supra, and the references cited therein.

Typically seen as conservative substitutions are the replacements, one for another, among the aliphatic amino acids Ala, Val, Leu and Phe; interchange of the hydroxyl residues Ser and Thr, exchange of the acidic residues Asp and Glu, substitution between the amide residues Asn and Gln, exchange of the basic residues Lys and Arg and replacements among the aromatic residues Phe, Tyr. In addition, the following groups of amino acids generally represent equivalent changes: (1) Ala, Pro, Gly, Glu, Asp, Gln, Asn, Ser, Thr; (2) Cys, Ser, Tyr, Thr; (3) Val, Ile, Leu, Met, Ala, Phe; (4) Lys, Arg, His; (5) Phe, Tyr, Trp, His.

The term RGS2 polypeptide also encompasses all RGS2 polypeptides encoded by RGS2 gene analogs, orthologs, and/or species homologues. As used herein, the term "gene analogs" refers to genes of different and unrelated organisms which perform the same functions in each organism but which did not originate from an ancestral structure that the organisms' ancestors had in common. Instead, analogous genes arose separately and then later evolved to perform the same function (or similar functions). In other words, analogous RGS2 polypeptides are polypeptides with quite different amino acid sequences but that perform the same biological activity, namely a RGS2 biological activity. As used herein, the term "gene orthologs" refers to genes within two different species which sequence are related to each other via a common homologous gene in an ancestral species but which have evolved to become different from each other. As used herein, the term "gene homologs" refers to genes of different organisms which perform the same functions in each organism and which originate from an ancestral structure that the organisms' ancestors had in common. In other words, homologous RGS2 polypeptides are polypeptides with quite similar amino acid sequences that perform the same biological activity, namely a RGS2 biological activity. Preferably, RGS2 polypeptide homologs may be defined as polypeptides exhibiting at least 40%, 50%, 60%, 70%, 80%, 90%, 95%, 96%, 97%, 98% or 99% identity to a reference RGS2 polypeptide, preferably a mammalian RGS2 polypeptide, more preferably a human or rodent RGS2 polypeptide.

As used herein, the term "RGS2 polypeptide derivatives" refers to modified RGS2 polypeptides during or after translation, e.g., by glycosylation, acetylation, phosphorylation, amidation, derivatization by known protecting/blocking groups, proteolytic cleavage, linkage to an antibody molecule or other cellular ligand, and the like. Any of numerous chemical modifications may be carried out by known techniques, including but not limited, to specific chemical cleavage by cyanogen bromide, trypsin, chymotrypsin, papain, V8 protease, NaBH4; acetylation, formylation, oxidation, reduction; and metabolic synthesis in the presence of tunicamycin.

Additional post-translational modifications encompassed by the invention include, for example, e.g., N-linked or O-linked carbohydrate chains, processing of N-terminal or C-terminal ends), attachment of chemical moieties to the amino acid backbone, chemical modifications of N-linked or O-linked carbohydrate chains, and addition or deletion of an N-terminal methionine residue as a result of prokaryotic host cell expression. The polypeptides may also be modified with a detectable label, such as an enzymatic, fluorescent, isotopic or affinity label to allow for detection and isolation of the protein.

Also provided by the invention are chemically modified derivatives of RGS2 polypeptides that may provide additional advantages such as increased solubility, stability and circulating time of the polypeptide, or decreased immunogenicity See U.S. Patent No: 4,179,337, hereby incorporated by reference in its entirety. The chemical moieties for derivatization may be selected from water soluble polymers such as polyethylene glycol, ethylene glycollpropylene glycol copolymers, carboxymethylcellulose, dextran, polyvinyl alcohol and the like. The polypeptides may be modified at random positions within the molecule, or at predetermined positions within the molecule and may include one, two, three or more attached chemical moieties.

Thus, a RGS2 polypeptide according to the invention may be, for example: (i) one in which one or more of the amino acid residues are substituted with a conserved or non-conserved amino acid residue (preferably a conserved amino acid residue) and such substituted amino acid residue may or may not be one encoded by the genetic code: or (ii) one in which one or more of the amino acid residues includes a substituent group: or (iii) one in which the RGS2 polypeptide is fused with another compound, such as a compound to increase the half-life of the polypeptide (for example, polyethylene glycol): or (iv) one in which the additional amino acids are fused to the above form of the polypeptide, such as an IgG Fc fusion region peptide or leader or secretory sequence or a sequence which is employed for purification of the above form of the polypeptide or a pro-protein sequence.

RGS2 polypeptides may be monomers or multimers. Multimers may be dimers, trimers, tetramers or multimers comprising at least five monomeric polypeptide units. Multimers may also be homodimers or heterodimers. Multimers of the invention may be the result of hydrophobic, hydrophilic, ionic and/or covalent associations and/or may be indirectly linked, by for example, liposome formation. In one example, covalent associations are between the heterologous sequences contained in a fusion protein containing a RGS2 polypeptide or fragment thereof (see, e.g., US Patent Number 5,478,925, which disclosure is hereby incorporated by reference in its entirety). In another example, a RGS2 polypeptide or fragment thereof is joined to one or more polypeptides that may be either RGS2 polypeptides or heterologous polypeptides through peptide linkers such as those described in U.S. Pat. No. 5,073,627 (hereby incorporated by reference). Another method for preparing multimer RGS2 polypeptides involves use of RGS2 polypeptides fused to a leucine zipper or isoleucine zipper polypeptide sequence known to promote multimerization of the proteins in which they are found using techniques known to those skilled in the art including the teachings of WO 94/10308. In another example, RGS2 polypeptides may be associated by interactions between Flag® polypeptide sequence contained in fusion RGS2 polypeptides containing Flag® polypeptide sequence. RGS2 multimers may also be generated using chemical techniques known in the art such as cross-linking using linker molecules and linker molecule length optimization techniques known in the art (see, e.g., US 5,478,925), techniques known in the art to form one or more inter-molecule cross-links between the cysteine residues located within the sequence of the polypeptides desired to be contained in the multimer (see, e.g., US 5,478,925, addition of cysteine or biotin to the C terminus or N-terminus of RGS2 polypeptide and techniques to generate multimers containing one or more of these modified polypeptides (see, e.g., US 5,478,925), or any of the 30 techniques to generate liposomes containing RGS2 multimers (see, e.g., US Patent Number 5,478,925,), which disclosures are incorporated by reference in their entireties.

As used herein, the term "RGS2 polypeptide fragment" refers to any peptide or polypeptide comprising a contiguous span of part of the amino acid sequence of a reference RGS2 polypeptide, preferably a mammalian RGS2 polypeptide, more preferably a human or rodent RGS2 polypeptide.

More specifically, a RGS2 polypeptide fragment comprising at least 6, preferably at least 8 to 10, more preferably 12, 15, 20, 25, 30, 35, 40, 50, 60, 75, 100, 125, 150, 175, or 200 consecutive amino acids of a RGS2 polypeptide according to the present invention. RGS2 polypeptide fragment may additionally be described as sub-genuses of RGS2 polypeptides comprising at least 6 amino acids, wherein "at least 6" is defined as any integer between 6 and the integer representing the C-terminal amino acid of a RGS2 polypeptide, preferably of a mammalian RGS2 polypeptide, more preferably of a human or rodent RGS2 polypeptide. Further included are species of RGS2 polypeptide fragments at least 6 amino acids in length, as described above, that are further specified in terms of their N-terminal and C-terminal positions. Also encompassed by the term "RGS2 polypeptide fragment" as individual species are all RGS2 polypeptide fragments, at least 6 amino acids in length, as described above, that may be particularly specified by a N-terminal and C-terminal position. That is, every combination of a N-terminal and C-terminal position that a fragment at least 6 contiguous amino acid residues in length could occupy, on any given amino acid sequence of the sequence listing or of the present invention is included in the present invention.

It is noted that the above species of polypeptide fragments of the present invention may alternatively be described by the formula "a to b"; where "a" equals the N-terminal most amino acid position and "b" equals the C-terminal most amino acid position of the polynucleotide; and further where "a" equals an integer between I and the number of amino acids of a RGS2 polypeptide sequence minus 6, and where "b" equals an integer between 7 and the number of amino acids of the RGS2 polypeptide sequence; and where "a" is an integer smaller then "b" by at least 6.

The above RGS2 polypeptide fragments can be immediately envisaged using the above description and are therefore not individually listed solely for the purpose of not unnecessarily lengthening the specification. Moreover, the above fragments do not necessarily need to have a RGS2 biological activity, although polypeptides having these activities are preferred embodiments of the invention, since they would be useful, for example, in immunoassays, in epitope mapping, epitope tagging, as vaccines, and as molecular weight markers. The above fragments may also be used to generate antibodies to a particular portion of the polypeptide

Also encompassed by the term "RGS2 polypeptide fragment" are domains of RGS2 polypeptides. Such domains may eventually comprise linear or structural motifs and signatures including, but not limited to, leucine zippers, helix-turn-helix motifs, post-translational modification sites such as glycosylation sites, ubiquitination sites, alpha helices, and beta sheets, signal sequences encoding signal peptides which direct the secretion of the encoded proteins, sequences implicated in transcription regulation such as homeoboxes, acidic stretches, enzymatic active sites, substrate binding sites, and enzymatic cleavage sites. Such domains may present a particular biological activity such as DNA or RNA-binding, secretion of proteins, transcription regulation, enzymatic activity, substrate binding activity, etc...

A domain has a size generally comprised between 3 and 1000 amino acids. In preferred embodiment, domains comprise a number of amino acids that is any integer between 6 and 200. Domains may be synthesized using any methods known to those skilled in the art, including those disclosed herein for the preparation of RGS2 polypeptides to produce anti-RGS2 antibodies. Methods for determining the amino acids that make up a domain with a particular biological activity include mutagenesis studies and assays to determine the biological activity to be tested.

Particularly preferred fragments in the context of the present invention are RGS2 polypeptides retaining a substantial biological activity, namely a RGS2 biological activity, preferably the induction of a GTPase activity, more preferably the ability to modulate $G_\alpha$ activity, even more preferably the ability to modulate $G_q$ activity.

Preferred fragments are RGS2 fragments containing the RGS domain defined in Chatterjee and Fisher (2000) *supra* and in Zheng *et al.* (1999) *supra* or fragments containing the three regions thought to be important for interaction with $G_\alpha$ (Heximer *et al. (1999) supra).*

Illustrating but not limiting examples of preferred fragments are the following:

- A N-terminus truncated human RGS2 protein lacking amino acid 1 to 4, 1 to 15 or 1 to 32 amino acid as described in Heximer *et al.* (2001) *supra,*
- A mutated human RGS2 protein (L45D) as described in Heximer *et al.* (2001) *supra,*
- A mutated human RGS2 protein (K42A/R44A) as described in Heximer *et al.* (2001) *supra,*
- A mutated human RGS2 protein (W41A/L45A/F48A/L49A) as described in Heximer *et al.* (2001) *supra,*
- A RGS2 triple mutant human protein (C106S, N184D, E191K) as described in Heximer *et al. (1999) supra.*

Alternatively, the polypeptides of the invention may be scanned for motifs, domains and/or signatures in databases using any computer method known to those skilled in the art. Searchable databases include Prosite (Hofmann et al., (1999) Nucl. Acids Res. 27:215-219; Bucher and Bairoch (1994) Proceedings 2nd International Conference on Intelligent Systems for Molecular Biology. Altman et al, Eds., pp53-61, AAAIPress, Menlo Park), Pfam (Sonnhammer et al., (1997) Proteins. 28(3):405-20; Henikoff et al., (2000) Nucleic Acids Res. 28(1):228-30; Bateman et al., (2000) Nucleic Acids Res. 28(1):263-6), Blocks (Henikoff et al., (2000) Electrophoresis 21(9):1700-6), Print (Attwood et al., (1996) Nucleic Acids Res. 24(1):182-8), Prodom (Sonnhammer and Kahn (1994) Protein Sci. 3(3):482-92; Corpet et al. (2000) Nucleic Acids Res. 28(1):267-9), Sbase (Pongor et al. (1993) Protein Eng. 6(4):391-5; Murvai et al., (2000) Nucleic Acids Res. 28(1):260-2), Smart (Schultz et al., (1998) Proc Natl Acad Sci U S A 95, 5857-5864), Dali/FSSP (Holm and Sander (1996) Nucleic Acids Res. 24(1):206-9 ; Holm and Sander (1997) Nucleic Acids Res. 25(1):231-4 ; Holm and Sander (1999) Nucleic Acids Res. 27(1):244-7), HSSP (Sander and Schneider (1991) Proteins. 9(1):56-68.), CATH (Orengo *et al.,* (1997) Structure. 5(8):1093-108; Pearl *et al.,* (2000) Biochem Soc Trans. 28(2):269-75), SCOP (Murzin *et al.,* (1995) J Mol Biol. 247(4):536-40; Lo Conte *et al.,* (2000) Nucleic Acids Res. 28(1):257-9), COG (Tatusov *et al.,* (1997) Science, 278, 631 :637 ; Tatusov *et al.,* (2000) Nucleic Acids Res. 28(1):33-6), specific family databases and derivatives thereof (Nevill-Manning *et al.,* (1998) Proc. Natl. Acad. Sci. U S A. 95, 5865-5871; Yona *et al.,* (1999) Proteins. 37(3):360-78; Attwood *et al.,* (2000) Nucleic Acids Res. 28(1):225-7), each of which disclosures are hereby incorporated by reference in their entireties. For a review on available databases, see issue 1 of volume 28 of Nucleic Acid Research (2000), which disclosure is hereby incorporated by reference in its entirety.

The term "RGS2 polypeptide fragment" also encompasses epitopes-bearing fragments. These epitopes may be antigenic epitopes or both an antigenic epitope and an immunogenic epitope. An immunogenic epitope is defined as a part of a protein that elicits an antibody response *in vivo* when the polypeptide is the immunogen. On the other hand, a region of polypeptide to which an antibody binds is defined as an antigenic epitope. An epitope can comprise as few as 3 amino acids in a spatial conformation, which is unique to the epitope. Generally an epitope consists of at least 6 such amino acids, and more often at least 8-10 such amino acids.

A RGS2 epitope-bearing fragment according to the invention may be any fragment which length is between 6 amino acid and the full-length sequence of a RGS2 polypeptide, preferably a fragment between 6 and 50 amino acid. The epitope-bearing fragments may be specified by either the number of contiguous amino acid residues (as a subgenus) or by specific N-terminal and C-terminal positions (as species) as described above.

Fragments which function as epitopes may be produced by any conventional means *(See, e.g.,* Houghten (1985), Proc. Natl. Acad. Sci. USA 82:5131-5135 and U.S. 4,631,21, which disclosures are hereby incorporated by reference in their entireties). Methods for determining the amino acids which make up an epitope include x-ray crystallography, 2-dimensional nuclear magnetic resonance, and epitope mapping, e.g., the Pepscan method described by Geysen *et al.,* (1984), Proc. Natl. Acad. Sci. U.S.A. 81:3998-4002; PCT Publications WO 84/03564 and WO 84/03506, which disclosures are hereby incorporated by reference in their entireties. Another example is the algorithm of Jameson and Wolf, (1988), Comp. Appl. Biosci 4:181-186 (said reference incorporated by reference in its entirety). The Jameson-Wolf antigenic analysis, for example, may be performed using the computer program PROTEAN, using default parameters (Version 4.0 Windows, DNASTAR, Inc.)

The present invention also provides for the exclusion of any RGS2 fragment species specified by N-terminal and C-terminal positions or of any fragment sub-genus specified by size in amino acid residues as described above. Any number of fragments specified by N-terminal and C-terminal positions or by size in amino acid residues as described above may be excluded as individual species. The present invention also provides for the exclusion of any RGS2 domain or epitope-bearing fragment in the same manner.

The RGS2 polypeptides of the present invention can be prepared in any suitable manner Such RGS2 polypeptides and fragments thereof may be purified from natural sources, chemically synthesized, produced by recombinant techniques including *in vitro* translation techniques or expression in a recombinant cell able to express RGS2 cDNA, or a combination of these methods, using techniques known to those skilled in the art (See, for example, "Methods in Enzymology, Academic Press, 1993" for a variety of methods for purifying proteins; Creighton, (1983) Proteins: Structures and Molecular Principles, W.H. Freeman & Co. 2nd Ed., T. E., New York; and Hunkapiller *et al.,* (1984) Nature. 310(5973): 105-11 for chemical synthesis of proteins and Davis *et al.* (1986) Basic Methods in Molecular Biology, ed., Elsevier Press, NY for recombinant techniques, which disclosures are incorporated by reference in their entireties). The polypeptides of the present invention are preferably provided in an isolated form, and may be partially or preferably substantially purified.

RGS2 biological activity

As used herein, the terms "RGS2 biological activity" and "RGS2 activity" refer to all activities exhibited by RGS2 polypeptides including but not limited to induction of GTPase activity, modulation of $G_{\alpha}$ activity, modulation of adenyl cyclase activity, or modulation of COPI binding to Golgi. The term "RGS2 activity" also encompasses any biological activity induced subsequently to induction of GTPase activity, modulation of $G_{\alpha}$ activity, modulation of adenyl cyclase activity, or modulation of COPI binding to Golgi, by RGS2 such as regulation of odorant-evoked intracellular signaling, modulation of M-type potassium currents in sympathetic neurons, or modulation of glucose-dependent insulinotropic peptide-mediated insulin secretion. Preferably, these terms refer to induction of GTPase activity. In a specific embodiment, these terms refer to modulation of $G_{\alpha}$ activity, preferably to inhibition of $G_{\alpha}$ activity. In another specific embodiment, these terms refer to modulation of $G_q$ activity, preferably to inhibition of $G_q$ activity. RGS2 activities may be measured using any assay known to those skilled in the art including those described elsewhere in the application.

RGS2 polynucleotides

The term "RGS2 polynucleotide", when used herein, encompasses all polynucleotides, including genomic DNA, mRNA and cDNA nucleic acid molecules, encoding a RGS2 polypeptide as defined below, preferably from mammalian species, more preferably from human and murine species, as well as their variants, analogs, orthologs, homologs, and derivatives, and fragments thereof. Illustrating but not limiting examples of nucleic acid sequences of such RGS2 polynucleotides may be found in sequence databases such as Genbank, EMBL, OMIM, Genseq, RefSeqn, Unigene or in the following publication: Siderovski *et al.* (1994) *supra.*

In a preferred embodiment, the term "RGS2 polynucleotide" refers to polynucleotides encoding a mammalian RGS2 polypeptide, preferably a human or rodent RGS2 polypeptide, as well as its variants and derivatives exhibiting essentially the same biological activity, namely a RGS2 biological activity.

As used herein, the term "RGS2 polynucleotides variant" refers to polynucleotides from the same species but differing from a reference polynucleotide. Generally, differences are limited so that the nucleotide sequences of the reference and the variant are closely similar overall and, in many regions, identical. Preferably, RGS2 polynucleotides are at least 50%, 60%, 70%, 80%, 90%, 95%, 96%, 97%, 98% or 99% identity to a reference RGS2 polynucleotide, preferably a mammalian RGS2 polynucleotide, more preferably a human or rodent RGS2 polynucleotide. By a polynucleotide having a nucleotide sequence at least, for example, 95% "identical" to a reference nucleotide sequence of the present invention, it is intended that the nucleotide sequence of the polynucleotide is identical to the reference sequence except that the polynucleotide sequence may include up to five point mutations per each 100 nucleotides of the reference nucleotide sequence encoding the RGS2 polypeptide. The query sequence may be an entire nucleic sequence of the reference polynucleotides, or the ORF (open reading frame) of the reference polynucleotide, or any fragment specified as described herein.

Such RGS2 polynucleotide variants may be naturally occurring variants, such as naturally occurring allelic variants being one of several alternate forms of a gene occupying a given locus on a chromosome of an organism, or naturally occurring splice variants originating from a single primary transcript. Alternatively, a RGS2 polynucleotide variant may be a variant that is not known to occur naturally and that can be made using art-known mutagenesis techniques.

Such RGS2 polynucleotides variants may be degenerate variants, i.e. polynucleotides that comprise a sequence substantially different from those described above but which, due to the degeneracy of the genetic code, still encode a RGS2 polypeptide according to the present invention. That is, all possible polynucleotide sequences that encode the RGS2 polypeptides of the present invention are completed. This includes the genetic code and species-specific codon preferences known in the art. Thus, it would be routine for one skilled in the art to generate the degenerate variants described above, for instance, to optimize codon expression for a particular host (e.g., change codons in the human mRNA to those preferred by other mammalian or bacterial host cells).

Nucleotide changes present in a variant polynucleotide may be silent, which means that they do not alter the amino acids encoded by the polynucleotide. However, nucleotide changes may also result in amino acid substitutions, additions, deletions, fusions and truncations in the polypeptide encoded by the reference sequence. The substitutions, deletions or additions may involve one or more nucleotides. The variants may be altered in coding or non-coding regions or both. Alterations in the coding regions may produce conservative or non-conservative amino acid substitutions, deletions or additions. In the context of the present invention, preferred embodiments are those in which the polynucleotide variants encode RGS2 polypeptides that retain substantially the same biological properties or activities as the RGS2 protein. More preferred polynucleotide variants are those containing conservative substitutions.

The term RGS2 polynucleotides also encompasses RGS2 gene analogs, orthologs, and/or species homologues as defined above. Preferably, RGS2 polynucleotide homologs may be defined as polynucleotides exhibiting at least 50%, 60%, 70%, 80%, 90%, 95%, 96%, 97%, 98% or 99% identity to a reference RGS2 polynucleotide, preferably a mammalian RGS2 polynucleotide, more preferably a human or rodent RGS2 polynucleotide .

As used herein, the term "RGS2 polynucleotide fragment" refers to any oligonucleotide or polynucleotide comprising a contiguous span of part of the nucleic acid sequence of a reference RGS2 polynucleotide, preferably a mammalian RGS2 polynucleotide, more preferably a human or rodent RGS2 polynucleotide.

More specifically, a RGS2 polynucleotide fragment comprising at least 8, preferably at least 10, 12, 15, or 18, more preferably at least 20, 25, 28, 30, 35, 40, 50, 75, 100, 150, 200, 300, 400, 500, 800, 1000, 1500, 2000 or 5000 nucleotides of a RGS2 polynucleotide according to the present invention. RGS2 polynucleotide fragment may additionally be described as sub-genuses of RGS2 polynucleotides comprise at least 8 nucleotides, wherein "at least 8" is defined as any integer between 8 and the integer representing the 3' most nucleotide position of a RGS2 polynucleotide, preferably a mammalian RGS2 polynucleotide, more preferably a human or rodent RGS2 polynucleotide.

Further included are species of RGS2 polynucleotide fragments at least 8 nucleotides in length, as described above, that are further specified in terms of their 5' most and 3' most positions. Also encompassed by the term "RGS2 polynucleotide fragment" as individual species are all RGS2 polynucleotide fragments, at least 8 nucleotides in length, as described above, that may be particularly specified by a 5' most and 3' most nucleotide position. That is, every combination of a 5' most and 3' most nucleotide position that a fragment at least 8 contiguous nucleotide residues in length could occupy, on any given nucleic acid sequence of the sequence listing or of the present invention is included in the present invention. The 5' and 3' positions are represented by the position numbers set forth in the appended sequence listing. For allelic, degenerate and other variants, position I is defined as the 5' most nucleotide of the ORF, i.e., the nucleotide "A" of the start codon with the remaining nucleotides numbered consecutively. Therefore, every combination of a 5' and 3' nucleotide position that a polynucleotide fragment of the present invention, at least 8 contiguous nucleotides in length, could occupy on a polynucleotide of the invention is included in the invention as an individual species. The above RGS2 polynucleotide fragments can be immediately envisaged using the above description and are therefore not individually listed solely for the purpose of not unnecessarily lengthening the specification.

It is noted that the above species of RGS2 polynucleotide fragments of the present invention may alternatively be described by the formula "a to b"; where "a" equals the 5' most nucleotide position and "b" equals the 3' most nucleotide position of the RGS2 polynucleotide; and further where "a" equals an integer between 1 and the number of nucleotides of the polynucleotide sequence of the present invention minus 8, and where "b" equals an integer between 9 and the number of nucleotides of the polynucleotide sequence of the present invention; and where "a" is an integer smaller then "b" by at least 8.

Also encompassed by the term "RGS2 polynucleotide fragment" are polynucleotides comprising polynucleotides encoding domains of RGS2 polypeptides or polynucleotides comprising epitope-bearing fragments, as defined above.

The present invention also provides for the exclusion of any RGS2 fragment species specified by 5' most and 3' most nucleotide or of any fragment sub-genus specified by size in nucleotide residues as described above. Any number of fragments specified by 5' most and 3' most nucleotide positions or by size in nucleotide residues as described above may be excluded as individual species. The present invention also provides for the exclusion of any polynucleotides fragment encoding a RGS2 domain or epitope-bearing fragment in the same manner.

**Identity Between Polynucleotides Or Polypeptides**

The terms "polynucieotides having at least x% identity with a polynucleotides of reference" and "polypeptides having at least x% identity with a polypeptide of reference" encompass polynucleotides or polypeptides which residue (nucleotide or amino acid respectively) sequence exhibit an identity percentage, as defined below, equal or superior to x compared to said reference polynucleotide or polypeptide sequence, respectively.

The identity percentage is determined after optimal alignment of two polynucleotides or polypeptide sequences over a comparison window, wherein portions of the polynucleotide or polypeptide sequences in the comparison window may comprise additions or deletions of one or more residue in order to optimize sequence alignment. The comparison window contains a certain number of positions (either a residue or a gap corresponding to an insertion/deletion of a residue), this number of positions corresponding to the window size. Each window position may present one of the following situations:

1°/ There is a residue (nucleotide or amino acid) on this position on the first aligned sequence and a different residue at the same position on the second aligned sequence, in other words the second sequence has a substituted residue at this position compared to the first sequence.

2°/ There is a residue (nucleotide or amino acid) on this position on the first aligned sequence and the same residue at the same position on the second aligned sequence.

3°/ There is a residue (nucleotide or amino acid) on this position on the first aligned sequence and no residue at the same position on the second aligned sequence, in other words the second sequence presents a deletion at this position compared to the first sequence.

The number of positions within the comparison window belonging to the first above-defined category is called R1.

The number of positions within the comparison window belonging to the second above-defined category is called R2.

The number of positions within the comparison window belonging to the third above-defined category is called R3.

The identity percentage (%id) is may be calculated by any of the following formulas:

$$\%id=R2/(R1+R2+R3)\times100,$$

or

$$\%id=(R2+R3)/(R1+R2+R3)\times100$$

$$\%id=R2/(\text{total length of reference sequence})\times100,$$

or

$$\%id=(R2+R3)/(\text{total length of reference sequence})\times100$$

Alignment of sequences to compare may be performed using any of the variety of sequence comparison algorithms and programs known in the art. Such algorithms and programs include, but are by no means limited to, TBLASTN, BLASTP, FASTA, TFASTA, FASTDB, WU-BLAST, Gapped-BLAST, PSI-BLAST (Pearson and Lipman, (1988), Proc. Natl. Acad. Sci. USA 85:2444-2448; Altschul et al., (1990), J. Mol. Biol. 215:403-410; Altschul et al., (1993), Nature Genetics 3:266-272; Altschul et al., (1997), Nuc. Acids Res. 25:3389-3402; Thompson et al., (1994), Nuc. Acids Res.. 22:4673-4680; Higgins et al., (1996), Meth. Enzymol. 266:383-402; Brutlag et al. (1990) Comp. App. Biosci. 6:237-245; Jones and Swindells, (2002) Trends Biochem Sci 27:161-4; Olsen et al. (1999) Pac Symp Biocomput; 302-13), the disclosures of which are incorporated by reference in their entireties.

In a particular embodiment, the Smith-Waterman method is used with scoring matrix such as PAM, PAM 250 or preferably with BLOSUM matrices such as BLOSUM60 or BLOSUM62 and with default parameters (Gap Opening Penalty=10 and Gap Extension Penalty=1) or with user-specified parameters preferably superior to default parameters.

In another particular embodiment, protein and nucleic acid sequences are aligned using the Basic Local Alignment Search Tool ("BLAST") programs with the default parameters or with modified parameters provided by the user. Preferably, the scoring matrix used is the BLOSUM62 matrix (Gonnet *et al.,* (1992), Science 256:1443-1445; Henikoff and Henikoff, (1993), Proteins 17:49-61, which disclosures are hereby incorporated by reference in their entireties). Less preferably, the PAM or PAM250 matrices may also be used (see, e.g., Schwartz and Dayhoff, (1978), eds., Matrices for Detecting Distance Relationships: Atlas of Protein Sequence and Structure, Washington: National Biomedical Research Foundation, which disclosure is hereby incorporated by reference in its entirety).

In still another particular embodiment, polynucleotide or polypeptide sequences are aligned using the FASTDB computer program based on the algorithm of Brutlag *et al.* (1990), *supra.* Preferred parameters used in a FASTDB alignment of DNA sequences are: Matrix=Unitary, k-tuple=4, Mismatch Penalty= 1, Joining Penalty=30, Randomization Group Length=0, Cutoff Score= 1, Gap Penalty=5, Gap Size Penalty 0.05, Window Size=500 or the length of the subject nucleotide sequence, whichever is shorter. Preferred parameters used in a FASTDB amino acid alignment are: Matrix=PAM 0, k-tuple=2, Mismatch Penalty= 1, Joining Penalty=20, Randomization Group25Length=0, Cutoff Score= 1, Window Size=sequence length, Gap Penalty=5, Gap Size Penalty=0.05, Window Size=500 or the length of the subject amino acid sequence, whichever is shorter.

**Polynucleotide**

As used interchangeably herein, the terms "nucleic acid molecules)", "oligonucleotide(s)", and "polynucleotide (s)" include RNA or DNA (either single or double stranded, coding, complementary or antisense), or RNA/DNA hybrid sequences of more than one nucleotide in either single chain or duplex form (although each of the above species may be particularly specified). The term "nucleotide" is used herein as an adjective to describe molecules comprising RNA, DNA, or RNA/DNA hybrid sequences of any length in single-stranded or duplex form. More precisely, the expression "nucleotide sequence" encompasses the nucleic material itself and is thus not restricted to the sequence information (i.e. the succession of letters chosen among the four base letters) that biochemically characterizes a specific DNA or RNA molecule.

The term "nucleotide" is also used herein as a noun to refer to individual nucleotides or varieties of nucleotides, meaning a molecule, or individual unit in a larger nucleic acid molecule, comprising a purine or pyrimidine, a ribose or deoxyribose sugar moiety, and a phosphate group, or phosphodiester linkage in the case of nucleotides within an oligonucleotide or polynucleotide. The term "nucleotide" is also used herein to encompass "modified nucleotides" which comprise at least one modifications such as (a) an alternative linking group, (b) an analogous form of purine, (c) an analogous form of pyrimidine, or (d) an analogous sugar. For examples of analogous linking groups, purine, pyrimidines, and sugars see for example PCT publication No WO 95/04064, which disclosure is hereby incorporated by reference in its entirety.

Polynucleotides of the invention, including RGS2 polynucleotides or fragments thereof, can be obtained using methods that comprise sequentially linking together nucleotides to produce the nucleic acids having the desired sequences. Polynucleotides of the invention may be synthesized enzymatically using genetic engineering techniques well known to those skilled in the art including cloning and restriction of appropriate sequences, amplification-based methods, hybridization-based methods as described herein. Alternatively, a variety of chemical methods of synthesizing nucleic acids are known to those skilled in the art including but not limited to direct chemical synthesis by a method such as the phosphodiester method of Narang et al, (1979) Methods Enzymol 68:90-98, the phosphodiester method of Brown *et al.,* (1979), *Meth. Enzymol.* 68:109-151, the diethylphosphoramidite method of Beaucage *et al.,* (1981) *Tetrahedron Lett,* 22: 1859-1862 and the solid support method described in EP 0 707 592, which disclosures are hereby incorporated by reference in their entireties. In many of these methods, synthesis is conducted on a solid support. These included the 3' phosphoramidite methods in which the 3' terminal base of the desired oligonucleotide is immobilized on an insoluble carrier. The nucleotide base to be added is blocked at the 5' hydroxyl and activated at the 3' hydroxyl so as to cause coupling with the immobilized nucleotide base. Deblocking of the new immobilized nucleotide compound and repetition of the cycle will produce the desired polynucleotide. Alternatively, polynucleotides may be prepared as described in U.S. Patent No. 5,049,656, which disclosure is hereby incorporated by reference in its entirety. In some embodiments, several polynucleotides prepared as described above are ligated together to generate longer polynucleotides having a desired sequence.

**Anti-RGS2 antibodies**

As used herein, "anti-RGS2 antibody" is meant to include whole antibodies, including single-chain whole antibodies, antigen binding fragments thereof, and any functional or recombinant derivatives thereof, which are capable of binding the whole RGS2 polypeptide or a RGS2 epitopic determinant. Antigen binding antibody fragments include, but are not limited to, Fab, Fab' F(ab)2 and F(ab')2, Fd, single-chain Fvs (scFv), single-chain antibodies, disulfide-linked Fvs (sdFv) and fragments comprising either a $V_L$ or $V_H$ domain. Antigen-binding antibody fragments, including single-chain antibodies, may comprise the variable regiori(s alone or in combination with the entire or partial of the following: hinge region, CH1, CH2, and CH3 domains. Also encompassed by the anti-RGS2 antibody term are any combinations of variable regiori(s and hinge region, CH1, CH2, and CH3 domains. Such anti-RGS2 antibody may be a hybrid, chimeric, humanized, univalent, monoclonal or polyclonal antibody. It may be monospecific, bispecific, trispecific or have greater multispecificity. Multispecific antibodies may be specific for different epitopes of a RGS2 or may be specific for both a polypeptide of the present invention as well as for heterologous compositions, such as a heterologous polypeptide or solid support material. *See, e.g,* WO 93/17715; WO 92/08802; WO 91/00360; WO 92/05793; Tutt, *et al.* (1991), J. Immunol. 147:60-69; US Patents 5,573,920, 4,474,893, 5,601,819, 4,714,681, 4,925,648; Kostelny *et al.* (1992),), J. Immunol. 148:1547-1553, which disclosures are hereby incorporated by reference in their entireties.

## II. Uses of a biomarker

As described in the Example section, Applicants have found that RGS2 mRNA transcription is up regulated in activated macrophages compared to non activated macrophages and to activated monocytes. Therefore, the invention relates to the use of RGS2 as a biomarker for macrophage activation in any method, process, assays of any nature, whether experimental or not, using the fact that an increase in RGS2 expression and/or activity level is indicative of macrophage activation.

Such use encompasses the use of experimental tools derived from RGS2 expression products (RGS2 polynucleotides or polypeptides) or fragments thereof, such as primers and probes able to hybridize to RGS2 polynucleotides or fragments thereof, compounds able to bind to RGS2 polypeptides or fragments thereof, such as anti-RGS2 antibodies, as assayable biomarkers for macrophage activation. RGS2 may be used as a biomarker for macrophage activation alone or in combination with another marker.

Such a biomarker may be useful for a number of different purposes including experimental, diagnostic, prognostic, monitoring and screening purposes.

Such biomarker may indeed be used as a tool for experimental purposes in order to determine whether macrophages are activated in a given experimental context compared to a reference context. For example, such biomarker may be used to determine whether macrophages in a given sample are activated by a given stimulus compared to the same sample without any stimulus or activated with another stimulus.

Such biomarker may also be used for diagnostic, prognostic and monitoring purposes. Indeed, such biomarker may be used, alone or in combination with other markers, to determine whether a patient suffers from an activated-macrophage-related disorder such as an inflammatory disorder. It may also be used to determine the risk for a patient to develop complications associated with activated-macrophage-associated disorders such as chronic inflammation. It may be used to monitor the level of macrophage activation in an individual suffering from an activated-macrophage-related disorder. Such monitoring may be useful to monitor the level of inflammation in a patient. In addition, such biomarker may also be used to monitor the effect on macrophage activation of a treatment of such an activated-macrophage-related disorder. For example, such biomarker may be used to monitor the effect of an anti-inflammatory drug on macrophage activation in a patient suffering from an inflammatory disorder such as COPD or arthritis.

In addition, such a biomarker may be used in screening assays wherein agents are tested for their ability to modulate macrophage activation. For example, such biomarker may be very useful to determine whether an agent, for example an anti-inflammatory agent, is effective in interfering in *vitro* with the activation of macrophages.

## III. Methods of determination of macrophage activation

Another object of the invention is a method of determining whether macrophages are activated in a biological sample, wherein the RGS2 expression and/or activity level is measured in said biological sample in order to determine whether RGS2 expression level and/or activity is modulated, preferably elevated, in said sample compared to the RGS2 expression and/or activity level in a control sample.

Quantification of RGS2 expression level and/or activity may be achieved using methods for determining the amount of RGS2 polynucleotides in a sample, methods for determining the amount of RGS2 polypeptides in a sample and/or methods for determining the amount of RGS2 activity in a sample, as will be described below.

Determination of whether a RGS2 expression and/or activity level is modulated in a test sample may be achieved through relative comparison of levels of RGS2 expression and/or activity between said test sample and a control sample. Examples of test and control samples include: stimulated macrophages versus non stimulated macrophages, macrophages from an individual suffering from an inflammatory disorder versus an individual not suffering from said disorder, etc. Said comparison may be conducted through parallel quantification of both test sample and control sample submitted to the same experimental conditions in order to be able to directly compare the signal intensity given by both samples. In such case, relative and not necessarily absolute amounts of RGS2 expression and/or activity are determined.

Alternatively, determination of a modulated level of RGS2 expression and/or activity in a test sample may be achieved through absolute quantification of the amount of RGS2 expression and/or activity level. Such absolute quantification may be performed either through parallel quantification of a test sample and at least one control sample containing a known amount of RGS2 polynucleotides or polypeptides and submitted to the same experimental conditions. Such control samples allows to plot a signal vs. amount curve allowing absolute quantification to be performed. Alternatively, quantification of RGS2 expression and/or activity is performed and compared to existing calibration curves allowing absolute quantification to be determined. Then, the RGS2 expression and/or activity level in the test sample is compared to a reference level of RGS2 expression and/or activity. Such references levels may be those determined for a healthy individual for example.

For the purpose of the methods of the invention, a RGS2 expression and/or activity level in a sample will be considered modulated if a significant increase or decrease in RGS2 expression and/or activity exists for said sample compared to a control sample. Said significant increase or decrease depends on the reproducibility and sensitivity of the assay used. Therefore the threshold of said increase or decrease to consider that RGS2 expression and/or level are modulated depends on the quantification assay that is performed. Generally, a threshold of at least 2-fold, preferably at least 21-fold, preferably using quantitative PCR, preferably real-time PCR, will be considered significant to determine that the RGS2 expression and/or activity is modulated, preferably elevated.

## 1. Suitable biological samples

Biological samples for use in the methods of the present invention include, without any particular limitation, any sample that contains macrophages or macrophage-like cells.

Such suitable samples may be obtained from a cell, a tissue or an animal, for example, a mammal, particularly a human. Biological samples suitable for use in this method include but are not limited to i) *in vitro* cultured macrophagic cell lines such as differentiated U937, THP1, HL60, and Raw 264.7, ii) biological fluids such as blood, broncho alveolar lavage, sputum, synovial fluid, and iii) tissue samples (e.g. biopsies) especially from tissues rich in macrophages such as hematopoeitic tissues, lung, spleen, thymus, bone marrow, lamina propria, as well as liver, bone, central nervous system or skin. Cell cultures or cell extracts derived, for example, from tissue biopsies can also be used. Depending on the quantification assays to be performed, as can be readily envisioned by the man skilled in the art, suitable biological samples include but are not limited to cell lysates, intact cells, supernatants, or frozen tissue sections.

Biological samples may originate from individuals having different physiological conditions. For example, biological samples originate from individuals suffering from an activated-macrophage-related disorder. In such cases, control samples originate from individuals not suffering from this disorder, preferably healthy individuals. Biological samples may also originate from individuals suffering from an activated-macrophage-related disorder to which a treatment has been or is being given. Said individual are animals, preferably mammals, more preferably humans or rodents.

Alternatively, biological samples to compare may originate from samples submitted to different experimental conditions. For example, in the context of screening assays, a test sample may be contacted with a test agent which ability to interfere with macrophage activation needs to be assessed whereas the control sample is not contacted with such test agent. Another example is a test sample wherein macrophages have been stimulated by a stimulus which activation efficiency needs to be assessed whereas the control sample contains non-stimulated macrophages.

These biological samples are illustrating but not limiting examples and one skilled in the art could envision and prepare other types of biological samples in order to compare macrophage activation in different conditions.

<u>Preparation of biological samples</u>

Prior to quantification of RGS2 expression levels and/or activity, additional steps of preparation of suitable samples for conducting assays may be necessary, such as macrophage isolation and/or macrophage purification. The necessity of such additional steps could be easily envisioned by one skilled in the art depending on the type of biological sample used and of the type of assay to be performed to determine RGS2 expression levels and/or activity.

If the biological sample is a sample recovered from an animal, isolation of macrophages may be necessary when quantification techniques require that macrophages need to be removed from their natural environment in order for the assay to be performed. Alternatively, quantification of levels of RGS2 expression and/or activity may be performed using *in situ* techniques or histochemical techniques not requiring macrophage isolation. Techniques of isolation of macrophages from biological fluids, tissue and immune response sites are well known to those skilled in the art including isolation techniques described in "Handel-Fernandez and Lopez, "Macrophages", Practical Approach series, Chap 1, pp 1-30, Ed Paulnock, Oxford University Press (2000), and described in Example 1 of the PCT publication WO9747325, which disclosures are incorporated by reference in their entireties.

Optionally, macrophages may need to be purified from other sample materials, mostly from other cells, using any techniques known to those skilled in the art including adherence-based methods, velocity sedimentation-based methods, elutriation and isopycnic sedimentation methods such as those described in Gessani *et al.,* "Macrophages", Practical Approach series, Chap 2, pp 31-60, Ed Paulnock, Oxford University Press (2000), hereby incorporated by reference in its entirety. Further purification of macrophages may be obtained using magnetic activated cell sorting (MACS) techniques or fluorescence activated cell sorting (FACS) techniques such as those described in Ecsedy *et al.,*

J. Lpid Res. (1998) 39:2218-27; Baldus *et al.,* Histochem. J. (1998) 30:285-91; Adeleye *et al,* Hum. Reprod. (1996) 11:451-6; Davey *et al.,* Br J. Haernatol. (2000) 111:934-42 and Johnston *et al.,* Ann. Neurol. (2001) 49:650-8, which disclosures are incorporated by reference in their entireties. These techniques are based on the use of antibodies able to bind to macrophage cell surface antigens such as CD68, CD11b, CD18, CD14, CD32, F4/80, or SR-A. More macrophage cell surface antigens may be found in Weir's handbook of experimental immunology (5th edn), chapters 174 and 175, Ed Herzenberg *et al,* Blackwell Scientific publications (1997), hereby incorporated by reference in its entirety. Alternatively, the natural auto fluorescence of resident alveolar macrophages (Viksman *et al.,* (1994) J Immunol Methods;172(1):17-24) may be used, and eventually enhanced by a fluorescent dye such as PHK26-PCL, to isolate them from other cells as described in Maus *et al.,* (2001) Am. J. Physiol. Lung Cell. Mol. Physiol. 280:L58-L68, which disclosures are incorporated by reference in their entireties.

**2. Quantification of RGS2 expression levels**

Quantification of RGS2 expression levels may be achieved either by measuring the amount of RGS2 polynucleotides present in a sample or the amount of RGS2 polypeptides present in a sample.

2.1. Quantification of RGS2 polynucleotides present in a sample

Quantification RGS2 polynucleotides, either at the cellular or tissue level, may be achieved using any techniques known to those skilled in the art including hybridization-based techniques as well as amplification-based techniques.

Suitable samples for conducting these assays may be any type of samples containing any type of nucleic acid molecules. Such samples may contain either single stranded or double stranded molecules or a mixture thereof. They may either contain RNA, preferably mRNA, or contain DNA, preferably cDNA, or a mixture thereof.

Suitable biological samples to conduct such methods are samples in which nucleic acid molecules are accessible, including crude cell extracts or nucleic acid preparations (either RNA preparation or cDNA preparation). Preferably, nucleic acid molecules are extracted and eventually purified from biological samples using any techniques of preparation of total RNA or polyA RNA known to those skilled in the art such as those described in *Current Protocols in Molecular Biology,* John Wiley & Sons, Inc. (1997) and Sambrook and Russsell, (2001), Molecular Cloning: A Laboratory Manual 3rd ed. Cold Spring Harbor Laboratory, Cold Spring Harbor, New York, which disclosures are incorporated by reference in their entireties .

When cDNA preparation is necessary, reverse transcription and eventually synthesis of a second cDNA strand are performed preliminary to actual quantification of RGS2 polynucleotides. Techniques for generating appropriate oligonucleotide primers for reverse transcription and primer extension, for reverse transcribing a primer hybridized to mRNA to generate a first cDNA strand, and for extending a primer to make a second cDNA strand complementary to the first cDNA strand are well known to those skilled in the art including those described *in Current Protocols in Molecular Biology,* (1997) supra and Sambrook and Russell, (2001), supra.

2.1.1. Using hybridization-based techniques

In one embodiment of said method of determining whether macrophages are activated in a sample, said RGS2 expression level is determined using hybridization-based techniques. Techniques used to quantify the expression levels of nucleic acids capable of hybridizing to a detectable probe include techniques well known to those skilled in the art such as Northern blotting, Southern blotting, *in situ* hybridization, dot blotting, slot blotting or Rnase protection assays (Parker and Barnes, (1999) Meth. Mol. Biol. 106:247-283; Hod (1992) Biotechniques 13:852-854; Saccomano *et al.* (1992) Biotechniques 13:846-850; Sambrook and Russell (2001) supra; which disclosures are incorporated by reference in their entireties).

In such hybridization-based techniques, a nucleic acid sample is incubated in presence of a nucleic acid probe, generally an oligonucleotide probe, under conditions allowing hybridization to occur between the probe and RGS2 polynucleotides present in the sample to form a complex. Design of appropriate probe is well known to those skilled in the art and described elsewhere in the application. Preferably, stringent conditions of hybridization are used in order to ensure that the nucleic acid probe hybridizes only to nucleic acids molecules having a high degree of homology to the probe so that it binds specifically to its RGS2 polynucleotide target. Generally, stringent conditions are selected to be about 5°C to 20°C lower than the thermal melting point (Tm) for the probe sequence at a defined ionic strength and pH. The Tm is the temperature (under defined ionic strength and pH) at which 50% of the target sequence hybridizes to a perfectly matched probe.

For probes between 14 and 70 nucleotides in length the melting temperature (Tm) may be calculated using the formula: Tm=81.5+16.6(log (Na+))+0.41(fraction G+C)-(600/N) where N is the length of the probe.

If the hybridization is carried out in a solution containing formamide, the melting temperature may be calculated using the equation: Tm=81.5+16.6(log (Na+))+0.41(fraction G+C)-(0.63% formamide)-(600/N) where N is the length of the probe.

For example, the following stringent hybridization conditions are suitable for a probe of about 20 nucleotide in length. The hybridization step is realized at 65°C in the presence of 6 x SSC buffer, 5 x Denhardt's solution, 0,5% SDS and 100μg/ml of salmon sperm DNA and is followed by four washing steps:

- two washings during 5 min, preferably at 65°C in a 2 x SSC and 0.1%SDS buffer;
- one washing during 30 min, preferably at 65°C in a 2 x SSC and 0.1% SDS buffer,
- one washing during 10 min, preferably at 65°C in a 0.1 x SSC and 0.1%SDS buffer.

There is no need to say that the hybridization conditions described above have to be adapted according to the length of the desired nucleic acid, following techniques well known to the one skilled in the art. The suitable hybridization conditions may for example be adapted according to the teachings disclosed in the book of Hames and Higgins (1985) Nucleic Acid Hybridization: A Practical Approach. Hames and Higgins Ed., IRL Press, Oxford or using the disclosure of Sambrook *et al.,* (1989), supra.

Following hybridization and washing steps, detection is performed using standard direct or indirect detection techniques well known to those skilled in the art.

In such assays, either the probe (as described below) or the polynucleotides sample containing the RGS2 target sequence may be immobilized on any convenient support.

2.1.2. Using amplifcation-based techniques

In a preferred embodiment of said method of determining whether macrophages are activated in a biological sample, said RGS2 expression level is determined using amplification-based techniques.

Optionally, when the RGS2 polynucleotide to be amplified is a RNA molecule, preliminary reverse transcription and eventually synthesis of a second cDNA strand may be necessary to provide a DNA template to be amplified.

Amplification techniques that can be used in the context of the present invention include, but are not limited to semi-quantitative or quantitative PCR, including competitive PCR, non-competitive PCR and real-time PCR, branched DNA (bDNA)-based signal amplification assays, nucleic acid sequence based amplification assays (NASBA), and transcription mediated amplification (TMA).

*2.1.2.1. Quantitative PCR*

Quantitative PCR technology is the preferred amplification technique used in the present invention as it allows either relative or absolute quantification of specific nucleic acid molecules in a sample. A variety of quantitative PCR techniques are familiar to those skilled in the art including non-competitive PCR, competitive PCR and real-time PCR assays. For a review of experimental designs of quantitative PCR technology, see Freeman *et al.,* Biotechniques (1999) 26:112-22; Bustin, J. Mol. Endocrinol. (2000) 25:169-93; Watzinger *et al.,* Nuc. Acids Res. (2001) 29:E52-2, Walker (2001) J. Biochem. Molec. Toxicol. 15:121-127; US 5,824,516 and EP publications EP1138783 A2 and EP1138784 A2, which disclosures are incorporated by reference in their entireties. In each of these PCR procedures, PCR primers on either side of the nucleic acid sequences to be amplified are added to a suitably prepared nucleic acid sample along with dNTPs and a thermostable polymerase such as Taq polymerase, Pfu polymerase, Tth polymerase or Vent polymerise. The nucleic acid in the sample is denatured and the PCR primers are specifically hybridized to complementary nucleic acid sequences in the sample. The hybridized primers are extended. Thereafter, another cycle of denaturation, hybridization, and extension is initiated. The cycles are repeated multiple times to produce an amplified fragment containing the nucleic acid sequence between the primer sites. Amplicons are then analyzed using any conventional means known to those skilled in the art including but not limited to size-separation on a polyacrylamide or agarose gel, hybridization with a labeled probe, detection of incorporated labeled dNTPs, and detection of double stranded DNA using a double stranded DNA specific dye such as Sybr Green or ethidium bromide.

In competitive PCR, different amounts of the specific target sequence are co amplified together with a dilution series of an internal standard of a known copy number. The initial copy number of the target sequence is then extrapolated from the mixture containing an identical PCR product quantity of standard and target sequence at the so-called equivalence point (Zimmermann and Mannhalter, (1996) Bio-Techniques 21:280-279; Freeman *et al,* (1999) supra; Watzinger *et al.* (2001), supra, which disclosures are incorporated by reference in their entireties).

In non-competitive PCR, the same amount of the specific target sequence is co amplified together with a dilution series of an internal standard of a known copy number. The initial copy number of the target sequence at the equivalence point is then defined as the intersection between the plot of the log of standard signal vs. the log of input standard and the plot of the target signal vs. the log of input standard (Freeman *et al.* (1999), supra).

In real-time PCR, the formation of PCR products is monitored in each cycle of the PCR. The amplification is usually measured in thermocyclers that have additional devices for measuring fluorescence signals during or after the amplification reaction. Typical examples of these apparatus are the Roche Diagnostics LightCycler and the Perkin-Elmer ABI Prism 7700. In real-time PCR, the fluorescence signal is displayed immediately after each measurement, allowing amplification runs to be terminated or extended as appropriate. The amplification products may be detected using fluorescent dyes that bind to double-stranded DNA such as SYBG Green and ethidium bromide. Alternatively, amplicons may be detected by hybridization to a fluorescence-labeled probe that is specific to the target sequence, thus obviating the need for further confirmation of the analysis of the amplicon. Such fluorescence-labeled probes may be molecular beacons that only emit fluorescence signals when they are bound to the target nucleic acid, end-labeled fluorescent probes used alone or in combination such as allowing detection of a signal only when two independent probes hybridize to their correct target sequence, or hydrolysis probes that are hydrolyzed only when bound to their target sequence such as Taqman probes (PE applied Biosystems). For a review on the different types of fluorescence-labeled probes see Bustin (2000), *supra.* For each series of experiments, a signal threshold corresponding to the signal intensity for which all PCR reactions to be analyzed are in an exponential phase is determined. The number of cycles, so-called Ct, required to reach this threshold value is then determined for the target nucleic acid as well as for a reference nucleic acid such as a standard or housekeeping gene. The absolute or relative copy numbers of the target molecule can be determined on the basis of the Ct values obtained for the target nucleic acid and the reference nucleic acid (Gibson *et al.,* (1996) Genome Research 6:995-1001; Bieche *et al.,* (1999) Cancer Research 59:2759-2765; Bustin (2000), supra; Freman *et al.* (2000), supra; WO 97/46707; and WO 97/46714, which disclosures are incorporated by reference in their entireties). A detailed example of use of real time RT-PCR to quantify RGS2 expression level is given in Example 1.

*2.1.2.2. Other quantitative or semi-quantitative amplification techniques*

The NASBA and TMA technologies are isothermal nucleic acid amplification assays that are virtually identical in principle and practice. These isothermal nucleic acid amplification assays use oligonucleotide probes, a reverse transcriptase, Rnase H, a DNA polymerase and an RNA polymerase to amplify a target sequence. An isothermal nucleic acid amplification assay, unlike PCR, is an isothermal amplification method, thus it does not cycle between high and low temperatures to facilitate target amplification. NASBA was described in Guatelli *et al.*(1990) Proc. Natl. Acad. Sci. USA., 35:273-286 and in Compton (1991) *Nature* 350:91-92 and used to amplify human immunodeficiency virus type (HIV-1) (Kievits *et al.,* (1991) J. Virol. Meth., 35: 273-286), which disclosures are incorporated by reference in their entireties. TMA was described in PCT Publication WO 9322461, hereby incorporated by reference in its entirety.

In a NASBA assay, for example, the target RNA molecule is placed into solution with oligonucleotide primers and two polymerases. The temperature of the sample is raised to prepare the target molecules for amplification. The temperature is then lowered to permit the primer bind to the template. After binding the target, a DNA molecule complementary to the target sequence is synthesized using a reverse transcriptase, such as the AMV-reverse transcriptase. Subsequent to this synthesis, the RNA-DNA hybrid molecule is digested with RNase H, removing the RNA strand. A complementary strand to the single stranded DNA target sequence is synthesized through another round of DNA polymerization using a DNA polymerase enzyme Following the synthesis of this double stranded DNA molecule, single stranded RNA is synthesized from the double stranded DNA template with T7 RNA polymerase. This step amplifies the original target sequence 100 to 1000-fold. Simultaneously, new double stranded DNA templates are being synthesized from the replicated single stranded RNA templates produced in the first round of amplification. From this series of reactions, the target sequence is amplified.

In a branched DNA assay (bDNA), amplification involves previously synthesizing a branched polymer single-stranded DNA probe, and hybridizing the branched polymer single-stranded DNA probe to a target gene to detect the target gene as described in Urdea *et al.,* (1991) Nucleic Acids Symp. Ser. 24:197-200, hereby incorporated by reference in its entirety. Such amplification assays were used to quantify viral load as described in Wilber (1997) Immunol. Invest. 26:9-13, hereby incorporated by reference in its entirety.

2.1.3. Primers and probes

*2.1.3.1. Design of primers and probes*

Any polynucleotide able to hybridize to RGS2 polynucleotides may be used as a primer or probe according to the invention. Particularly preferred probes and primers include polynucleotides comprising a contiguous span of at least 12, 15, 18, 20, 25, 30, 35, 40, 50, 60, 70, 80, 90, 100, 150, 200, 500, 1000, 2000, 5000 or 10 000 nucleotides of a RGS2 polynucleotide or of a sequence fully complementary thereto.

A probe or a primer according to the invention has between 8 and 10 000 nucleotides in length. The length of primers can range from 8, 10, 15, 20,30, or 100 to 500 nucleotides, preferably from 10 to 50, more preferably from 15 to 30 nucleotides. The length of probes can range from 8,10,15, 20, 30, 50,100, 200, 500,1000, 2000, 5000 to 10000 nucleotides, preferably from 10 to 100, more preferably from 20 to 70 nucleotides. The appropriate length for primers and probes under a particular set of assay conditions may be empirically determined by one of skill in the art. The formation of stable hybrids depends on the melting temperature (Tm) of the DNA. The Tm depends on the length of the primer or probe, the ionic strength of the solution and the G+C content. The higher the G+C content of the primer or probe, the higher is the melting temperature because G:C pairs are held by three H bonds whereas A:T pairs have only two.

For amplification purposes, pairs of primers with approximately the same Tm, preferably identical Tm, are preferable. Primers may be designed using the OSP software (Hillier and Green, (1991) PCR Methods Appl., 1: 124-8, the disclosure of which is incorporated by reference in its entirety), based on GC content and melting temperatures of oligonucleotides, or using PC-Rare based on the octamer frequency disparity method (Griffais *et al.,* (1991) Nucleic Acids Res. 19: 3887-3891, the disclosure of which is incorporated by reference in its entirety), or any other methods or algorithm known to those skilled in the art.

As an illustration, primers and probes for carrying out a real-time PCR may be chosen using the Primer Express (Applied Biosystems) software according to the following criteria. Tm is chosen to range from 58°C to 60°C, preferably 59°C. The GC percentage is chosen to range between 40 and 60%. The primer length is chosen to range between 15 and 25 base pairs, preferably 20 base pairs. In addition, primers having more than 2 cytosine or guanine residues within the last 5 nucleotides of their 3' end are eliminated as well as couples of primers susceptible of forming intermolecular or secondary structure base-pairing as can be deduced from their primary structure. Preferably, couples of primers are chosen in order not to be able to amplify RGS2 genomic DNA. Primers of SEQ ID Nos 1 and 2 were chosen according to these criteria.

Determination of primer amplification efficiency for Real-time PCR

For competitive PCR and real-time PCR, couples of primers need to be validated experimentally according to standard methods known to those skilled in the art (see for example Bieche *et al.,* (1999) supra). For Real-time PCR, the efficiency of amplification of a couple of primers can be assessed as follows.

An optimal PCR reaction may be described as:

$$C = C0 * (1+E)^{Ct}$$

where C is the concentration of DNA targets when the signal threshold is reached, C0 is the concentration of DNA targets in the initial sample before amplification, E is the efficiency of amplification and Ct the number of cycles performed when the signal threshold is reached.

To measure the efficiency E for a given couple of primers, a standard curve plotting the number of cycles numbers necessary to reach a signal threshold versus the logarithm of the target concentration is drawn from experimental results, for example, using a range of dilutions for a sample containing a known amount of target DNA.

Such standard curve Ct=f(LogC) can be described as:

$$Ct = 1/(Log(1+E) * (LogC-LogC0)).$$

To have an optimized PCR, the efficiency of amplification should be as close to 1 as possible to allow for doubling of the number of DNA targets per cycle. Thus, the efficiency of couples of primers is assessed by their ability to give a standard curve with a slope of 1/Log(1+E)=3.32. Alternatively, E can be computed as $E=10^{1/m}-1$ where m is the slope of the standard curve.

*2.1.31. Preparation of primers and probes*

Primers and probes may be prepared by any suitable method, including, for example, cloning and restriction of appropriate sequences and direct chemical synthesis as described elsewhere in the present application.

Detection probes are generally nucleic acid sequences or uncharged nucleic acid analogs such as, for example peptide nucleic acids which are disclosed in International Patent Application WO 92/20702, morpholino analogs which are described in U.S. Patents Numbered 5,185,444; 5,034,506 and 5,142,047, which disclosures are hereby incorporated by reference in their entireties. The probe may have to be rendered "non-extendable" in that additional dNTPs cannot be added to the probe, generally by modifying the 3' end of the probe such that the hydroxyl group is no longer capable of participating in elongation. Techniques to render probes non extendable are well known to those skilled in the art including techniques to functionalize the 3' end of the probe with the capture or detection label to thereby consume or otherwise block the hydroxyl group or simply by cleaving, replacing or modifying the 3' hydroxyl group (see U.S. Patent Application Serial No. 07/049,061 filed April 19, 1993, for descriptions of modifications, which can be used to render a probe non-extendable).

*2.1.3.3. Labeling of probes*

Primers and probes according to the present invention can be labeled, if desired, by incorporating any label known in the art to be detectable by spectroscopic, photochemical, biochemical, immunochemical, or chemical means. For example, useful labels include radioactive substances (including, $^{32}$p, $^{35}$S, $^{3}$H, $^{125}$I), fluorescent dyes (including, 5-bromodesoxyuridin, fluorescein, acetylaminofluorene, digoxigenin) or biotin. Preferably, polynucleotides are labeled at their 3' and 5' ends. Examples of non-radioactive labeling of nucleic acid fragments are described in the French patent No. FR-7810975, Urdea *et al,* (1988) Nuc. Acids Res. 11:4937-4957 and Sanchez-Pescador *et al* (1988), J. Clin. Microbiol. 26:1934-1938, which disclosures are hereby incorporated by reference in their entireties. In addition, the probes according to the present invention may have structural characteristics such that they allow the signal amplification, such structural characteristics being, for example, branched DNA probes as those described in Urdea *et al.* (1991), supra, Horn *et al.,* (1991) Nucleic Acids Symp. Ser. 24:201-202 or in the European patent No. EP 0 225 807, which disclosures are hereby incorporated by reference in their entireties.

The detectable probe may be single stranded or double stranded and may be made using techniques known in the art, including *in vitro* transcription, nick translation, or kinase reactions. A nucleic acid sample containing a sequence capable of hybridizing to the labeled probe is incubated with the labeled probe. If the nucleic acid in the sample is double stranded, it may be denatured prior to incubation with the probe.

*2.1.3.4 Immobilization of primers or probes*

A label can also be used to capture the primer, so as to facilitate the immobilization of either the primer or a primer extension product, such as amplified DNA, on a solid support. A capture label is attached to the primers or probes and can be a specific binding member that forms a binding pair with the solid's phase reagent's specific binding member (e.g. biotin and streptavidin). Therefore depending upon the type of label carried by a polynucleotide or a probe, it may be employed to capture or to detect the target DNA. Further, it will be understood that the polynucleotides, primers or probes provided herein, may, themselves, serve as the capture label. For example, in the case where a solid phase reagent's binding member is a nucleic acid sequence, it may be selected such that it binds a complementary portion of a primer or probe to thereby immobilize the primer or probe to the solid phase. In cases where a polynucleotide probe itself serves as the binding member, those skilled in the art will recognize that the probe will contain a sequence or "tail" that may not be complementary to the target. In the case where a polynucleotide primer itself serves as the capture label, at least a portion of the primer will be free to hybridize with a nucleic acid on a solid phase. DNA Labeling techniques are well known to the skilled technician.

Any of the primers or probes of the present invention can be conveniently immobilized on a solid support. The solid support is not critical and can be selected by one skilled in the art. Thus, latex particles, microparticles, magnetic beads, non-magnetic beads (including polystyrene beads), membranes (including nitrocellulose or nylon strips), plastic tubes, walls of microtiter wells, glass or silicon chips, sheep (or other suitable animal's) red blood cells and duracytes are all suitable examples. Suitable methods for immobilizing nucleic acids on solid phases include ionic, hydrophobic, covalent interactions and the like. A solid support, as used herein, refers to any material that is insoluble, or can be made insoluble by a subsequent reaction. The solid support can be chosen for its intrinsic ability to attract and immobilize the capture reagent. Alternatively, the solid phase can retain an additional receptor that has the ability to attract and immobilize the capture reagent. The additional receptor can include a charged substance that is oppositely charged with respect to the capture reagent itself or to a charged substance conjugated to the capture reagent. As yet another alternative, the receptor molecule can be any specific binding member which is immobilized upon (attached to) the solid support and which has the ability to immobilize the capture reagent through a specific binding reaction. The receptor molecule enables the indirect binding of the capture reagent to a solid support material before the performance of the assay or during the performance of the assay. The solid phase thus can be a plastic, derivatized plastic, magnetic or non-magnetic metal, glass or silicon surface of a test tube, microtiter well, sheet, bead, microparticle, chip, sheep (or other suitable animal's) red blood cells, duracytes® and other configurations known to those of ordinary skill in the art. The primers or probes of the invention can be attached to or immobilized on a solid support individually or in groups of distinct polynucleotides to a single solid support.

## 2.2. Ouantification of RGS2 polypeptides present in a sample

Alternatively to methods quantifying RGS2 polynucleotides in a sample, methods determining the amount of RGS2 polypeptides present in a given context may be used to determine RGS2 expression level in a sample as described below.

Quantification of RGS2 polypeptides in a test sample can be carried out using any compound that binds to a RGS2 polypeptide or a fragment thereof. Such products may be antibodies, binding fragments of antibodies, compounds able to bind specifically to RGS2 polypeptides or fragments thereof, including RGS2 agonists and antagonists, substrates or ligands. Examples of binding compounds include $G_\alpha$ subunits, Gq subunits, or fragments thereof able to bind to RGS2.

The binding compounds may be labeled or unlabeled depending on the type of assay used. Labels which may be coupled to the antibodies include those known in the art and include, but are not limited to, enzymes (e.g., horseradish peroxidase or glucose oxidase), radioisotopes, fluorogenic (e. g., fluorescein isothiocyanate (FTTC), fluorescein isocyanate (FIC), 5dimethylamine-1-napthalenesulfonyl chloride (DANSC), tetramethyl- rhodamine isothiocyanate (TRITC), lissamine, and the like), chromogenic substrates, cofactors, biotin/avidin, chemiluminescent compounds, phosphorescent compounds, colloidal gold, colored particles and magnetic particles. As is appreciated by the skilled practitioner, in such cases in which the principal indicating group is an enzyme, additional reagents (e. g., substrates) are required for the production of a visible signal. Radioactive elements of various classes, such as such as $^{124}$I, $^{125}$I, $^{128}$I, $^{132}$I and $^{51}$Cr, (gamma ray emitters); $^{32}$p, $^{3}$H, $^{35}$S (beta emitters), and $^{11}$C, $^{14}$C, 150, or $^{13}$N (positron emitters), may also be used as detectable labels. The labeled complex may be detected visually, with a spectrophotometer, or by another detector, depending on the labeling or indicating group.

Modification of the binding compounds allows for coupling by any known means to carrier proteins or peptides or to known water-insoluble supports or matrices, for example, polystyrene or polyvinyl chloride microtiter plates, wells, or tubes; glass tubes or glass beads; and chromatographic supports, such as paper, cellulose and cellulose derivatives, and silica. Other suitable solid supports or matrices include the following as non limiting examples: crosslinked dextran (Pharmacia, Piscataway, NJ); agarose, polystyrene beads (about 1-5 microns in diameter; e.g., Abbott Laboratories, Illinois); and crosslinked polyacrylamide; nitrocellulose- or nylon-based webs, such as sheets, strips, paddles, or sticks.

Quantification of RGS2 polypeptide level in a sample may be achieved using any techniques known to those skilled in the art, including but not limiting to enzyme-linked immunosorbent assays (ELISA), radioimmunoassays (RIA), immunoblotting assays (e.g. Western blot), immunofluorescent assays, immunoprecipitation assays, chemiluminescent assays, and immunohistochemical assays. An example of quantification of RGS2 polypeptide levels by immunoblotting using an anti-RGS2 polyclonal antiserum is described in Sinnarajah *et al.* (2001), *supra* and in Sullivan *et al*. (2000) *supra*

A preferred protein quantification technique is the ELISA technique (Engvall *et al.,* (1971), Immunochemistry, 8:871-4; Stites *et al.,* (1982) in Basic and Clinical Immunochemistry, 4th Edition, Chap. 22, Lange Medical Publications, Los Altos, CA.; Reen, (1994), Methods Mol. Biol. 32:461-6; which disclosures are incorporated by reference in their entireties). The ELISA assays embraced by the present invention may be, for example, of direct format (where the labeled first antibody reacts with the antigen, e.g., RGS2 polypeptide), of indirect format (where a labeled second antibody reacts with the first antibody that binds to the antigen), of competitive format (such as the addition of a labeled antigen), or of sandwich format (where both labeled and unlabelled antibodies are utilized), as well as of any other format one skilled in the art could envision.

An ELISA assay initially requires preparing an antibody specific to the antigen, i.e. said RGS2 polypeptide or fragment thereof, which can be monoclonal or polyclonal. In addition, when the anti-RGS2 antibody is not labeled, another antibody, so-called reporter antibody, is prepared which binds to the specific antibody, or directly to the antigen itself. To the reporter antibody is attached a detectable entity such as a radioactive, fluorescent or enzymatic reagent.

In a typical ELISA, a specific anti-RGS2 antibody is first incubated on a solid support, e.g. a polystyrene well that binds the antibody permanently. Incubating with an unrelated protein such as bovine serum albumin then covers any free binding site on the wells. Next, the test sample is incubated on the antibody bound to the wells; standards with known amounts of antigen may also be included in the assay in parallel to provide a quantitative measure. After the wells are washed with buffer, a second specific anti-RGS2 antibody, which either is a monoclonal or polyclonal antibody different from the first antibody used to coat the well, or is a specific antibody already conjugated to a detection reagent, is incubated in the wells. During this time the second antibody attaches to any RGS2 polypeptides attached to the first antibody coated on the polystyrene well. Unbound antibody is washed out with buffer. If a reporter conjugated antibody has not already been used, a third antibody linked to a reporter molecule, e.g. horseradish peroxidase, is placed in the dish which can bind to the second antibody without binding to the first antibody used to coat the plate. Unattached reporter antibody is then washed out. The quantification of the signal provided by the reporter antibody then indicates the amount of RGS2 polypeptide present in the sample. Quantitative results typically are obtained by reference to a standard curve.

*How to make such RGS2-binding compounds*

Methods for producing an anti-RGS2 antibody

Anti-RGS2 antibodies may be prepared by any suitable method known in the art. Polyclonal anti-RGS2 antibodies may be prepared using an intact RGS2 polypeptide or fragments thereof as the immunogen. As will be appreciated by those having skill in the art, the immunogen can be conjugated to a carrier protein, if desired, to increase immunogenicity, particularly, if a small RGS2 peptide is used. Commonly used carriers that are routinely chemically coupled to peptides include but are not limited to bovine serum albumins, thyroglobulin, hemocyanin and tetanus toxoid. The coupled immunogen-carrier is then used to immunize a recipient animal (e.g., mouse, rat, sheep, goat, or rabbit) and anti-RGS2 antibodies isolated from said immunized animal, preferably from the serum of said animal.

Alternatively, anti-RGS2 antibodies may be monoclonal antibodies (mAb). As used herein, the term "monoclonal antibody" is not limited to antibodies produced through hybridoma technology but it rather refers to an antibody that is derived from a single clone, including eukaryotic, prokaryotic, or phage clone, and not the method by which it is produced. Monoclonal anti-RGS2 antibodies may be prepared by any conventional methods, including isolation of mAb from splenic cells of an immunized animal, hybridoma, genetically engineered monoclonal antibody or antibodies fragments or antibody produced by *in vitro* immunization by certain cells, and by phase display techniques. For example, see Kohler, *et al, Nature,* 256:495, 1975; *Current Protocols in Molecular Biology;* Harlow and Lane, *Antibodies: A Laboratory Manual,* Cold Spring Harbor Laboratory, New York (1988), Hammerling, *et al,* (1981) Monoclonal Antibodies and T Cell Hybridomas, Elsevier, N.Y. 563-681). (Said references incorporated by reference in their entireties).

Antigen binding fragments such as Fab, F(ab')2 and ssFv fragments may be produced, for example, from hybridoma-produced antibodies, by proteolytic cleavage, using enzymes such as papain (to produce Fab fragments) or pepsin (to produce F(ab')2 fragments) or other proteases.

Alternatively, antibodies of the present invention can be produced through the application of recombinant DNA technology or through synthetic chemistry using methods known in the art. For example, in phage display methods, functional antibody domains are displayed on the surface of a phage particle, which carries polynucleotide sequences encoding them. Phages with a desired binding property, e.g. a RGS2 polypeptide binding property, are selected from a repertoire or combinatorial antibody library (e.g. human or murine) by selecting directly with antigen, typically antigen bound or captured to a solid surface or bead. Phage used in these methods are typically filamentous phage including fd and M13 with Fab, Fv or disulfide stabilized Fv antibody domains recombinantly fused to either the phage gene III or gene VIII protein. Examples of phage display methods that can be used to make the antibodies of the present invention include those disclosed in Brinkman *et al.* (1995) *J. Immunol Methods.* 182:41-50; Ames, *et al.* (1995) *J. Immunol. Meth.* 184:177-186; Kettleborough, *et al.* (1994) Eur. L Immunol. 24:952-958; Persic, *et al.* (1997) Gene. 1879-81; Burton *et al.* (1994), *Adv. Immunol.* 57:191-280; PCT/GB91/01134; WO 90/02809; WO 91/10737; WO 92/01047; WO 92/18619; WO 93/11236; WO 95/15982; WO 95/20401; and US Patents 5,698,426, 5,223,409, 5,403,484, 5,580,717, 5,427,908, 5,750,753, 5,821,047, 5,571,698, 5,427,908,5,516,637,5,780,225, 5,658,727 and 5,733,743 (said references incorporated by reference in their entireties).

If necessary as assessed by one skilled in the art, polyclonal or monoclonal antibodies can be further purified, for example, by binding to and elution from a matrix to which the polypeptide or a peptide to which the antibodies were raised is bound. Those of skill in the art will know of various techniques common in the immunology arts for purification and/or concentration of polyclonal antibodies, as well as monoclonal antibodies (See for example, Coligan, *et al.,* Unit 9, *Current Protocols in immunology,* Wiley Interscience, 1994, incorporated herein by reference). For example, an anti-RGS2 antiserum was made in rabbits against the C-terminus peptide (PQITTEPHAT) of RGS2 coupled to KHL followed by affinity purification with the immunizing peptide (Sinnarajah *et al.* (2001) *supra)*

Screening for binding compounds

Alternatively, RGS2 binding compounds may be found using ligand screening methods. In such screening method, a biological sample or a defined compound to be tested as a putative ligand of a RGS2 protein is brought into contact with the corresponding purified RGS2 protein, for example the corresponding purified recombinant RGS2 protein produced by a recombinant cell host, or fragments thereof in order to form a complex between said RGS2 protein or fragments thereof and the putative ligand molecule to be tested. The compound to be tested for binding may be labeled with a detectable label, such as a fluorescent , radioactive, or enzymatic tag and placed in contact with immobilized RGS2 protein, or fragments thereof under conditions that permit specific binding to occur. After removal of non-specifically bound molecules, bound molecules are detected using appropriate means.

Various candidate substances or molecules can be assayed for interaction with a RGS2 polypeptide. These substances or molecules include, without being limited to, natural or synthetic organic compounds or molecules of biological origin such as polypeptides.

Interactions with drugs or small molecules, such as molecules generated through combinatorial chemistry approaches may, for example, be tested using the microdialysis coupled to HPLC method described by Wang *et al.* (1997) Chromatographia,44: 205-208 or the affinity capillary electrophoresis method described by Bush *et al.* (1997)), J. Chromatogr., 777: 311-328.

In further methods, peptides, drugs, fatty acids, lipoproteins, or small molecules able to interact with said RGS2 protein or fragments thereof may be identified using assays such as affinity chromatography, competition experiments or optical biosensor methods (Edwards and Leatherbarrow (1997) Analytical Biochemistry, 246, 1-6; Szabo *et al.* (1995) Curr Opin Struct Biol 5, 699-705.

When the candidate substance or molecule comprises a polypeptide, this polypeptide may be the resulting expression product of a phage clone belonging to a phage-based random peptide library (Parmley and Smith, (1988) Gene 73:305-318; Oldenburg *et al.,* (1992) Proc. Natl. Acad. Sci. USA 89:5393-5397; Valadon *et al.,* (1996), J. Mol. Biol., 261:11-22; Westerink, (1995) Proc. Natl. Acad. Sci USA., 92:4021-4025; Felici *et al.,* (1991) J. Mol. Biol., 222: 301-310, or alternatively the polypeptide may be the resulting expression product of a cDNA library cloned in a vector suitable for performing a two-hybrid screening assay (US Patents N° US 5,667,973 and 5,283,173 ; Harper *et al.* (1993) Cell, 75 : 805-816 ; Cho *et al.* (1998) *Proc. Natl. Acad. Sci. USA,* 95(7) : 3752-3757 ; Fromont-Racine *et al.* (1997) Nature Genetics, 16(3) : 277-282, which disclosures are hereby incorporated by reference in their entireties.

### 3. Quantification of RGS2 biological activity

Alternatively to methods aiming at determining RGS2 expression levels through determination of the amount of RGS2 polynucleotides or the amount of RGS2 polypeptides in a sample, assays for quantification of RGS2 activity may be used.

Assessing RGS2 activity may be performed using a variety of techniques. Assays to measure induction of GTPase activity involve assays measuring the rate of GTP hydrolysis including but not limited to *in vitro* single turnover assays described by Ingi *et al.* (1998) *supra* and *ex vivo* assays in lipid vesicles (Ingi *et al* (1998), *supra)* or in proteoliposomes (Cunningham *et al.* (2001) *supra).*

Assays to measure RGS2 biological activity via the modulation of $G_\alpha$ activity also involve assays to measure the modulation of $G_\alpha$ mediated intracellular signaling including but not limited to $G_\alpha$-mediated MAP kinase activation by IL-8 or M1 mAChR (Druey *et al.* (1996) Nature: 379:742-746 hereby incorporated by reference in its entirety; Ingi *et al.* (1998) *supra),* Gq-mediated phosphoinositide hydrolysis (Heximer *et al.* (1999), *supra;* Heximer *et al.* (2001), *supra),* Gq-mediated PLC activation in NG-108 cell membranes or in phospholipid vesicles (Heximer (1997a) *supra),* Gq-induced CREB reporter gene expression (Beadling *et al.* (1999) *supra),* Gi-mediated MAP kinase activation by IL-8 (Beadling *et al.* (1999) *supra),* Gi-mediated MAP kinase activation by M2 mAChR (Ingi *et al.* (1998) *supra,* halo assay (Heximer *et al.* (1999) *supra;* Druey *et al.* (1996) *supra),* and Gs-mediated cAMP production and insulin release by GIP (Tseng and Zhang (1998) *supra).*

Assays to measure RGS2 biological activity also include assays to measure the modulation of adenyl cyclase activity including but not limited to measurement of cAMP production by olfactory epithelium membranes and whole-cell voltage clamp recordings by odorant-stimulated olfactory neurons (Sinnarajah *et al.* (2001) *supra).*

### IV. Methods of diagnostic, prognostic and monitoring of activated-macrophage-related disorders

Methods to determine whether macrophages are activated in a biological sample based on the quantification of RGS2 expression and/or activity may also be very useful for diagnostic, prognostic, and monitoring purposes. Indeed, an elevated RGS2 expression and/or activity level may be indicative of an aberrant level of macrophage activation in an individual and may therefore have a predictive value for activated-macrophage-related disorders.

Thus, other objects of the invention concern methods of diagnosis, prognosis and monitoring of activated-macrophage-related disorder for an individual. In such methods, the macrophagic RGS2 expression levels and/or activity are quantified in at least one biological sample recovered from said individual using any one of the methods described herein.

In diagnosis and prognosis methods, said macrophagic RGS2 expression levels and/or activity in said individual is compared to the macrophagic RGS2 expression levels and/or activity of a control sample. For diagnosis purposes, control samples may be samples derived from individuals not suffering from an activated-macrophage-related disorder, preferably healthy individuals. Alternatively, said level of macrophagic RGS2 expression and/or activity in said individual is compared to standard values for individuals not suffering from an activated-macrophage-related disorder, preferably healthy individuals. An elevated level of RGS2 expression and/or activity is then taken as a sign of the presence of an activated-macrophage-related disorder.

For prognosis purposes, said individual already suffers from a disorder, preferably an activated-macrophage-related disorder and even more preferably at an early stage. Control samples may then be samples derived from individuals suffering from the activated-macrophage-related disorder, preferably the same activated-macrophage-related-disorder, even more preferably at a later stage. A level of RGS2 expression and/or activity similar in said individual to be tested and said control samples is then taken as a sign of the possibility for said individual to move towards a more advanced stage of the disorder. As an illustrating example, the individual may be classified at stage 0 of COPD and his macrophagic RGS2 expression and/or activity level compared to the macrophagic RGS2 expression and/or activity level of a patient suffering from a stage I, II, or III of COPD. Alternatively, such methods and assays may be very useful to predict chronic inflammation in a patient suffering from a disorder that is not an activated-macrophage-related disorder.

In monitoring methods, the macrophagic level of RGS2 expression and/or activity is measured in different samples recovered from the same individual suffering from an activated-macrophage-related-disorder at different time points. Levels of RGS2 expression and/or activity are then compared in order for example to follow the level of macrophage activation over time and eventually to evaluate the degree of severity or the evolution of said activated-macrophage-related-disorder.

Methods for determining whether macrophages are activated in a biological sample are also useful to assess the efficacy of a treatment against activated-macrophage-related-disorders. Indeed, quantifying the RGS2 expression and/or activity level in macrophages in a patient suffering from an activated-macrophage-related disorder and submitted to a treatment may be an indication of the ability of said treatment to interfere with macrophage activation, and therefore with the disorder.

Therefore, a further object of the invention is a method of assessing the efficacy of a treatment for an individual suffering from an activated-macrophage-related disorder, wherein said method comprises the step of determining whether macrophagic RGS2 expression levels and/or activity in a biological sample recovered from said individual submitted to said treatment are modulated using any one of the methods described herein. In such methods, the efficacy of the treatment is assessed by comparing the macrophagic RGS2 expression and/or activity levels in said individual submitted to said treatment to the macrophagic RGS2 expression and/or activity levels to a control sample. Said control sample may be of two types: either a ($1^{st}$ type )sample derived from an individual suffering from an activated-macrophage-related disorder and not submitted to said treatment, preferably the same individual before he was submitted to said treatment, or a $2^{nd}$ type sample derived from an individual not suffering from an activated-macrophage-related disorder, preferably a healthy individual. The efficacy of said treatment may be then assessed by either or both of the following criteria: i) a decreased level of the macrophagic RGS2 expression and/or activity in said individual submitted to said treatment when compared to a control sample of the $1^{st}$ type; ii) a difference as small as possible between the macrophagic RGS2 expression and/or activity in said individual submitted to said treatment and a control of the $2^{nd}$ type.

### V. Screening assays for agents modulating macrophage activation

RGS2 may also be useful as a biomarker in screening assays, so called "drug screening assays" or "bioassays", in order to test the ability of an agent to interfere with macrophage activation. Such agents may then be used in the prevention or treatment of activated-macrophage-related disorders. Therefore, the invention also relates to methods of determining whether an agent interfere, i.e. either reduce/inhibit or trigger/enhance macrophage activation, wherein said interference is measured via the modulation of the level of RGS2 expression and/or activity.

In such drug screening assays, the agent to be tested is contacted with macrophages either before activation or after activation under conditions permissive for the quantification of the macrophagic RGS2 expression and/or activity level. The RGS2 expression and/or activity level is then measured and compared to the level of RGS2 expression and/or activity of control samples. Such controls typically include activated macrophages not contacted with the agent and non activated macrophages. A significant change in the RGS2 expression and/or activity levels in the presence of the agent relative to what is detected in the absence of the agent is indicative of a modulation of macrophage activation by the agent. Indeed, a decrease in RGS2 expression and/activity in samples containing the agent compared to samples not containing said agent is indicative of an agent able to reduce macrophage activation. An increase in RGS2 expression and/ activity in samples containing the agent compared to sample not containing said agent is indicative of an agent able to enhance macrophage activation.

Any assay well known to those skilled in the art may be used in the context of the invention. Generally, a plurality of assay mixtures is run in parallel with different agent concentrations to obtain a differential response to the various concentrations. Typically, one of these concentrations serves as a negative control, i.e at zero concentration or below the level of detection.

Also included in the screening method of the invention are combinatorial chemistry methods for identifying chemical compounds. See, for example, Plunkett & Ellman, ("Combinatorial Chemistry and New Drugs." Scientific *American,* April, 69, 1997). Areas of investigation for combinatorial chemistry are the development of therapeutic treatments. Of particular interest are screening assays for agents that have a low toxicity for human cells.

A variety of reagents may be included in the screening assay. These include reagents like salts, neutral proteins, e.g. albumin, detergents, etc that are used to facilitate optimal protein-protein binding and/or reduce non-specific or background interactions. Reagents that improve the efficiency of the assay, such as protease inhibitors, nuclease inhibitors or anti-microbial agents may be used. The mixture of components is added in any order that provides for the requisite binding. Incubations are performed at any suitable temperature, typically between 4 and 40° C. Incubation periods are selected for optimum activity, but may also be optimized to facilitate rapid high-throughput screening. Typically between 0.1 and 1 hour will be sufficient.

**1. Description of agents:**

Definition

The term "agent" describes any molecule, particularly proteins, peptides, polypeptides, aptamers, carbohydrates, and small organic molecules. Candidate agents encompass numerous chemical classes, though typically they are organic molecules, preferably small organic compounds having a molecular weight of more than 50 and less than about 2,500 daltons (Da). Candidate agents often comprise functional groups necessary for structural interaction with proteins, particularly hydrogen bonding, and typically include at least an amine, carbonyl, hydroxyl or carboxyl group, preferably at least two of the functional chemical groups. The candidate agents often comprise cyclical carbon or heterocyclic structures and/or aromatic or polyaromatic structures substituted with one or more of the above functional groups. Candidate agents are also found among biomolecules including, but not limited to: peptides, polypeptides (such as antisense polypeptides), saccharides, fatty acids, steroids, purines, pyrimidines, derivatives, structural analogs or combinations thereof.

Preparation

Candidate agents are obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification or amidification to produce structural analogs.

**2. Screening for RGS2 modulators**

Of particular interest for the present invention are agents that are able to reduce or inhibit macrophage activation via the modulation of RGS2 expression and/or level, namely RGS2 modulators. As used herein, the term "RGS2 modulator" refers to both RGS2 inhibitors (i.e. compounds able to reduce or inhibit RGS2 expression and/or activity) and RGS2 activators (i.e. compounds able to trigger or enhance RGS2 expression and/or activity). Such RGS2 modulators may be particularly useful in the prevention and/or treatment of activated-macrophage-related disorders. Therefore other objects of the invention relate to compounds acting as RGS2 modulators as well as to methods of determining whether a compound acts as a RGS2 modulator in macrophages upon activation or in already activated macrophages.
Candidate compounds may be first isolated in standard screening assays for compounds modulating RGS2 expression and/or activity as described below and then further tested for their ability to operate as RGS2 modulators in the context of macrophage activation.

2.1. Compounds acting as RGS2 modulators

RGS2 modulators may interfere with RGS2 expression and/or activity at one or several of the different steps leading from transcription of RGS2 genomic DNA to activity of the RGS2 polypeptide. In particular, RGS2 modulators may interact with RGS2 polynucleotides and/or they may interact with RGS2 polypeptides.

2.1.1. Modulators of RGS2 expression

Gene expression is a complex process by which a gene's coded information (genomic DNA) is converted into structures (mostly proteins) present and operating into a cell. It involves several steps including transcription, stability, splicing, export, translation, and post-translational modifications. Each of this step is susceptible to be modulated by a compound directly through binding or indirectly.

Of particular interest as RGS2 modulators are compounds able to interact with the regulatory sequences of a RGS2 polynucleotide or a fragment thereof. Screening assays for such compounds are detailed below.

Other interesting compounds able to act as RGS2 inhibitors are RGS2 oligonucleotides used in antisense or triple helix approaches as described below.

*a) Compounds interacting with regulatory sequences of a RGS2 polynucleotide.*

Of particular interest are compounds able to interact with the regulatory sequences of RGS2 polynucleotides, including genomic DNA, pre-mRNA or mRNA, such as for example promoter or enhancer sequences in untranscribed regions of the genomic DNA, or regulatory sequences located in untranslated regions of RGS2 mRNA. Such compounds may be isolated and eventually subsequently identified as follows.

Sequences within untranscribed or untranslated regions of a RGS2 polynucleotide may be identified by comparison to databases containing known regulatory sequence such as transcription start sites, transcription factor binding sites, promoter sequences, enhancer sequences, 5'UTR and 3'UTR elements (Ghosh (2000) Nucleic Acids Res;28 (1):308-10; Perier *et al.,* (2000) Nucleic Acids Res 2000 Jan 1;28(1):302-3; Kolchanov *et al.,* (2000) Nucleic Acids Res 2000 Jan 1;28(1):298-301; Wingender *et al.,* (2001) Nucleic Acids Res,29(1):281-3; Pesole *et al.,* (2000) Nucleic Acids Res, 28(1):193-196 which disclosures are incorporated by reference in their entireties. See Baxevanis (2001) Nucleic Acids Res 29(1):1-10 and http://www. nar.oupjournals.org for up-to-dated links to databases). Alternatively, the regulatory sequences of interest may be identified through conventional reporter vectors in which a reporter gene such as beta galactosidase, chloramphenicol acetyl transferase, green fluorescent protein, or luciferase, is under the control of RGS2 sequences which regulatory function on gene expression needs to be assessed. Furthermore, such reporter vector systems allows to define whether the regulatory sequences of interest increase (RGS2 activator) or decrease (RGS2 inhibitor) RGS2 expression. Further refinement of identified RGS2 regulatory sequences may be achieved using conventional mutagenesis or deletion analyses including but not limited to site-directed mutagenesis, nested 5' and/or 3' deletions, linker scanning analysis or other techniques familiar to those skilled in the art. See for example assays described in Coles *et al.,* (1998) *Hum Mol Genet* 7:791-800, WO 97/17359, US Patent No. 5,374,544; EP 582 796; US Patent No. 5,698,389; US Patent No. 5,643,746; US Patent No. 5,502,176; and US Patent 5,266,488, which disclosures are incorporated by reference in their entireties.

Following the identification of potential RGS2 regulatory sequences, proteins which interact with these regulatory sequences may be identified as described below.

Gel retardation assays may be performed independently in order to screen candidate molecules that are able to interact with the regulatory sequences of a RGS2 polynucleotides or a fragment thereof, such as those described in Sambrook *et al.* (2001), supra or by Dent and Latchman (1993) The DNA mobility shift assay. In: Transcription Factors: A Practical Approach (Latchman DS, ed.) pp1-26. Oxford: IRL Press, which disclosure is hereby incorporated by reference in its entirety. These techniques are based on the principle according to which a DNA or RNA fragment that is bound to a protein migrates slower than the same unbound DNA or RNA fragment. Briefly, the target nucleotide sequence is labeled. Then the labeled target nucleotide sequence is brought into contact with either a total nuclear extract from cells containing regulation factors, or with different candidate molecules to be tested. The interaction between the RGS2 target regulatory sequence and the candidate molecule or the regulation factor is detected after gel or capillary electrophoresis through retardation in migration.

Nucleic acids encoding proteins which are able to interact with the regulatory sequences sequence of a RGS2 polynucleotide may be identified using a one-hybrid system, such as that described in the booklet enclosed in the Matchmaker One-Hybrid System kit from Clontech (Catalog Ref. n° K1603-1), the technical teachings of which are herein incorporated by reference. Briefly, the target RGS2 nucleotide sequence is cloned upstream of a selectable reporter sequence and the resulting polynucleotide construct is integrated in the yeast genome. Preferably, multiple copies of the target sequences are inserted into the reporter plasmid in tandem. The yeast cells containing the reporter sequence in their genome are then transformed with a library comprising fusion molecules between cDNAs encoding candidate proteins for binding onto the RGS2 regulatory sequences and sequences encoding the activator domain of a yeast transcription factor such as GAL4. The recombinant yeast cells are plated in a culture broth for selecting cells expressing the reporter sequence. The recombinant yeast cells thus selected contain a fusion protein that is able to bind onto the RGS2 target regulatory sequence. Then, the cDNAs encoding the fusion proteins are sequenced and may be cloned into expression or transcription vectors *in vitro.* The binding of the encoded polypeptides to the target RGS2 regulatory sequences may be confirmed by techniques familiar to the one skilled in the art, such as gel retardation assays or DNAse protection assays well known to those skilled in the art. The ability of such polypeptides to effectively regulate RGS2 expression may also be confirmed using techniques known to those skilled in the art including co-transfection experiments with a reporter vector system bearing the RGS2 regulatory sequences of interest and an expression vector for the polypeptide to be tested.

*b) Antisense and triple helix RGS2 oligonucleotides*

RGS2 expression may also be inhibited or reduced either *in vitro* or *in vivo* using one or several RGS2 oligonucleotide fragments as an antisense tool or a triple helix tool that binds to a RGS2 polynucleotides, thereby preventing further RGS2 expression.

Antisense approach

In antisense approaches, nucleic acid sequences complementary to a RGS2 polynucleotide hybridize to the RGS2 polynucleotide intracellularly, thereby blocking the expression of the RGS2 polypeptide encoded by the RGS2 polynucleotide. The antisense nucleic acid molecules to be used in such approaches may be either DNA molecules, RNA molecules or hybrid DNA/RNA molecules.

Generally, antisense nucleic acid molecules are 7-25 long oligonucleotides that are complementary to a RGS2 polynucleotide, preferably a RGS2 mRNA, or a fragment thereof. A combination of different antisense polynucleotides complementary to different parts of the desired targeted gene can also be used. Antisense oligonucleotides may be designed to be complementary to the 5'end of RGS2 mRNA, preferably to the translation initiation codon. Alternatively, they may be complementary to a sequence of RGS2 genomic DNA containing a splicing donor or acceptor site. Optimal selection of antisense oligonucleotides may also be performed using the teaching of Green *et al.,* (1986) Ann. Rev. Biochem. 55:569-597; Izant and Weintraub, (1984) Cell 36(4):1007-15; Wagner *et al.,* (1996) Nat Biotechnol. 14(7): 840-4), Mir and Southern (1999) Nat. Biotechnol. 17:788-792; Patzel *et al.* (1999) Nuc. Acids Res. 27:4328-4334; Lebedeva and Stein (2001) Anu; Rev. Pharmacol. Toxicol. 41:403-419; Smith *et al.,* (2000) Eur. J. Pharm. Sci. 11(3): 191-198; Sohail and Southern (2000) Adv. Drug. Deliv. Rev. 44(1):23-34, which disclosures are incorporated by reference in their entireties. The antisense nucleic acids should have a length and melting temperature sufficient to permit formation of an intracellular duplex having sufficient stability to inhibit the expression of a RGS2 polynucleotides in the duplex.

The antisense polynucleotides of the invention may have a 3' polyadenylation signal that has been replaced with a self-cleaving ribozyme sequence, such that RNA polymerase II transcripts are produced without poly(A) at their 3' ends, these antisense polynucleotides being incapable of export from the nucleus, such as described by Liu *et al.,* (1994) Proc. Natl Acad. Sci. USA. 91: 4528-4262, which disclosure is hereby incorporated by reference in its entirety. In a preferred embodiment, these RGS2 antisense polynucleotides also comprise, within the ribozyme cassette, a histone stem-loop structure to stabilize cleaved transcripts against 3'-5' exonucleolytic degradation, such as the structure described by Eckner *et al.,* (1991) EMBO J. 10:3513-3522, which disclosure is hereby incorporated by reference in its entirety.

Antisense polynucleotides may be obtained using different techniques well known to those skilled in the art . For example, antisense polynucleotides may be obtained by reversing the orientation of the targeted RGS2 region, for example the RGS2 coding region in the case a mRNA is targeted, with respect to a promoter so as to transcribe the opposite strand from that which is normally transcribed in the cell. The antisense nucleic acid molecules may then be transcribed using in *vitro* transcription systems such as those which employ T7 or SP6 polymerase to generate the transcript. Alternatively, the antisense nucleic acid molecules may then be transcribed *in vivo* by operably linking DNA containing the antisense sequence to a promoter in a suitable expression vector. Alternatively, antisense oligonucleotides of a desired sequence may be chemically synthesized using any techniques known to those skilled in the art including those described herein.

In some embodiments, antisense polynucleotides are modified to increase stability, make them less sensitive to RNase activity and/or decrease their potential toxicity. Examples of modifications suitable for use in antisense strategies include modification in the phosphate backbone, the sugar moiety, and/or the nucleobase (McKay *et al.* (1996) *Nucleic Acids Res* 24: 411-7; Monia *et al.,.* (1993) *J Biol Chem* 268: 14514-22; Crooke (1997) *Ciba Found Symp* 209: 158-164.; Monia (1997) *Ciba Found Symp* 209: 107-119.; Flanagan *et al.,* (1999) *Proc Natl Acad Sci USA* 96:3513-8; Flanagan *et al.,* (1999) *Nat Biotechnol* 17: 48-52), morpholino oligonucleotides (Summerton and Weller (1997) *Antisense Nucleic Acid Drug Dev 7: 187-95),* chemically conjugated oligonucleotides (Spiller *et al.,* (1998) *Blood* 91: 4738-46), peptide nucleic acids (Good and Nielsen (1997) *Antisense Nucleic Acid Drug Dev 7:* 431-7; Nielsen (1999) *Curr Opin Struct Biol 9:* 353-7), and plasmid-derived RNA (Weiss*et al.,* (1999) *Cell Mol Life Sci* 55: 334-58; Inouye *et al.,* (1997) *Ciba Found Symp* 209: 224-233), which disclosures are incorporated by reference in their entireties. Further examples are described in Rossi *et al.,* (1991) Pharmacol. Ther. 50:245-254 ; in Herdewijn (2000) antisense Nucleic Acid Drug Dev. 10(4) :297-310 ; and in Lebedeva and Stein (2001), supra, which disclosures are incorporated by reference in their entireties.

The appropriate level of antisense nucleic acids required to inhibit gene expression may be determined using *in vitro* expression analysis. The antisense polynucleotides may be introduced into test cells or organisms by diffusion, injection, infection or transfection using procedures known in the art. For example, the antisense nucleic acids can be introduced into the body as a bare or naked polynucleotide, polynucleotide encapsulated in lipid, polynucleotide sequence encapsidated by viral protein, or as an polynucleotide operably linked to a promoter contained in an expression vector. The expression vector may be any of a variety of expression vectors known in the art, including retroviral or viral vectors, vectors capable of extrachromosomal replication, or integrating vectors. The vectors may be DNA or RNA. Such delivery systems include cationic lipids (Hope *et al.,* (1998) *Mol Membr Biol* 15:1-14; Gokhale *et al.,* (1997) *Gene Ther 4:* 1289-99) molecular umbrellas (Janout *et al.,* (1997) *Bioconjug Chem 8:* 891-5), water-soluble cationic porphyrins (Benimetskaya *et al..,* (1998) *Nucleic Acids Res* 26: 5310-7; Talkie *et al., (1997) Antisense Nucleic Acid Drug Dev* 7:177-85), Starburst polyamidoamine dendrimers (Helin *et al.,* (1999) *Biochem Pharmacol* 58: 95-107; Bielinska *et al.,* (1996) *Nucleic Acids Res* 24: 2176-82); lipophilic conjugates (Rump *et al.,* (1998) *Bioconjug Chem 9:* 341-9; Spiller *et al.,* (1998) *Blood 91:* 4738-468), fusion peptides containing both hydrophobic and hydrophilic sequences (Chaloin *et al.,* (1998) *Biochem Biophys Res Commun 243:* 601-8). For reviews of such delivery systems see Juliano and Yoo (2000) Curr. Opin. Mol. Ther. 2(3):297-303; Garcia-Chaumont *et al.,* (2000) Pharmacol. Ther. 87(2):255-277; Lebedeva *et al.,* (2000) Eur. J. Pharm. Biopharm. 50(1):101-119, which disclosures are incorporated by reference in their entireties).

The antisense molecules are introduced onto cell samples at a number of different concentrations preferably between $1x10^{-10}$M to $1x10^{-4}$M. Once the minimum concentration that can adequately control gene expression is identified, the optimized dose is translated into a dosage suitable for use in *vivo.* For example, an inhibiting concentration in culture of $1x10^{-7}$ translates into a dose of approximately 0.6 mg/kg bodyweight. Levels of oligonucleotide approaching 100 mg/kg bodyweight or higher may be possible after testing the toxicity of the oligonucleotide in laboratory animals. It is additionally contemplated that cells from the vertebrate are removed, treated with the antisense oligonucleotide, and reintroduced into the vertebrate. In a preferred application of this invention, the effectivenes of antisense inhibition on translation is monitored using techniques to quantify not only the amount of RGS2 polynucleotides but also the amount of RGS2 polypeptides and/or the amount of RGS2 activity.

An alternative to the antisense technology that is used according to the present invention comprises using ribozymes that will bind to a target sequence via their complementary polynucleotide tail and that will cleave the corresponding RGS2 target RNA (pre-mRNA or preferably mRNA) by hydrolyzing its target site (namely "hammerhead ribozymes"). Briefly, the simplified cycle of a hammerhead ribozyme comprises (1) sequence specific binding to the RGS2 target RNA via complementary antisense sequences; (2) site-specific hydrolysis of the cleavable motif of the target strand; and (3) release of cleavage products, which gives rise to another catalytic cycle. Indeed, the use of long-chain antisense polynucleotide (at least 30 bases long) or ribozymes with long antisense arms are advantageous. A preferred delivery system for antisense ribozyme is achieved by covalently linking these antisense ribozymes to lipophilic groups or to use liposomes as a convenient vector.

Ribozyme activity can be optimized as described by Stinchcomb *et al.,* "Method and Composition for Treatment of Restenosis and Cancer Using Ribozymes," filed May 18, 1994, U.S. Ser. No. 08/245,466. The details will not be repeated here, but include altering the length of the ribozyme binding arms (stems I and III, see FIG. *2c),* or chemically synthesizing ribozymes with modifications that prevent their degradation by serum ribonucleases (see e.g., WO 92/07065; Perrault *et al.,* (1990) *Nature* 344, 565; Pieken *et al.,* (1991) *Science* 253, 314; Usman and Cedergren, (1992) *Trends in Biochem. Sci.* 17, 334; WO 93/15187; and WO 91/03162, as well as U. S. Patent Application 07/829,729, and EP 92110298.4 which describe various chemical modifications that can be made to the sugar moieties of enzymatic RNA molecules. Modifications which enhance their efficacy in cells, and removal of stem II bases to shorten RNA synthesis times and reduce chemical requirements. (All these publications are hereby incorporated by reference herein.).

Antisense ribozymes according to the present invention may be prepared and used as described by Rossi *et al.,* (1991) Pharmacol. Ther. 50:245-254 ; Sczakiel *et al.,* (1995) Trends Microbiol. 3(6):213-217, Arndt and Rank (1997) *Genome* 40: 785-97; Rossi (1997) *Ciba Found Symp 209:* 195-204; Kohler *et al.,* (1999) *J Mol Biol* 285: 1935-50; Lewin and Hauswirth (2001) Trends Mol Med 7(5):221-8; Phylactou (2000) Adv Drug Deliv Rev;44(2-3):97-108; Amarzguioui and Prydz (1998) Cell Mol Life Sci; 54(11):1175-202; U.S. Pat. No. 5,254,678; US Pat. No. 6,307,041, Bertrand. *et al.,* (1994) Nucleic Acids Res. 22:293-300, US RE37411, US Patent 6,280,936, US Patent No. 6,271,359, US Patent No. 6,265,167, US Publication No. 20010006801, US Patent No. 6,204,027, US Patent No. 6,183,959, EP 1144599 and EP 1088086 which disclosures are incorporated by reference in their entireties.

Triple Helix Approach

RGS2 expression may also be inhibited using intracellular triple helix approaches. Such triple helix polynucleotides are able to inhibit transcription and are particularly useful for studying alterations in cell activity when it is associated with a particular gene. Traditionally, homopurine sequences were considered the most useful for triple helix strategies. However, homopyrimidine sequences can also inhibit gene expression. Such homopyrimidine oligonucleotides bind to the major groove at homopurine:homopyrimidine sequences. Thus, both types of sequences from the RGS2 genomic DNA are contemplated within the scope of this invention.

To carry out gene therapy strategies using the triple helix approach, the RGS2 genomic DNA sequence is first scanned to identify 10-mer to 20-mer homopyrimidine or homopurine stretches which could be used in triple-helix based strategies for inhibiting RGS2 expression. Following identification of candidate homopyrimidine or homopurine stretches, their efficiency in inhibiting RGS2 expression is assessed by introducing varying amounts of oligonucleotides containing the candidate sequences into tissue culture cells which express RGS2. For information on the generation of oligonucleotides suitable for triple helix formation see Griffin *et al.,* (1989) Science 245:967-971, Casey and Glazer (2001) Prog Nucleic Acid Res Mol Biol;67:163-92 and US Patent No US6303376; which disclosures are incorporated by reference in their entireties.

The oligonucleotides can be introduced into the cells using a variety of methods known to those skilled in the art, including but not limited to calcium phosphate precipitation, DEAE-Dextran, electroporation, liposome-mediated transfection or native uptake.

Treated cells are then monitored for altered cell function or reduced RGS2 expression and/or activity using techniques such as Northern blotting, RNase protection assays, or PCR based strategies to monitor the transcription levels of the RGS2 gene in cells which have been treated with the oligonucleotide.

The oligonucleotides which are effective in inhibiting gene expression in tissue culture cells may then be introduced *in vivo* using the techniques and at a dosage calculated based on the *in vitro* results, as described in the section entitled "Antisense approach".

In some embodiments, the natural (beta) anomers of the oligonucleotide units can be replaced with alpha anomers to render the oligonucleotide more resistant to nucleases. Further, an intercalating agent such as ethidium bromide, or the like, can be attached to the 3' end of the alpha oligonucleotide to stabilize the triple helix.

### 2.1.2. Modulators of RGS2 activity

Compounds able to modulate RGS2 activity may also be compounds able to interact with a RGS2 polypeptide or a fragment thereof either directly (e.g. through binding) or indirectly (e.g., by modulating the ability of a RGS2 polypeptide to perform its activity). Such compounds may either be RGS2 inhibitors or RGS2 activators.

Such a RGS2 inhibitor may be an antibody, or a fragment thereof, specific for a RGS2 protein or a functional portion of RGS2. Anti-RGS2 antibodies and fragments thereof may be produced using technologies described elsewhere in the application and then tested for their ability to inhibit and/or reduce RGS2 activity as described below.

Alternatively, a RGS2 inhibitor may be a compound other than an antibody (e.g., small organic molecule, protein or peptide) that binds to a RGS2 protein or fragment thereof and blocks its activity, i.e. an antagonist or a fragment thereof. Inactive $G_\alpha$ subunits, such as Gq subunits, or fragments thereof that retain their ability to bind to RGS2 but are unable to mediate intracellular events may be such RGS2 antagonists. Such inactive $G_\alpha$ proteins may be produced using standard mutagenesis techniques such as those described herein and then tested for their ability to mediate intracellular events in assays known to those skilled in the art including assays to measure GTPase activity or $G_a$ mediated intracellular events as described elsewhere in the present application. Directions to screen for RGS2 ligands are described in detail below. Such ligands may then be tested for their ability to inhibit and/or reduce RGS2 activity.

Alternatively, a RGS2 inhibitor may be a compound that binds to or interacts with a molecule that normally binds to or interacts with a RGS2 protein, thus preventing said RGS2 protein from exerting the effects it would normally exert. For example, such a RGS2 inhibitor may be a compound that mimics RGS2 structurally, but lacks its function such as a mutated RGS2 polypeptide or a fragment thereof. Directions to synthesize such mutated non-functional RGS2 polypeptides are described in detail below.

Compounds able to act as RGS2 activators may be compounds (e.g., small organic molecules, proteins or peptides) that bind to a RGS2 protein or fragment thereof and trigger or enhances its activity, i.e. an agonist or a fragment thereof. Directions to screen for RGS2 ligands are described in detail below. Such ligands may then be tested for their ability to trigger and/or increase RGS2 activity.

Alternatively, a RGS2 activator may be a compound that binds to or interacts with a molecule that normally binds to or interacts with a RGS2 protein, thus triggering said RGS2 protein to exerting its effects.

### a) *Ligand screening methods*

RGS2 ligands may be screened in so-called "ligand screening assays" using techniques well known to those skilled in the art. In such ligand screening methods, a biological sample or a defined molecule to be tested as a putative ligand of a RGS2 protein is brought into contact with RGS2 proteins or fragments thereof under conditions to allow the formation of a complex between said RGS2 protein or fragment thereof and the putative ligand molecule to be tested.

Depending on the type of assays used and on whether said RGS2 protein is secreted wholly (e.g. in the extracellular medium), partially (e.g. present at the membrane) or not at all (e.g. intracellular protein), different sources for said RGS2 protein or fragment thereof may be used including purified recombinant RGS2 protein, whole cells or cellular extracts of cells expressing RGS2 proteins, or extracellular medium of RGS2-expressing cells.

Various candidate ligands can be assayed for interaction with a RGS2 polypeptide or fragment thereof. These substances or molecules include, without being limited to, natural or synthetic organic compounds or molecules of biological origin such as polypeptides. Compounds may be identified from a variety of sources, for example, cells, cell-free preparations, chemical libraries, and natural product mixtures. Different types of assays are available depending on the type of compounds to be tested.

As an illustrative example, to study the interaction of a RGS2 protein or a fragment thereof with drugs or small molecules, such as molecules generated through combinatorial chemistry approaches, the microdialysis coupled to HPLC method described by Wang *et al.,* (1997), Chromatographic, 44 : 205-208, or the affinity capillary electrophoresis method described by Bush *et al.,* (1997), J. Chromatogr., 777 : 311-328, the disclosures of which are incorporated by reference, can be used.

In further methods, peptides, drugs, fatty acids, lipoproteins, or small molecules that interact with a RGS2 protein, or a fragment thereof may be identified using assays such as the followings. The molecule to be tested for binding is labeled with a detectable label, such as a fluorescent, radioactive, or enzymatic label and placed in contact with immobilized a RGS2 protein or a fragment thereof under conditions allowing specific binding to occur. After removal of non-specifically bound molecules, bound molecules are detected using appropriate means.

Such candidate ligands may also be screend using competition experiments, by affinity chromatography, by optical biosensor methods or any other techniques known to those skilled in the art. When the candidate ligand comprises a polypeptide, this polypeptide may be the resulting expression product of a phage clone belonging to a phage-based random peptide library, or alternatively the polypeptide may be the resulting expression product of a cDNA library cloned in a vector suitable for performing a two-hybrid screening assay.

A. Candidate ligands screened from random peptide libraries

In such screening methods, the putative ligand is the expression product of a DNA insert contained in a phage vector. Specifically, random peptide phages libraries encoding peptides of 8 to 20 amino acids in length are generally used.

The recombinant phage population is brought into contact with an immobilized RGS2 protein or fragment thereof. Then the preparation of complexes is washed in order to remove non-specifically bound recombinant phages. The phages that bind specifically to the RGS2 protein are then eluted using a buffer with an acid pH or immunoprecipitated by an anti-RGS2 antibody. The recovered phage population is subsequently amplified by an over-infection of bacteria (for example E. coli). The selection step may be repeated several times, preferably 2-4 times, in order to select for more specific recombinant phage clones. Finally, the peptide produced by each recombinant phage clone is characterized by expression in infected bacteria and isolation, by expression of the phage insert in another host-vector system, or by sequencing the phage insert.

Teachings to build random peptide libraries and use them in screening assays may be found in Parmley and Smith, (1988) Gene 73: 305-318; Cortese *et al* (199) Curr Opin Biotechnol; 7(6):616-21; Oldenburg *et al.,* (1992), Proc. Natl. Acad. Sci. USA 89:5393-539; Valadon *et al.,* (1996), J. Mol. Biol., 261:11-22; Westerink, (1995), Proc. Natl. Acad Sci USA., 92:4021-4025; Felici, (1991), J. Mol. Biol., 222:301-310; US Patent Nos 6,180,343, US 5,834,318; US 5,747,334; US 5,564,325, US 6,107,059, US 5,733,731, US 5,723,286; US 5,498,530 EP 0990051, WO 9846 796, which disclosures are hereby incorporated by reference in their entireties.

B. Candidate ligands screened using competition experiments.

Compounds such as peptides, drugs or small molecules that bind to a RGS2 protein or fragment thereof may be identified in competition experiments. In such assays, the RGS2 protein, or a fragment thereof, is immobilized to a surface, such as a plastic plate. Increasing amounts of the compounds to be tested are placed in contact with the immobilized RGS2 protein, or a fragment thereof, in the presence of a detectable labeled known RGS2 protein ligand such as $G_\alpha$, preferably Gq, or a fragment thereof retaining the ability to bind to RGS2. For example, the RGS2 ligand may be detectably labeled with a fluorescent, radioactive, or enzymatic tag. The ability of the test molecule to bind the RGS2 protein, or a fragment thereof, is determined by measuring the amount of detectably labeled known ligand bound in the presence of the test molecule. A decrease in the amount of known ligand bound to the RGS2 protein, or a fragment thereof, when the test molecule is present indicated that the test molecule is able to bind to the RGS2 protein, or a fragment thereof. Teachings to practise such competitive assays may be found in US 5,434,052, WO 0171314, WO 0079260, US 6,322,992, and in US 2001 0026944, which disclosures are incorporated by reference in their entireties

C. Candidate ligands screened using affinity chromatography.

Proteins or other molecules interacting with a RGS2 protein, or fragment thereof, may also be screened using affinity columns onto which the RGS2 protein or fragment thereof is bound using conventional techniques, e.g. chemical coupling to a suitable column matrix such as agarose, Affi Gel®, or other matrices familiar to those of skill in art. In such methods, the affinity column may contain chimeric proteins comprising the RGS2 protein or fragment thereof fused to glutathion S transferase (GST). A mixture of compounds, such as a cellular protein extract, is then run through the affinity column. Proteins or other molecules interacting with the immobilized RGS2 protein or fragment thereof attached to the column can then be isolated and analyzed, for example using 2-D electrophoresis gel as described in Ramunsen *et al.,* (1997), Electrophoresis, 18: 588-598, the disclosure of which is incorporated by reference. Alternatively, the proteins retained on the affinity column can be purified using electrophoresis-based methods and then sequenced (see for example Evans *et al.,* (1996) Nat Biotechnol 14(4):504-7; Huang and Carbonell, (1999) Biotechnol. Bioeng. 20;63(6):633-41, which disclosures are incorporated by reference in their entireties), The same method can be used to isolate antibodies, to screen phage display products, or to screen phage display human antibodies.

D. Candidate ligands obtained by optical biosensor methods

Proteins interacting with a RGS2 protein or fragment thereof can also be screened using an Optical Biosensor as described in Edwards and Leatherbarrow, (1997) Analytical Biochemistry, 246, 1-6 and also in Szabo *et al.,* (1995) Curr Opin Struct Biol 5, 699-705, the disclosures of which are incorporated by reference. This technique allows the detection of interactions between molecules in real time, without the need of labeled molecules.

For screening of candidate ligand molecules, the RGS2 protein, or a fragment thereof, is immobilized onto a surface (such as a carboxymethyl dextran matrix). Then, a solution containing either a compound to be tested or a mixture of compounds such as a cellular extract flows onto the surface onto which the RGS2 protein or fragment thereof is bound. Binding of a compound to the RGS2 protein or fragment thereof, is detected as a change of the surface plasmon resonance signal. The candidate molecules tested may be proteins, peptides, carbohydrates, lipids, or small molecules generated by combinatorial chemistry. This technique may also be performed with immobilized eukaryotic or prokaryotic cells or lipid vesicles exhibiting an endogenous or a recombinantly expressed RGS2 protein at their surface.

The main advantage of the method is to allow the determination of the association rate between the RGS2 protein or fragment thereof and interacting compounds. It is thus possible to select specifically ligand molecules interacting with the RGS2 protein, or a fragment thereof, with desired association and dissociation kinetics.

E. Candidate ligands screened through a two-hybrid screening assay.

Proteins able to bind to RGS2 proteins or fragments thereof may also be screened using two-hybrid assays and its derivatives such as those described in Fields and Song (1989) Nature 340:245; Bram *et al.,* (1993), *Mol. Cell Biol.,* 13 : 4760-4769; Harper *et al.,* (1993), Cell, 75 : 805-816 ; Fields and Sternglanz (1994) Trends in Genetics 10: 286-292; Cho *et al.,* (1998), *Proc. Natl. Acad. Sci. USA,* 95(7) : 3752-3757; Fromont-Racine *et al.,* (1997), Nature Genetics, 16(3) : 277-282 ; Bartel *et al.* (1993) Biotechniques 14:920-924, Karimora *et al.,* (1998) Proc Nad. Acad. Sci. USA 95(10):5752-5756; WO 9410300, WO 99/28746, WO0173108, US 20010024794, US 5,667,973, US 5,283,173, US 6,251,676,US 6,2051,381, which disclosures are incorporated by reference in their entireties. Alternatively, kits such as the Matchmaker Two Hybrid System 2 (Catalog No. K1604-1, Clontech) may be used as described in the manual accompanying the kit, the disclosure of which is incorporated herein by reference.

That system utilizes chimeric genes and detects protein-protein interactions via the activation of reporter-gene expression. Reporter gene expression occurs as a result of reconstitution of a functional transcription factor caused by the association of fusion proteins encoded by the chimeric genes. Indeed, two-hybrid assays rely upon the fact that the DNA binding and polymerase activation domains of many transcription factors, such as GAL4 , can be separated and then rejoined to restore functionality.

Typically, polynucleotides encoding two hybrid proteins are constructed and introduced into a yeast host cell. The first hybrid protein consists of the yeast Gal4 DNA-binding domain fused to a RGS2 protein or fragment thereof (often referred to as the "bait"). The second hybrid protein consists of the Gal4 activation domain fused to a polypeptide sequence of a second protein to be tested (often referred to as the "prey"). Polynucleotides encoding proteins to be tested may be issued from DNA libraries, preferably cDNA libraries. Binding between the two hybrid proteins reconstitutes the Gal4 DNA-binding domain with the Gal4 activation domain, which leads to the transcriptional activation of a reporter gene (e.g., lacZ, URA3 or HIS3 ), which is operably linked to a Gal4 binding site.

*b) Non functional RGS2 polypeptides:*

Non functional RGS2 polypeptides are polypeptides that are very similar to RGS2 polypeptides as described herein in the Definition section but that do not exhibit a substantial RGS2 biological activity, as defined above.

Such non functional RGS2 polypeptides may arise naturally or may be obtained using mutagenesis techniques well known to those skilled in the art. Recombinant DNA technology can be used to create novel mutant proteins or muteins including single or multiple amino acid substitutions, deletions, additions, or fusion proteins.

Particularly interesting non functional RGS2 polypeptides able to inhibit normal RGS2 activity are those fragments of RGS2 polypeptides that retain the ability to interact with RGS2 ligands but that are deprived of functional domains for exerting RGS2 activity.

Standard techniques to mutate proteins include N-terminal and/or C-terminal deletions. Generally, such deletions are progressive in order to determine the minimal region that should be deleted in order to diminish significantly, or abolish, a desired RGS2 activity.

Other mutations in addition to N- and C-terminal deletion forms of the protein discussed above may be carried out in order to decrease or abolish a desired RGS2 activity. Such mutations are generally those that affect the structure, or the function of a RGS2 polypeptide or both. If such mutations are contemplated, it should be remembered that there are critical areas on the protein that determine activity. In order to determine such critical residues, an approach for studying the tolerance of an amino acid sequence to changes (See, Bowie *et al.* (1994) supra) that relies on the process of evolution may be used.

Domains and/or critical amino acids in the RGS2 proteins of the present invention that are essential for function can also be identified by methods known in the art such as site-directed mutagenesis (Braisted and Wells, (1996) Proc. Natl. Acad. Sci. USA, 93:5688-5692; Wells (1996) Proc. Natl. Acad. Sci. USA, 93:1-6), alanine-scanning mutagenesis (Cunningham *et al.* (1989), Science 244:1081-1085, Clackson and Wells (1995) Science, 267(5196):383-6; Leung and Hall (2000) Trends Cardiovasc. Med. 10(2):89-92), cysteine-scanning mutagenesis (Friligos *et al.* (1998) Faseb J. 12: 1281-1299), and pentapeptide scanning mutagenesis (Hayes and Hallet (2000) Trends Microbiol; 8(12):571-7), which disclosures are incorporated by reference in their entireties). In such techniques, amino acid changes genetic engineering are introduced at specific positions of a cloned gene and the resulting mutant molecules are then tested for biological activity using assays appropriate for measuring the function of the particular protein in order to identify sequences that maintain functionality. The studies indicate which amino acid changes at certain position sof the RGS2 protein are likely to affect RGS2 activity.

Alternatively, libraries of nucleic acids encoding mutated RGS2 polypeptides generated by random mutagenesis may be employed to reveal principles of protein structure. For example, cassette mutagenesis has been used to probe the "information content" of polypeptide sequences (Reidhaar-Olson and Sauer (1988) Science, 241(4861):53-7; Davidson and Sauer (1994) Proc Natl Acad Sci, 91(6):2146-50; Davidson *et al.,* (1995) Nat Struct Biol, 2 (10) p856-64, which disclosures are incorporated by reference in their entireties). These studies involve the construction of polypeptide mutants composed of random combinations of selected amino acids. The mutants are then analyzed for their thermal denaturation properties. Current methods for creating mutant proteins in a library format include but are limited to error-prone PCR-based techniques (See e.g. Leung *et al.,* (1989) Technique 1:11-15; Caldwell and Joyce (1992) PCR Methods and Applications 2:28-33; Gramm *et al.,* (1992) Proc. Natl. Acad. Sci. USA 89:3576-3580, which disclosures are incorporated by reference in their entireties) and cassette mutagenesis (Stemmer *et al.,* (1992) Biotechniques 14:256-265, Arkin and Youvan (1992) Proc. Natl. Acad. Sci. USA 89:7811-7815; Oliphant *et al.,* (1986) Gene 44:177-183; Hermes *et al.,* (1990) Proc. Natl. Acad. Sci. USA 87:696-700; Delagrave *et al.* (1993) Protein Engineering 6:327-331; Delgrave *et al.* (1993) Bio/Technology 11:1548-1552; Goldman, E. R. and Youvan D. C. (1992) Bio/Technology 10:1557-1561), in which the specific region of a polypeptide to be tested is replaced with a synthetically mutagenized oligonucleotide. Alternatively, mutator strains of host cells may be employed to add mutational frequency (see e.g. Greener and Callahan (1995) *Strategies in Mol. Biol.* 7: 32).

Domains and/or critical amino acids for RGS2 activity have been described in Heximer *et al.* (1999) *supra,* Zheng *et al.* (1999) *supra,* Chatterjee and Fisher (2000) *supra,* Sullivan *et al.* (2000) *supra,* and Heximer *et al.* (2001) *supra.* Therefore, non-functional RGS2 polypeptides preferably encompass polypeptides having mutated RGS domains. Illustrating but not limiting examples of such non-functional RGS2 polypeptides include a N-terminus truncated human RGS2 lacking the first 66 amino acids as described in Heximer *et al.* (2001), *supra,* and a human RGS2 in which the Asn residue in position 159 is mutated as described in Zheng *et al.* (1999) *supra.*

2.2. Methods of screening for compounds modulating RGS2 expression and/or activity during macrophage activation

Compounds suspected of modulating RGS2 expression and/or activity such as compounds identified by the above described methods, namely compounds able to bind to regulatory sequences of RGS2 polypeptides, antisense and triple helix polynucleotides, anti-RGS2 antibodies, RGS2 ligands and non functional RGS2 polypeptides, may then be tested for their ability to effectively modulate RGS2 expression and/or activity during macrophage activation. Alternatively, libraries of compounds that have not been pre-selected using any of the methods described above, may be screened directly for their ability to modulate RGS2 expression and/or activity during macrophage activation.

Therefore, the invention also relates to methods of determining whether a compound modulates RGS2 expression and/or activity as induced by macrophage activation or present in already activated macrophages.

In such screening assays, the compound to be tested is contacted with macrophages either before activation or after activation under conditions permissive for the quantification of macrophagic RGS2 expression and/or activity. The RGS2 expression and/or activity level is then measured and compared to the level of RGS2 expression and/or activity of control samples. Such controls typically include activated macrophages not contacted with the compound and/or non activated macrophages. A significant decrease in RGS2 expression and/or activity in samples containing said compound to be tested compared to samples not containing said compound is indicative of a compound able to reduce macrophage activation.

Screening assays for compounds modulating RGS2 expression and/or activity during macrophage activation

Screening assays for compounds modulating RGS2 expression and/or activity during macrophage activation may be of any type one skilled in the art could envision and include organisms, cells, cell lysates or cell-free systems.

*In vitro methods*

Cells expressing RGS2 are incubated *in vitro* in the presence and absence of the test compound. By determining the level of RGS2 expression and/or activity in the presence of the test compound and comparing it to the RGS2 expression and/or activity level in the absence of test compound, compounds can be identified that modulate RGS2 expression or activity. Alternatively, when the ability of a compound to interact with regulatory sequences of a RGS2 polynucleotide needs to be assessed, constructs comprising a RGS2 regulatory sequence operably linked to a reporter gene (e.g. luciferase, chloramphenicol acetyl transferase, LacZ, green fluorescent protein, etc.) can be introduced into host cells and the effect of the test compounds on expression of the reporter gene detected. Cells suitable for use in the foregoing assays include, but are not limited to, cells having the same origin as tissues or cell lines in which the RGS2 polypeptide is known to be expressed such as macrophages, macrophagic cell lines, acute leukemia cell lines (see Wu *et al.* (1995) *supra)* or such as host cells engineered to express RGS2.

*In vivo methods*

Compounds that modulate RGS2 expression and/or activity can also be identified using *in vivo* screens. In these assays, the test compound is administered (e.g. IV, IP, IM, orally, or otherwise), to the animal, for example, at a variety of dose levels. The effect of the compound on RGS2 expression and/or activity is determined by comparing RGS2 expression and/or activity levels, for example in cells or tissues known to express the gene of interest such as macrophages, heart, brain, spleen, lung, skeletal muscle, kidney, and using any of the quantifying assays described herein. Suitable test animals include rodents (e.g., mice and rats), and primates. Humanized mice can also be used as test animals, that is mice in which the endogenous mouse protein is ablated (knocked out) and the homologous human protein added back by standard transgenic approaches (sse techniques described herein in the "gene therapy" section). Such mice express only the human form of a protein. Humanized mice expressing only the human RGS2 can be used to study *in vivo* responses of activated-macrophages-related disorders such as inflammatory disorders in response to potential compounds regulating RGS2 expression and/or activity levels.

## VI. Kits

Another object of the invention is to provide kits containing necessary reagents for conducting methods according to the invention, namely methods of determining whether macrophages are activated in a biological sample, methods of determining whether an individual suffers from an activated-macrophage-related disorder, methods of determining whether an individual is susceptible to develop an activated-macrophage-related disorder, methods of monitoring an individual suffering from an activated-macrophage-related disorder, methods of assessing the efficacy of a treatment for an individual suffering from an activated-macrophage-related disorder, and methods of determining whether an agent modulate macrophage activation.

Such kits contain necessary reagents for carrying out any type of assays disclosed herein (hybridization-based assays, amplification-bases assays, protein quantification assays, RGS2 activity assays) in order to quantify RGS2 expression and/or activity level in a test sample. Eventually, control samples and/or reference amounts of RGS2 expression and/or activity in control conditions may be provided in order to determine whether the RGS2 expression and/or activity level is elevated in said test sample compared to another situation.

Instructions for use of the kit reagents are also typically included. Instructions for use generally include a description of the reagent concentration, or at least one assay method parameter, such as the relative amounts of reagent and sample to be admixed, maintenance time periods for reagent/sample admixtures, temperature, buffer conditions, and the like.

## VII. Treating and preventing activated-macrophage-related disorders

The present invention further relates to methods of preventing or treating activated-macrophage-related disorders by modulating, preferably decreasing RGS2 activity and/or expression. Such methods use compounds acting as RGS2 modulators, preferably RGS2 inhibitors such as those selected using screening protocols described herein, in pharmaceutical compositions and/or gene therapy approaches as described in the art and herein.

### Pharmaceutical and physiologically acceptable compositions

The present invention also relates to pharmaceutical or physiologically acceptable compositions comprising, as active agent, the polypeptides, nucleic acids or antibodies of the invention. More particularly, the invention relates to compositions comprising, as active agent, agents and compounds selected using the above-described screening protocols such as non-functional RGS2 polypeptides, anti-RGS2 antibodies, RGS2 antagonists or inhibitors, RGS2 agonists or activators. Preferably, the invention relates to compositions comprising, as active agent, RGS2 antagonists or inhibitors, RGS2 antisense oligonucleotides, oligonucleotides able to form a triple helix with RGS2 polynucleotides, non-functional RGS2 polypeptides and/or anti-RGS2 antibodies. Such compositions include the active agent in combination with a pharmaceutical or physiologically acceptable carrier. In the case of naked DNA, the "carrier" may be gold particles. The amount of active agent in the composition can vary with the agent, the patient and the effect sought. Likewise, the dosing regimen can vary depending on the composition and the disease/disorder to be treated.

Therefore, the invention relates to methods for the production of pharmaceutical composition comprising a method for selecting an active agent, such as a RGS2 modulator, preferably a RGS2 inhibitor, using any of the screening method described herein and furthermore mixing the identified active agent, with a pharmaceutically acceptable carrier.

The pharmaceutical compositions utilized in this invention may be administered by any number of routes including, but not limited to, oral, intravenous, intramuscular, infra-arterial, intramedullary, intrathecal, intraventricular, transdermal, subcutaneous, intraperitoneal, intranasal, enteral, topical, sublingual, or rectal means. In addition to the active ingredients, these pharmaceutical compositions may contain suitable pharmaceutically acceptable carriers comprising excipients and auxiliaries which facilitate processing of the active compounds into preparations which can be used pharmaceutically. Further details on techniques for formulation and administration may be found in the latest edition of Remington's Pharmaceutical Sciences (Maack Publishing Co. Easton, Pa).

Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

Pharmaceutical preparations for oral use can be obtained through a combination of active agents with solid excipient, sulting mixture is optionally grinding, and processing the mixture of granules, after adding suitable auxiliaries, if desired, to obtain tablets or dragee cores. Suitable excipients are carbohydrate or protein fillers, such as sugars, including lactose, sucrose, mannitol, or sorbitol; starch from corn, wheat, rice, potato, or other plants; cellulose, such as methyl cellulose, hydroxypropylmethyl-cellulose, or sodium carboxymethylcellulose; gums including arabic and tragacanth; and proteins such as gelatin and collagen. If desired, disintegrating or solubilizing agents may be added, such as the cross-linked polyvinyl pyrrolidone, agar, alginic acid, or a salt thereof, such as sodium alginate.

Dragee cores may be used in conjunction with suitable coatings, such as concentrated sugar solutions, which may also contain gum arabic, talc, polyvinylpyrrolidone, carbopol gel, polyethylene glycol, and/or titaniumdioxide, lacquer solutions, and suitable organic solvents or solvent mixtures. Dyestuffs or pigments may be added to the tablets or dragee coatings for product identification or to characterize the quantity of active agent, i.e., dosage.

Pharmaceutical preparations which can be used orally include push-fit capsules made of gelatin, as well as soft, sealed capsules made of gelatin and a coating, such as glycerol or sorbitol. Push-fit capsules can contain active ingredients mixed with a filler or binders, such as lactose or starches, lubricants, such as talc or magnesium stearate, and, optionally, stabilizers. In soft capsules, the active agents may be dissolved or suspended in suitable liquids, such as fatty oils, liquid, or liquidpolyethylene glycol with or without stabilizers.

Pharmaceutical formulations suitable for parenteral administration may be formulated in aqueous solutions, preferably in physiologically compatible buffers such as Hanks solution, Ringer's solution, or physiologically buffered saline. Aqueous injection suspensions may contain substances which increase the viscosity of the suspension, such as sodium carboxymethylcellulose, sorbitol, or dextran. Additionally, suspensions of the active agents may be prepared as appropriate oily injection suspensions. Suitable lipophilic solvents or vehicles include fatty oils such as sesame oil, or synthetic fatty acid esters, such as ethyl oleate or triglycerides, or liposomes. Optionally, the suspension may also contain suitable stabilizers or agents which increase the solubility of the compounds to allow for the preparation of highly concentrated solutions.

For topical or nasal administration, penetrants appropriate to the particular barrier to be permeated are used in the formulation. Such penetrants are generally known in the art.

The pharmaceutical compositions of the present invention may be manufactured in a manner that is known in the art, e.g., by means of conventional mixing, dissolving, granulating, dragee-making, levigating, emulsifying, encapsulating, entrapping, or lyophilizing processes.

The pharmaceutical composition may be provided as a salt and can be formed with many acids, including but not limited to, hydrochloric, sulfuric, acetic, lactic, tartaric, malic, succinic, etc. Salts tend to be more soluble in aqueous or other protonic solvents than are the corresponding free base forms. In other cases, the preferred preparation may be a lyophilized powder which may contain any or all of the following: 1-50 mM histidine, 0.1%-2% sucrose, and 2-7% mannitol, at a pH range of 4.5 to 5.5, that is combined with buffer prior to use.

After pharmaceutical compositions have been prepared, they can be placed in an appropriate container and labeled for treatment of an indicated condition. For administration, such labeling would include amount, frequency, and method of administration.

Pharmaceutical compositions suitable for use in the invention include compositions wherein the active agent is contained in an effective amount to achieve the intended purpose. The determination of an effective dose is well within the capability of those skilled in the art.

For any compound, the therapeutically effective dose can be estimated initially either in cell culture assays, e.g., of neoplastic cells, or in animal models, usually mice, rabbits, dogs, or pigs. The animal model may also be used to determine the appropriate concentration range and route of administration. Such information can then be used to determine useful doses and routes for administration in humans.

A therapeutically effective dose refers to that amount of active agent which ameliorates the symptoms or condition. Therapeutic efficacy and toxicity may be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50. Pharmaceutical compositions which exhibit large therapeutic indices are preferred. The data obtained from cell culture assays and animal studies is used in formulating a range of dosage for human use. The dosage contained in such compositions is preferably within a range of circulating concentrations that include the ED50 with little or no toxicity. The dosage varies within this range depending upon the dosage form employed, sensitivity of the patient, and the route of administration.

The exact dosage will be determined by the practitioner, in light of factors related to the subject that requires treatment. Dosage and administration are adjusted to provide sufficient levels of the active moiety or to maintain the desired effect. Factors which may be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions maybe administered every 3 to 4 days, every week, or once every two weeks depending on half-life and clearance rate of the particular formulation.

Normal dosage amounts may vary from 0.1 to 100,000 micrograms, up to a total dose of about 1 g, depending upon the route of administration. Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc.

**Gene therapy**

Gene therapy can be used to trigger or to enhance the expression of a gene of interest, such as for example a gene encoding a RGS2 inhibitor or a RGS2 polypeptide, in an individual suffering from an activated-macrophage-related disorder. In such approaches, the coding sequence of the gene of interest can be cloned into an appropriate expression vector and targeted to a particular cell type(s), such as macrophages, to achieve an efficient high level expression using methods known to those skilled in the art. Introduction of the coding sequence for the gene of interest into target cells can be achieved, for example, using particle mediated DNA delivery, (Haynes *et al., (1996)* J Biotechnol. 44(1-3):37-42 ; Maurer *et al.,* (1999) Mol Membr Biol. 16(1):129-40), direct injection of naked DNA, (Levy *et al.,* (1996) Gene Ther. 3(3):201-11; Felgner (1996) Hum Gene Ther. 7(15):1791-3), or viral vector mediated transport (Smith *et al.,* (1996) Antiviral Res. 32(2):99-115, Stone *et al.,* (2000) J Endocrinol. 164(2):103-18; Wu and Ataai, (2000) Curr Opin Biotechnol. 11(2):205-8), each of which disclosures are hereby incorporated by reference in their entireties.

Alternatively, the naturally occuring regulatory sequences of the gene of interest, such as the promoter and enhancer sequences, may be replaced by stronger regulatory sequences promoting a higher expression level of said gene of interest using homologous recombination techniques well known to those skilled in the art, such as those described in Koller and Smithies(1992) *Ann. Rev. Immun.,* 10:705-730; Morrison, and Takeda (2000) Int. J. Biochem. Sci. 32:817-831; Haber (2000) Trends Genet. 16:259-264; Karran (2000) curr. Opin. Genet. Dev. 10:144-150, Crichlow and Jackson (1998) Trends Biochem. Sci. 23:394-398, Paques and Haber, (1999) Microbiol. Mol. Biol. Rev. 63:349-404, Danska and Guidos (1997) Semin. Immunol. 9:199-206, Cheah and Berhinger (2000) Methods. Mol. Biol. 136:455-463, Lanzov (1999) Mol. Genet. Metab. 68:276-282, Muller (1999) Mech. Dev. 82:3-21, Sargent and Wilson (1998) curr. Res. Mol. Ther. 1:584-692, Jasin (1996) Trends Genet. 12:224-228, Waldamn (1992) Crit. Rev. Oncol. Hematol. 12: 49-64, U.S. Patent Nos. US 5,272,071, US 6,255,113, US 6,200,812, US 6 187 305, WO 91/06666, WO 91/06667, WO 90/11354, WO 91/09955, WO 96/41 008, EP 1152058, Chan *et al.* (1999), J. Biol. Chem., 274(17):11541-11548, Culver *et al.,* (1999), Nature Biotechnology, 17, 989-993 and using the teachings of Vasquez *et al.*, (2001) Proc. Natl. Acad. Sci. USA 98(15):8403-8410 and Lai and Lien (1999) Exp. Nephrol. 7(1):11-14, which disclosures are incorporated by reference in their entireties.

Alternatively, gene therapy may be used to decrease or inhibit the expression of an undesirable gene, such as a RGS2 inhibitor, in an individual suffering from an activated-macrophage-related disorder. For example, the expression of the undesirable gene may be reduced or inhibited using homologous recombination to knock-out gene expression by deleting the whole undesirable gene or part thereof such as coding sequences or regulatory sequences, or by replacing them by non-functional sequences using homologous recombination techniques known to those skilled in the art (see references describes above as well as US Patent No 6015676 and Galli-Taliadoros *et al.,* (1995) J Immunol. Methods ;181(1):1-15, which disclosures are incorporated by reference in their entireties).

Alternatively, decrease or inhibition of the expression of the undesirable gene may be achieved using an antisense or triple helix approaches as described elsewhere in the present application. For example, antisense oligonucleotides complementary to undesirable polynucleotides can be used to selectively diminish or ablate undesirable gene expression, for example, at sites of inflammation. More specifically, antisense constructs or antisense oligonucleotides can be used to inhibit the production of the undesirable gene in cells expressing high levels of undesirable proteins. Antisense mRNA can be produced by transfecting into target cells an expression vector with the undesirable gene sequence, or portion thereof, oriented in an antisense direction relative to the direction of transcription. Appropriate vectors include viral vectors, including retroviral, adenoviral, and adeno-associated viral vectors, as well as nonviral vectors. Alternatively, antisense oligonucleotides can be introduced directly into target cells to achieve the same goal. (See also other delivery methodologies described herein in connection with gene therapy.).

Tissue specific effects in gene therapy can be achieved using delivery means that are tissue-specific (e.g. viral vectors that are tissue specific in the case of virus mediated transport) or using promoter sequences that promote expression specifically in the tissue or cell of interest. For example, macrophage specific promoters may be used such as those described in Sidiropoulos *et al.,* (1997) Hum Gene Ther May 1;8(7):803-15; and Fabunmi *et al.,* (2000) Atherosclerosis Feb; 148(2):375-86. Combinatorial approaches can also be used to ensure that the desired coding sequence is properly activated in the target tissue (Miller and Whelan, (1997) Hum Gene Ther. 8(7):803-15), hereby incorporated by reference in its entirety .

The therapeutic methodologies described herein are applicable to both human and non-human mammals (including cats and dogs).

## VIII. Examples

**Example 1 : Detection of an increased level of RGS2 expression in a macrophagic cell line upon activation**

Cells and culture

U937 cells (ECACCn°85011440) (these cells being referred to as non differentiated cells) are monocyte-like cells that differentiate into macrophage-like cells upon addition of a differentiating agent. U937 cells were cultured at 37°C, 5% $CO_2$, in suspension, RPMI-1640 + glutamax, 10% bovine fetal serum (Life Technologies) at pH 7.0 and cells were maintained at $0.25 \times 10^6$ to $10^6$ cells/ml. Differentiation of U937 cells into macrophage-like cells (these cells being referred to as differentiated cells) was carried out using 1 μM dibutyryl cyclic AMP (dbcAMP) for 48 hours. Differentiation was controlled by FACS analysis checking for an increased expression of CD32 and decreased expression of CD64 as previously described (Floto *et al.,* (1996) J. Physiol. 492:331-338) using anti-CD32 and anti-CD64 antibodies labeled with FITC. In addition, subsequent release of $Ca^{2+}$ from intracellular compartments in differentiated cells followed by calcium influx was then checked using fura-2 charged cells as described in Davis *et al.* (1995) Cell Calcium;17(5): 345-53. Briefly, intracellular calcium concentration was measured out using a Shimadzu Fluorometer, after charging cells by fura-2 in presence of BSA during 45 minutes at 37°C RPMI-16 Both non differentiated U937 cells and differentiated U937 cells were washed and calcium signaling stimulated by adding 600 nM of thapsigargin (in RPMI 1640 pH7 in absence of serum and phenol red), an inhibitor of the endoplasmic reticulum $Ca^{2+}$-ATPase of SERCA type (Thastrup *et al.,* (1990) Proc. Natl. Acad. Sci. USA 87:2466-270). Then, the intracellular calcium level was monitored for one hour.

RNA extraction

Total RNA was extracted with Trizol (Life Technologies) from $10^7$ cells/prep. Precipitated total RNA (average yield 100μg) was taken back in 40 μl and analyzed on agarose (1%) gel stained by Ethidium bromide. cDNA synthesis was carried out using Power Script Reverse Transcriptase (Clontech) with 5pg of total RNA / in 20 μl preparation mix, corresponding to the suppliers protocol, using random hexamer primers.

Quantitative real time PCR

Real time PCR was performed on a Gene Amp 5700 (Applied Biosystems) using SybrGreen technology (SybrGreen PCR Master Mix from Applied Biosystems, Part Number : 4309155 or Quantitect SybrGreen PCR kit from Qiagen, Catalog No 204143). Primers of SEQ ID Nos 1 and 2 were used to amplify a portion of RGS2 polynucleotides and primers of SEQ ID Nos 3 and 4 were used to amplify 18S ribosomal RNA chosen as the reference gene in those assays. Primers were used in a final concentration of 300nM with the quantity of cDNA of 2% of the cDNA preparation (corresponding to 100ng total RNA). After a preliminary step of 2 minutes at 50°C and a step of DNA polymerase activation of 10 minutes at 95°C, subsequent thermal cycles were carried out as follows: 10 seconds at 95°C to allow denaturation of double stranded cDNA and 1 minute at 60°C to allow annealing of primers and elongation of cDNA strands.

Results

Results are expressed as follows. The experiment was done in the six following different cell-culture experimental conditions:

1) non differentiated non activated U937, condition herein referred to as nd0h;
2) non differentiated U397 after 1 hour of activation, condition herein referred to as ndlh;
3) non differentiated U397 after 5 hours of activation, condition herein referred to as nd5h;
4) differentiated non activated U937, condition herein referred to as dbc0h;
5) differentiated U397 after 1 hour after activation, condition herein referred to as dbc1h;
6) differentiated U397 after 5 hours after of activation, condition herein referred to as dbc5h.

The number of cycles upon which the signal threshold is reached for RGS2 in a given experimental condition, herein referred to as Ct, was normalized by subtraction of the variation of Ct for the gene of reference, here 18S RNA, from the same RNA preparation. The geometric mean of all six experimental conditions, herein referred to as μCt, was then calculated and the relative gene expression level expressed as $2^{(\mu Ct-Ct)}$ for each experimental condition (see Table I).

Relative gene expression levels of two different experimental conditions, namely x and y, are then considered to be significantly different when the ratio of relative gene expression levels, i.e. x/y, is higher than 1.7, where x has a relative gene expression level higher than y.

RGS2 was thus classified as being specific for macrophage activation because the following criteria were met (see Table I):

a) the highest relative gene expression level of all six different experimental conditions (N) was observed in differentiated cells after activation, i.e. for dbc1h and/or dbc5h.
b) the highest relative gene expression level (N) was different from the relative gene expression level of differentiated, non-activated cells, i.e. N/dbc0h> 1.7.
c) the highest relative gene expression level (N) was different from the highest relative gene expression level of non-differentiated cells, i.e. N/max(nd0h, nd1h, nd5h) > 1.7.

As could be seen from Table I which present results from quadruplicate measures from 4 independent cell cultures, the highest relative gene expression level (N) was actually observed in differentiated cells after 1 hour of activation. This highest relative gene expression level is different by approximately 21-fold from the relative gene expression level of differentiated, non-activated cells. In addition, this highest relative gene expression level is different by 3.5-fold from the relative gene expression level of non-differentiated cells. Hence, RGS2 is specifically expressed upon macrophage activation compared to monocyte activation where RGS2 expression increases to a lesser extent.

Table I.

| Relative RGS2 expression in non differentiated and differentiated U937 upon activation as assessed using real-time RT-PCR. | | | | | | | |
|---|---|---|---|---|---|---|---|
| | nd0h | nd1h | nd5h | dbc0h | dbc1h | dbc5h | Mean |
| Ct | 31.87 | 28.76 | 28.92 | 31.24 | 27.02 | 26.97 | $\mu$Ct=29.13 |
| $2^{(\mu Ct-Ct)}$ | 0.18 | 1.32 | 1.39 | 0.24 | 4.91 | 4.51 | |
| N/dbc0h | 20.72 | | | | | | |
| N/max(nd0h, nd1h, nd5h) | 3.54 | | | | | | |

**Example 2 : Detection of RGS2 polynucleotide expression levels in an animal model for COPD**

Smoking mice model

Male A/J mice ages from 6 weeks known to present pulmonary susceptibility are challenged with tobacco smoke in order to induce emphysema. Animals are challenged with the smoke of 1 non-filtered cigarette per 10 animals, twice a day, 30 minutes each time and 5 days a week using a large-whole body chamber. Non-smoking, age-matched littermates are used as controls.

Broncho alveolar lavages

Animals are sacrificed at T0 (start of experiment), and at different time points such as T1 (1 month) and T4 (4 months) to recover cells from broncho alveolar lavages (BALs) as follows. A tracheotomy is realized and the trachea canulated. Then, the lungs are rinsed six times using a syringe with a sterile saline PBS solution (PBS without calcium, 10 $\mu$molar genistein, 10 $\mu$molar cycloheximide, 2.7 mM EDTA, 1mM orthovanadate, 1 tablet/50ml of a complete cocktail of protease inhibitors (Roche, catalog number 1873580)) warmed at 37°C (about 0.3 ml each time) and the fluid recovered by gentle aspiration. The cell suspension is collected into a conical tube, centrifuged and resuspended into nutritive medium, RPMI 640 containing 10% fetal calf serum.

Cells are then counted using a cell counter such as a Coulter Z2 in the presence of Zap-globin. Staining using successively May Grunwald and Giemsa stains (MGG) allows the study of the morphology of leukocytes and to determine the ratio of each population (macrophages, neutrophils, lymphoytes). Cells from the various BALs are pooled, washed with the saline solution by centrifugation at 300g 30 minutes at 4°C and resuspended in l ml of saline solution.

Enrichment in macrophages

To enrich for the macrophages population, cells recovered from BALs are then passed through a discontinuous Percoll gradient. This discontinuous Percoll gradient is made as follows. A stock solution made with 9 volumes of Percoll (Amersham Pharmacia Biotech APB catalog number 17-0891-01) and 1 volume of 1.5 M NaCl is diluted with 1.5 M NaCl to make two solutions at 1.01 and 1.072 g/ml Percoll using the following formula:

$$V_y = V_i \left[ (\rho_i - \rho)/(\rho - \rho_y) \right]$$

$V_y$ = volume of diluting medium in ml.
$V_i$ = volume of stock isotonic Percoll (SIP) in ml.
$\rho_i$ = density of stock isotonic Percoll (SIP) in g/ml = 1.123 g/ml.
$\rho_y$ = density of 0.15 M NaCl$\cong$ 1.0046 g/ml
$\rho$ = density of diluted solution produced in g/ml

To correct for slight variations in volumes and densities of diluting media that affects final density of Percoll solutions, the actual final density of Percoll solutions is measured with a variation of $\pm$ 0,001 g/ml using a densitometer (or a refractometer) or density markers beads (APB - Catalog number: 17-0459-01) as external markers.

The Percoll solutions of 1.01 and 1.072 g/ml respectively are then carefully layered one on the top of the other using, for example, a peristaltic pump set at a low flow rate, in low attachment tubes such as those in Polylabo catalog number 13183.01 starting with the densest solution at the bottom of the tube. The preformed gradient is then placed at 4°C.

The cell suspension recovered from BALs is gently layered on the top of the gradient and the cells are separated using a swing bucket head by centrifugation at 300g for 30 minutes at 4°C. Then, the interface is carefully collected with a Pasteur pipette and cells are pelleted by centrifugation (300 g for 30 minutes at 4°C).

Percoll-isolated macrophages harvested from this interface are routinely purified up to 98%, as identified by MGG. Usual yield is between 50 and 70 %.

An optional washing step with saline solution may be performed by centrifugation at 300 g for 10 minutes at 4°C to remove Percoll.

Quantification of RGS2 expression levels

Macrophagic RNA is then extracted using methods similar to the one described in Example 1. RGS2 expression levels are then quantified using real-time PCR essentially as described in Example 1 with the exception that the amplification primers are designed on the mouse RGS2 (Chen *et al.* (1997) *supra).* The same parameters are measured (Ct) or computed (uCt, relative gene expression level $2^{(\mu Ct-Ct)}$, etc) and the same criteria to define groups with similar or different expression levels. Then, a man skilled in the art uses criteria, such as those described above, to determine whether macrophages are activated in smoking mice. Examples of such criteria may be the following:

a) the highest relative gene expression level (N) must be observed in smoking mice at either T1 orT4.
b) the highest relative gene expression level (N) must be significantly different from the relative gene expression level of smoking mice at T0.
c) the highest relative gene expression level (N) must be significantly different from the highest relative gene expression level of non smoking mice whatever time point is considered.

The contents of all patents, patent applications, published articles, books, reference manuals and abstracts cited herein are hereby incorporated by reference in their entirety to more fully describe the state of the art to which the invention pertains.

As various changes can be made in the above-described subject matter without departing from the scope and spirit of the present invention, it is intended that all subject matter contained in the above description, or defined in the appended claims, be interpreted as descriptive and illustrative of the present invention. Many modifications and variations of the present invention are possible in light of the above teachings.

**Sequence listing free text**

Sequence source:/note=synthetic oligonucleotide *in Molecular Biology;* Harlow and Lane, *Antibodies: A Laboratory Manual,* Cold Spring Harbor Laboratory, New York (1988), Hammerling, *et al,* (1981) Monoclonal Antibodies and T Cell Hybridomas, Elsevier, N.Y. 563-681). (Said references incorporated by reference in their entireties).

Antigen binding fragments such as Fab, F(ab')2 and ssFv fragments may be produced, for example, from hybridoma-produced antibodies, by proteolytic cleavage, using enzymes such as papain (to produce Fab fragments) or pepsin (to produce F(ab')2 fragments) or other proteases.

Alternatively, antibodies of the present invention can be produced through the application of recombinant DNA technology or through synthetic chemistry using methods known in the art. For example, in phage display methods, functional antibody domains arc displayed on the surface of a phage particle, which carries polynucleotide sequences encoding them. Phages with a desired binding property, e.g. a RGS2 polypeptide binding property, are selected from a repertoire or combinatorial antibody library (e.g. human or murine) by selecting directly with antigen, typically antigen bound or captured to a solid surface or bead. Phage used in these methods are typically filamentous phage including fd and M13 with Fab, Fv or disulfide stabilized Fv antibody domains recombinantly fused to either the phage gene III or gene VIII protein. Examples of phage display methods that can be used to make the antibodies of the present invention include those disclosed in Brinkman *et al.* (1995)*J. Immunol Methods.* 182:41-50; Ames, *et al.* (1995) *J. Immunol. Meth.* 184:177-186; Kettleborough, *et al.* (1994) Eur. L Immunol. 24:952-958; Persic, *et al.* (1997) Gene. 1879-81; Burton *et al.* (1994), *Adv. Immunol.* 57:191-280; PCT/GB91/01134; WO 90/02809; WO 91/10737; WO 92/01047; WO 92/18619; WO 93/11236; WO 95/15982; WO 95/20401; and US Patents 5,698,426, 5,223,409, 5,403,484, 5,580,717, 5,427,908, 5,750,753, 5,821,047, 5,571,698, 5,427,908, 5,516,637, 5,780,225, 5,658,727 and 5,733,743 (said references incorporated by reference in their entireties).

If necessary as assessed by one skilled in the art, polyclonal or monoclonal antibodies can be further purified, for example, by binding to and elution from a matrix to which the polypeptide or a peptide to which the antibodies were raised is bound. Those of skill in the art will know of various techniques common in the immunology arts for purification and/or concentration of polyclonal antibodies, as well as monoclonal antibodies (See for example, Coligan, *et al.,* Unit 9, *Current Protocols in Immunology,* Wiley Interscience, 1994, incorporated herein by reference). For example, an anti-RGS2 antiserum was made in rabbits against the C-terminus peptide of RGS2 (Seq Id No: 3) coupled to KHL followed by affinity purification with the immunizing peptide (Sinnarajah *et al.* (2001) *supra*)

Screening for binding compounds

Alternatively, RGS2 binding compounds may be found using ligand screening methods. In such screening method, a biological sample or a defined compound to be tested as a putative ligand of a RGS2 protein is brought into contact with the corresponding purified RGS2 protein, for example the corresponding purified recombinant RGS2 protein produced by a recombinant cell

RNA extraction

Total RNA was extracted with Trizol (Life Technologies) from $10^7$ cells/prep. Precipitated total RNA (average yield 100μg) was taken back in 40 μl and analyzed on agarose (1%) gel stained by Ethidium bromide. cDNA synthesis was carried out using Power Script Reverse Transcriptase (Clontech) with 5 μg of total RNA / in 20 μl preparation mix, corresponding to the suppliers protocol, using random hexamer primers.

Quantitative real time PCR

Real time PCR was performed on a Gene Amp 5700 (Applied Biosystems) using SybrGreen technology (SybrGreen PCR Master Mix from Applied Biosystems, Part Number : 4309155 or Quantitect SybrGreen PCR kit from Qiagen, Catalog No 204143). Primers of SEQ ID Nos 1 and 2 were used to amplify a portion of RGS2 polynucleotides and primers were used to amplify 185 ribosomal RNA chosen as the reference gene in those assays. Primers were used in a final concentration of 300nM with the quantity of cDNA of 2% of the cDNA preparation (corresponding to 100ng total RNA). After a preliminary step of 2 minutes at 50°C and a step of DNA polymerase activation of 10 minutes at 95°C, subsequent thermal cycles were carried out as follows: 10 seconds at 95°C to allow denaturation of double stranded cDNA and 1 minute at 60°C to allow annealing of primers and elongation of cDNA strands.

Results

Results are expressed as follows. The experiment was done in the six following different cell-culture experimental conditions:

1) non differentiated non activated U937, condition herein referred to as nd0h;
2) non differentiated U397 after 1 hour of activation, condition herein referred to as nd1h;
3) non differentiated U397 after 5 hours of activation, condition herein referred to as nd5h;
4) differentiated non activated U937, condition herein referred to as dbc0h;
5) differentiated U397 after 1 hour after activation, condition herein referred to as dbc1h;
6) differentiated U397 after 5 hours after of activation, condition herein referred to as dbc5h.

The number of cycles upon which the signal threshold is reached for RGS2 in a given experimental condition, herein referred to as Ct, was normalized by subtraction of the variation of Ct for the gene of reference, here 18S RNA, from the same RNA preparation. The geometric mean of all six experimental conditions, herein referred to as μCt, was then calculated and the relative gene expression level expressed as $2^{(\mu Ct-Ct)}$ for each experimental condition (see Table I).

Relative gene expression levels of two different experimental conditions, namely x and y, are then considered to be significantly different when the ratio of relative gene expression levels, i.e. x/y, is higher than 1.7, where x has a relative gene expression level higher than y.

RGS2 was thus classified as being specific for macrophage activation because the following criteria were met (see Table I):

# Sequence Listing

<110> Warner Lambert Company

<120> RGS2 as a biomarker for macrophage activation and a target in inflammation

<130> 021015

<160> 2

<210> 1
<211> 24
<212> DNA
<213> Artificial sequence

<220>
<223> Sequence source:/note=synthetic oligonucleotide

<400> 1
gaacagcttc cctcactgtg taca                                              24

<210> 2
<211> 26
<212> DNA
<213> Artificial sequence

<220>
<223> Sequence source:/note=synthetic oligonucleotide

<400> 2
ttcatcatct tacattcctg cttttc                                            26

# Sequence Listing

<110> Warner Lambert Company

<120> RGS2 as a biomarker for macrophage activation and a target in inflammation

<130> 021015 EP-CVB

<140> EP 02291645.6
<141> 2002-06-25

<160> 3

<210> 1
<211> 24
<212> DNA
<213> Artificial sequence

<220>
<223> Sequence source:/note=synthetic oligonucleotide

<400> 1
gaacagcttc cctcactgtg taca                                    24

<210> 2
<211> 26
<212> DNA
<213> Artificial sequence

<220>
<223> Sequence source:/note=synthetic oligonucleotide

<400> 2
ttcatcatct tacattcctg cttttc                                  26

<210> 3
<211> 10
<212> PRT
<213> Homo sapiens

<400> 3
Pro Gln Ile Thr Thr Glu Pro His Ala Thr
1               5                   10

## Claims

1. Use of RGS2 as a biomarker for macrophage activation.

2. Use of RGS2 as a biomarker for an activated-macrophage-related disorder.

3. The use according to claim 2, wherein said activated-macrophage-related disorder is an inflammatory disorder.

4. A method of determining whether macrophages are activated in a biological sample, said method comprising the steps of

   a) quantifying RGS2 expression and/or activity level in macrophages of said biological sample; and

   b) determining whether said macrophagic RGS2 expression and/or activity level in said biological sample is elevated compared to the macrophagic RGS2 expression and/or activity level in a control sample.

5. The method of claim 4, wherein step b) comprises determining whether said macrophagic RGS2 expression and/or activity level in said biological sample is elevated from approximately 1.7-fold to 1000-fold compared to said macrophagic RGS2 expression and/or activity level in said control sample.

6. The method of any one of claims 4 or 5, wherein said quantifying RGS2 expression in step a) comprises quantifying RGS2 polynucleotides present in said biological sample.

7. The method of any one of claims 4 or 5, wherein said quantifying RGS2 expression in step a) comprises quantifying RGS2 polypeptides present in said biological sample

8. The method of any one of claims 4 or 5, wherein step a) comprises quantifying RGS2 activity in said biological sample

9. A method of determining whether an individual suffers from an activated-macrophage-related disorder, wherein said method comprises the steps of

   a) quantifying the level of macrophagic RGS2 expression and/or activity levels in a biological sample recovered from said individual;

   b) determining whether said macrophagic RGS2 expression and/or activity levels are elevated in said individual compared to the macrophagic RGS2 expression level and/or activity in a control; and

   wherein said elevated level of macrophagic RGS2 expression and/or activity is taken as a sign of the presence of an activated-macrophage-related disorder.

10. A method of determining whether an individual who already suffers from an activated-macrophage-related disorder is susceptible to move into a more advanced stage of said activated-macrophage-related disorder, wherein said method comprises the steps of

    a) quantifying the macrophagic RGS2 expression and/or activity level in macrophages of a biological sample recovered from said individual;
    b) determining whether said macrophagic RGS2 expression and/or activity level in said individual is similar to the macrophagic RGS2 expression and/or activity level of an individual suffering from a more advanced stage of said activated-macrophage-related-disorder; and

    wherein said similar level of macrophagic RGS2 expression and/or activity is taken as a sign of the evolution for said individual towards said more advanced stage of activated-macrophage-related disorder

11. A method of monitoring an individual suffering from an activated-macrophage-related-disorder, wherein said method comprises the steps of

    a) quantifying the macrophagic level of RGS2 expression and/or activity in different samples recovered from the said individual at different time points; and
    b) comparing the macrophagic RGS2 expression andlor activity levels in said different samples.

12. A method of assessing the efficacy of a treatment for an individual suffering from an activated-macrophage-related disorder and submitted to said treatment, wherein said method comprises the steps of

    a) quantifying the macrophagic RGS2 expression levels and/or activity in a biological sample recovered from said individual; and

b) determining whether said macrophagic RGS2 expression level and/or activity is modulated in said biological sample compared to the macrophagic RGS2 expression level and/or activity in a control sample.

13. A method of determining whether an agent interferes with macrophage activation, wherein said method comprises the steps of

a) contacting said agent with macrophages under conditions allowing quantification of RGS2 expression and/or activity; and
b) determining whether RGS2 expression levels and/or activity are modulated in said sample upon macrophage activation.

14. A diagnostic kit for detecting an activated-macrophage-related disorder comprising reagents to determine the level of RGS2 expression and/or activity.

**European Patent**
**Office**

## PARTIAL EUROPEAN SEARCH REPORT

which under Rule 45 of the European Patent Convention
shall be considered, for the purposes of subsequent
proceedings, as the European search report

Application Number

EP 02 29 1645

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 6 410 240 B1 (HODGE MARTIN R ET AL) 25 June 2002 (2002-06-25) * column 4, line 41-67 * * column 5, line 3,4 * * column 6, line 4,5 * * column 38, line 25-37 * * column 39, line 21-63 * * column 44, line 15-58 * * column 50, line 38-40 * * figure 5 * --- | 1-14 | C07K14/47 |
| A | CHEN ET AL: "Characterization of a novel mammalian RGS protein that binds to Ga proteins and inhibits pheromone signalling in yeast" JOURNAL OF BIOLOGICAL CHEMISTRY, AMERICAN SOCIETY OF BIOLOGICAL CHEMISTS, BALTIMORE, MD, US, vol. 272, no. 13, 28 March 1997 (1997-03-28), pages 8679-8685, XP002113560 ISSN: 0021-9258 * page 8680, line 3-28 * --- -/-- | 1-14 | TECHNICAL FIELDS SEARCHED (Int.Cl.7) C07K |

## INCOMPLETE SEARCH

The Search Division considers that the present application, or one or more of its claims, does/do
not comply with the EPC to such an extent that a meaningful search into the state of the art cannot
be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| MUNICH | 28 November 2002 | Helliot, B |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C07)

European Patent
Office

INCOMPLETE SEARCH
SHEET C

Application Number

EP 02 29 1645

Claim(s) searched incompletely:
     14

Reason for the limitation of the search:

Claim 14 which relates to a diagnostic kit for detecting an
activated-macrophage-related-disorder is only defined in that it
comprises reagents to determine the level of RGS2 expression and/or
activity. However, in the absence of any indication as to the
characteristics of the reagents, this sole feature is not sufficient for
the skilled person to understand without undue burden the actual scope of
the claim. The search was therefore limited to a diagnostic kit
comprising primers disclosed in the example 1, namely SEQ 1 and 2.

**European Patent Office**

**PARTIAL EUROPEAN SEARCH REPORT**

Application Number

EP 02 29 1645

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| A | DE VRIES LUC ET AL: "RGS proteins: More than just GAPs for heterotrimeric G proteins." TRENDS IN CELL BIOLOGY, vol. 9, no. 4, April 1999 (1999-04), pages 138-143, XP002218797 ISSN: 0962-8924 * figure 2 * --- | 1-14 | |
| A | ZHENG BIN ET AL: "Divergence of RGS proteins: Evidence for the existence of six mammalian RGS subfamilies." TRENDS IN BIOCHEMICAL SCIENCES, vol. 24, no. 11, November 1999 (1999-11), pages 411-414, XP002218798 ISSN: 0968-0004 * page 411, column 2, line 11-17 * --- | 1-14 | TECHNICAL FIELDS SEARCHED (Int.Cl.7) |
| A | SIDEROVSKI D P ET AL: "A human gene encoding a putative basic helix-loop-helix phosphoprotein whose mRNA increases rapidly in cycloheximide-treated blood mononuclear cells." DNA AND CELL BIOLOGY, vol. 13, no. 2, 1994, pages 125-147, XP001106530 ISSN: 1044-5498 * figure 6 * ----- | 1-14 | |

## ANNEX TO THE EUROPEAN SEARCH REPORT
## ON EUROPEAN PATENT APPLICATION NO.

EP 02 29 1645

This annex lists the patent family members relating to the patent documents cited in the above-mentioned European search report.
The members are as contained in the European Patent Office EDP file on
The European Patent Office is in no way liable for these particulars which are merely given for the purpose of information.

28-11-2002

| Patent document cited in search report | | Publication date | Patent family member(s) | | Publication date |
|---|---|---|---|---|---|
| US 6410240 | B1 | 25-06-2002 | US | 6274362 B1 | 14-08-2001 |
| | | | AU | 3483100 A | 25-08-2000 |
| | | | EP | 1147213 A2 | 24-10-2001 |
| | | | JP | 2002535979 T | 29-10-2002 |
| | | | WO | 0046236 A2 | 10-08-2000 |
| | | | US | 2002081683 A1 | 27-06-2002 |

EPO FORM P0459

For more details about this annex : see Official Journal of the European Patent Office, No. 12/82